# EUROPEAN PATENT SPECIFICATION

(11) **EP 3 762 373 B1**
(45) Date of publication and mention of the grant of the patent: **30.04.2025**
(21) Application number: 19709666.2
(22) Date of filing: 04.03.2019
(51) Int. Cl.: C07D 307/77, C09K 11/00, H05B 33/10, C07D 405/14, C07D 333/78, C07D 405/04, C07D 491/052, C07D 491/147, C07D 493/04, C07D 493/14, C07D 495/04, C07F 7/00, C07D 307/91

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 06.03.2018 EP 18160179
(43) Date of publication of application: 13.01.2021
(73) Proprietor: Merck Patent GmbH, 64293 Darmstadt (DE)
(72) Inventor: LINGE, Rouven, 64291 DARMSTADT (DE); MEYER, Sebastian, 60594 FRANKFURT AM MAIN (DE); RODRIGUEZ, Lara-Isabel, 64285 DARMSTADT (DE); LACKNER, Aaron, 68165 MANNHEIM (DE)
(74) Representative: Merck Patent Association
(86) International application number: PCT/EP2019/055265
(87) International publication number: WO 2019/170578

(56) References cited:
- WO-A1-2012/141273
- CN-A- 107 501 302
- KR-A- 20140 000 611
- KR-A- 20150 114 636
- US-A1- 2016 233 427

## Description

The present invention relates to a compound as defined in claim 1, to the use of the compound in an electronic device, and to an electronic device comprising the compound. The present invention furthermore relates to a process for the preparation of this compound and to a formulation comprising one or more compounds as defined in claim 1.

The development of functional compounds for use in electronic devices is currently the subject of intensive research. The aim is, in particular, the development of compounds with which improved properties of electronic devices in one or more relevant points can be achieved, such as, for example, power efficiency and lifetime of the device as well as colour coordinates of the emitted light.

In accordance with the present invention, the term electronic device is taken to mean, inter alia, organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

Of particular interest is the provision of compounds for use in the last-mentioned electronic devices called OLEDs. The general structure and the functional principle of OLEDs are known to the person skilled in the art and are described, for example, in US 4539507.

Further improvements are still necessary with respect to the performance data of OLEDs, in particular with a view to broad commercial use, for example in display devices or as light sources. Of particular importance in this connection are the lifetime, the efficiency and the operating voltage of the OLEDs and as well as the colour values achieved. In particular, in case of blue-emitting OLEDs, there is potential for improvement with respect to the lifetime and the efficiency of the devices.

An important starting point for achieving the said improvements is the choice of the emitter compound and of the host for the emitter compound employed in the electronic device.

In the prior art, it is known that compounds comprising anthracenes, such as disclosed in WO 2017/036573, are well suitable for use as host materials for fluorescent emitters in OLEDs. Furthermore, in KR20150128583, compounds comprising an anthracene and a dibenzofuran derivative are disclosed, which are employed as host material for fluorescent emitters. In KR20150114636 compounds comprising oxatruxene derivatives are also disclosed as OLED materials.

However, there is still a need for host materials, which show excellent performance data when used in the emitting layer of an OLED, in particular in regard to lifetime, efficiency and colour values of the emitted light.

Furthermore, it is known that an OLED may comprise different layers, which may be applied either by vapour deposition in a vacuum chamber or by processing from a solution. The processes based on vapour deposition lead to very good results but such processes are complex and expensive. Therefore, there is also a need for OLED materials that can be easily and reliably processed from solution. In this case, the materials should have good solubility properties in the solution that comprises them.

The present invention is thus based on the technical object of providing compounds which are suitable for use in electronic devices such as OLEDs, which are more particularly suitable as host materials for blue-fluorescent emitters in OLEDs and, which are suitable for vacuum processing or for solution processing.

In investigations on novel compounds for use in electronic devices, it has now been found, that compounds of the invention as defined below are eminently suitable for use in electronic devices. In particular, they achieve one or more, preferably all, of the above-mentioned technical objects.

The invention thus relates to compounds of formula (2B), (3B) or (4B), 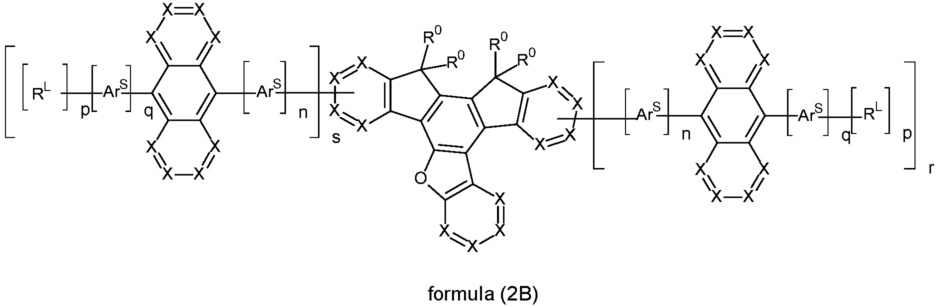 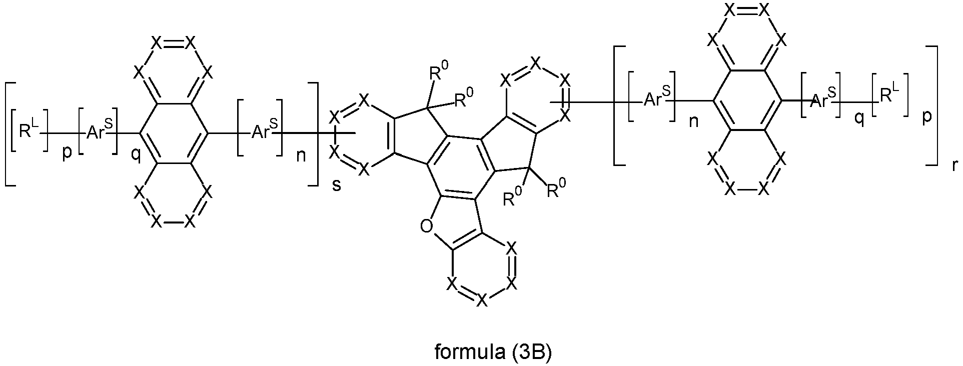 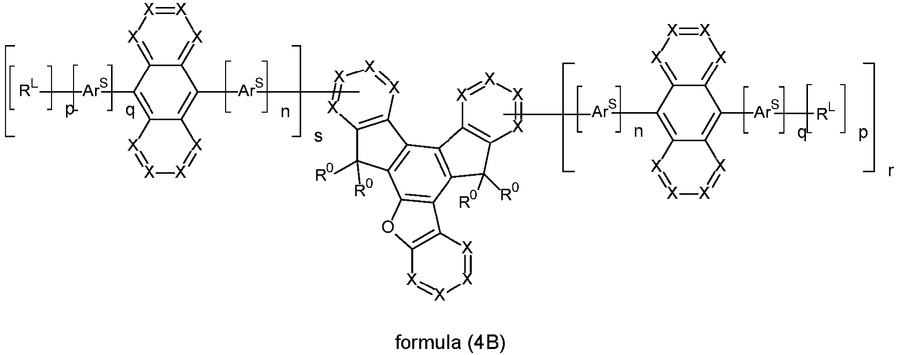 where the following applies to the symbols and indices used:
Ar^{S} stands on each occurrence, identically or differently, for an aromatic or heteroaromatic ring system having 5 to 25 aromatic ring atoms, which may in each case be substituted by one or more radicals R¹;
X stands for CR², N; or X stands for C, if it is bonded to an adjacent group;
R⁰, R¹, R² stand on each occurrence, identically or differently, for H, D, F, a straight-chain alkyl, alkoxy or thioalkyl groups having 1 to 20 C atoms or branched or a cyclic alkyl, alkoxy or thioalkyl groups having 3 to 20 C atoms, each of which may be substituted by one or more radicals R, where one or more H atoms may be replaced by D or F, an aromatic or heteroaromatic ring systems having 5 to 30 aromatic ring atoms, which may in each case be substituted by one or more radicals R, where two radicals R⁰ may form a ring system with one another, which may be substituted by one or more radicals R;
R^{L} stands on each occurrence, identically or differently, for H, D, F, a straight-chain alkyl group having 1 to 20 C atoms or branched or a cyclic alkyl group having 3 to 20 C atoms, each of which may be substituted by one or more radicals R, or an aromatic ring system having 5 to 25 aromatic ring atoms, which may in each case be substituted by one or more radicals R;
R stands on each occurrence, identically or differently, for H, D, F, a straight-chain alkyl group having 1 to 10 C atoms or branched or cyclic alkyl group having 3 to 20 C atoms, each of which may be substituted by one or more radicals R', where one or more H atoms may be replaced by D or F, or an aromatic ring system having 6 to 18 aromatic ring atoms;
Ar is an aromatic or heteroaromatic ring system having 5 to 24 aromatic ring atoms, which may in each case also be substituted by one or more radicals R';
R' stands on each occurrence, identically or differently, for H, D, F, a straight-chain alkyl group having 1 to 10 C atoms or branched or cyclic alkyl group having 3 to 10 C atoms, where one or more H atoms may be replaced by D or F, or an aromatic ring system having 5 to 18 C atoms;
n, q, p are identically or differently an integer selected from 0 or 1; and
r, s are identically or differently, equal to 0 or 1;
   with the proviso that r + s ≥ 1.

Furthermore, the following definitions of chemical groups apply for the purposes of the present application:
Adjacent substituents in the sense of the present invention are substituents which are bonded to atoms which are linked directly to one another or which are bonded to the same atom.

An aryl group in the sense of this invention contains 6 to 60 aromatic ring atoms, preferably 6 to 40 aromatic ring atoms, more preferably 6 to 20 aromatic ring atoms; a heteroaryl group in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and S. This represents the basic definition. If other preferences are indicated in the description of the present invention, for example with respect to the number of aromatic ring atoms or the heteroatoms present, these apply. An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system, preferably 6 to 40 C atoms, more preferably 6 to 20 C atoms. A heteroaromatic ring system in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoro-methylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following schemes:

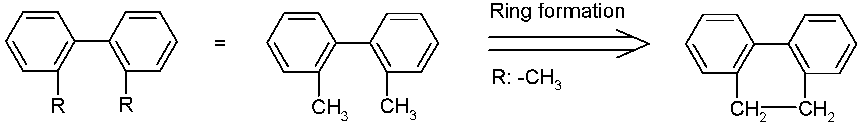

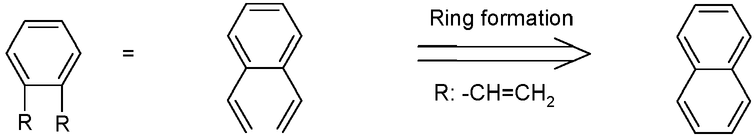

Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

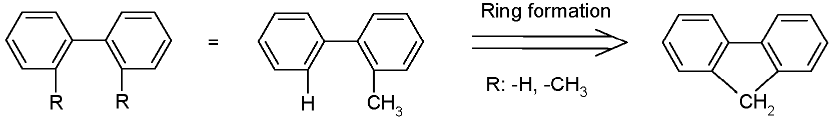

Preferably, n and q are, identically or differently, equal to 0 or 1 and p is equal to 1.

In accordance with a preferred embodiment, the compounds of formulae (2B), (3B) and (4B) are selected from the compounds of formulae (2B-1) to (4B-3), 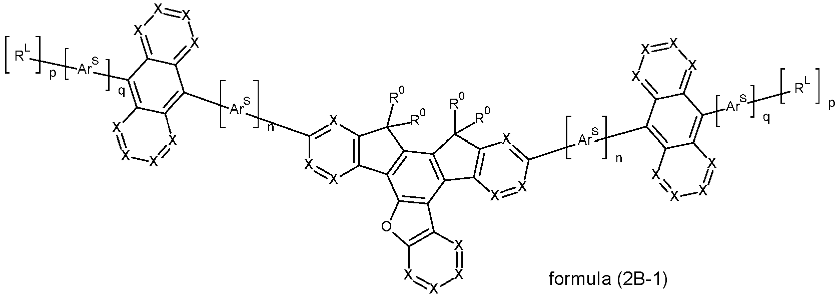 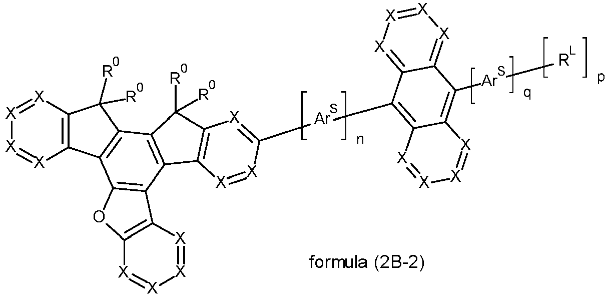 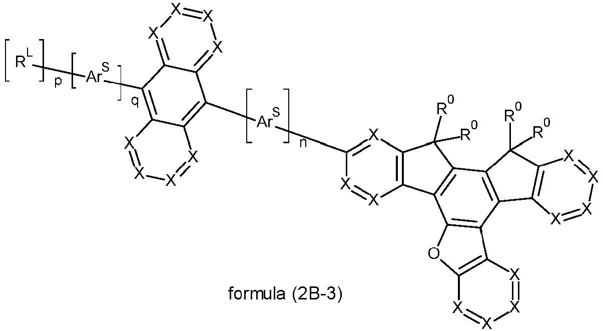 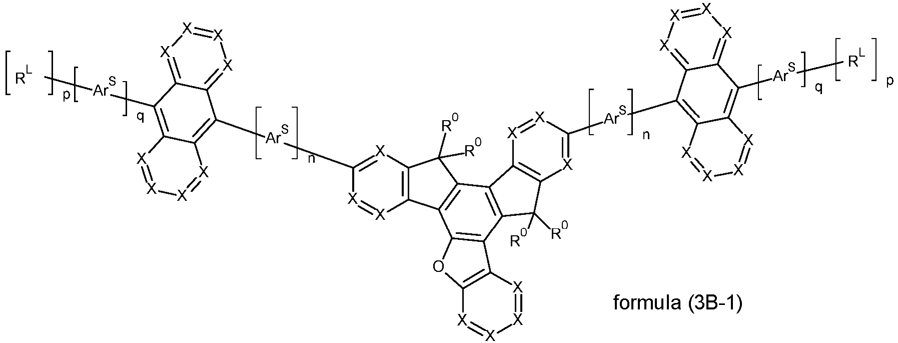 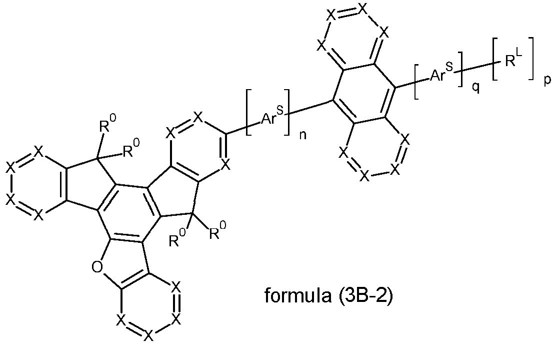 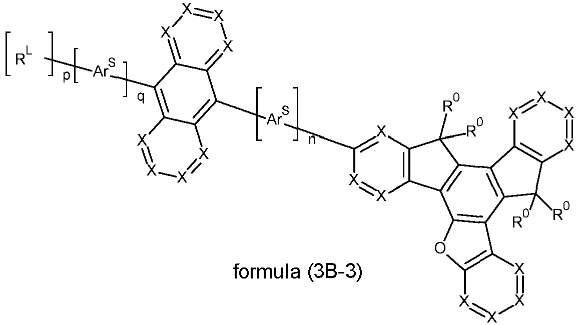 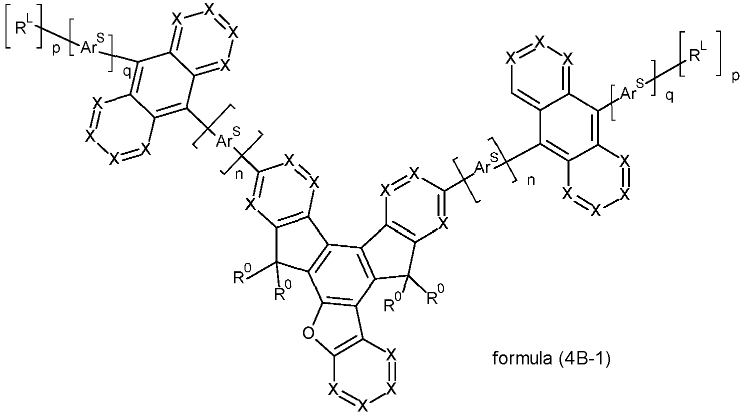 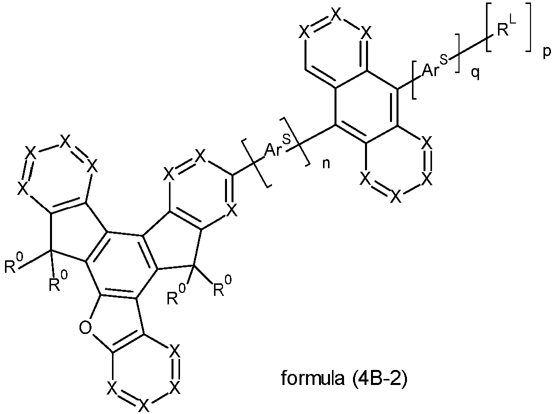 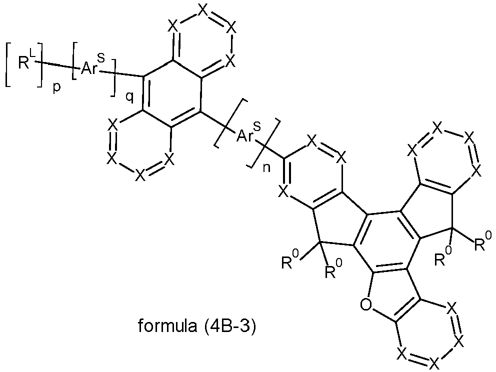 where the symbols and indices have the same meaning as above.

Ar^{S} stands on each occurrence, identically or differently, for an aromatic or heteroaromatic ring system having 5 to 25 aromatic ring atoms, which may in each case be substituted by one or more radicals R¹. Examples of suitable groups Ar^{S} are phenyl, biphenyl, fluorene, spirobifluorene, naphthalene, phenanthrene, anthracene, dibenzofuran, dibenzothiophene, carbazole, pyridine, pyrimidine, pyrazine, pyridazine, triazine, benzopyridine, benzopyridazine, benzopyrimidine and quinazoline, each of which may be substituted by one or more radicals R¹.

Examples of very suitable groups Ar^{S} are the groups of formulae (ArS-1) to (ArS-9),

| | | |
|---|---|---|
| 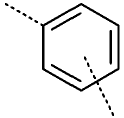 | 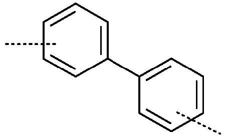 | 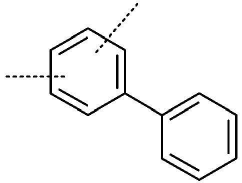 |
| (ArS-1) | (ArS-2) | (ArS-3) |
| 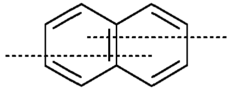 | 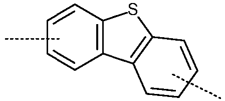 | 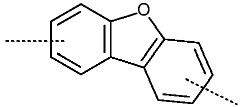 |
| (ArS-4) | (ArS-5) | (ArS-6) |
| 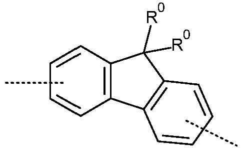 | 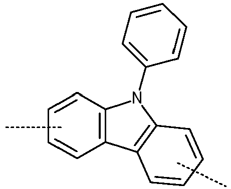 | 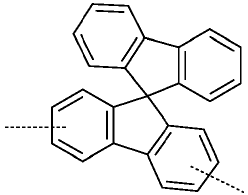 |
| (ArS-7) | (ArS-8) | (ArS-9) |

where the group R⁰ in formula (ArS-7) has the same meaning as above, where the dashed bonds indicate the bonding to the adjacent groups in formula (1); and
where the groups of formulae (ArS-1) to (ArS-9) may be substituted at each free position by a group R¹, which has the same meaning as above.

Among formulae (ArS-1) to (ArS-9), formulae (ArS-1), (ArS-2), (ArS-3), (ArS-4), (ArS-7) and (ArS-9) are preferred.

Preferably, when R^{L} stands for an aromatic or heteroaromatic ring system, then R^{L} is selected from the group consisting of phenyl, biphenyl, terphenyl, quaterphenyl, fluorene, spirobifluorene, naphthalene, phenanthrene, anthracene, triphenylene, fluoranthene, tetracene, chrysene, benzanthracene, benzophenanthracene, pyrene, perylene, indole, benzofuran, benzothiophene, dibenzofuran, dibenzothiophene, carbazole, indenocarbazole, indolocarbazole, pyridine, pyrimidine, pyrazine, pyridazine, triazine, quinolone, benzopyridine, benzopyridazine, benzopyrimidine, benzimidazole and quinazoline, each of which may be substituted by one or more radicals R.

Very preferably, when R^{L} stands for an aromatic or heteroaromatic ring system, then R^{L} is selected from the group consisting of phenyl, biphenyl, terphenyl, quaterphenyl, fluorene, naphthalene, phenanthrene, triphenylene, fluoranthene, tetracene, chrysene, benzanthracene, benzophenanthracene, pyrene or perylene, each of which may be substituted by one or more radicals R¹ at any free positions.

Suitable examples of the groups R^{L}, when R^{L} stands for an aromatic or heteroaromatic ring system, are the groups of formulae (RL-1) to (RL-27),

| | | |
|---|---|---|
| 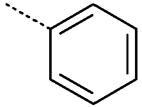 | 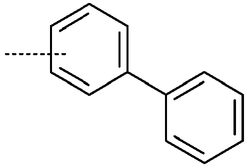 | 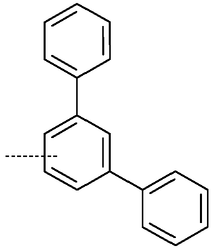 |
| (RL-1) | (RL-2) | (RL-3) |
| 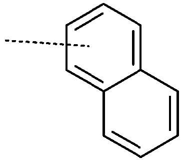 | 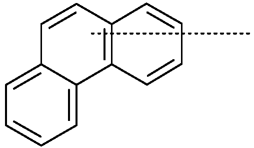 | 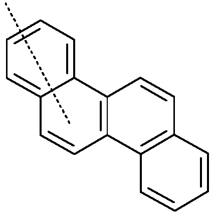 |
| (RL-4) | (RL-5) | (RL-6) |
| 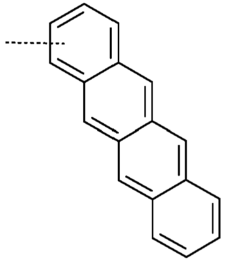 | 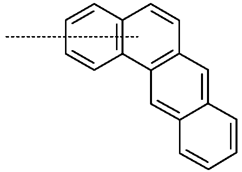 | 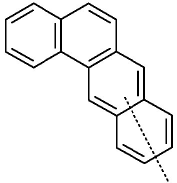 |
| (RL-7) | (RL-8) | (RL-9) |
| 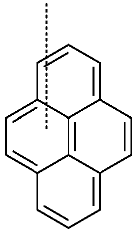 | 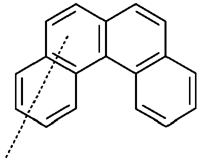 | 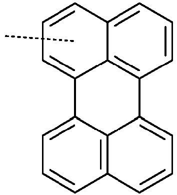 |
| (RL-10) | (RL-11) | (RL-12) |
| 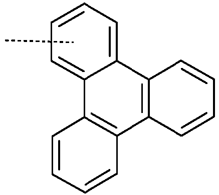 | 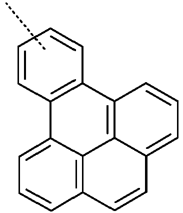 | 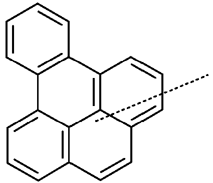 |
| (RL-13) | (RL-14) | (RL-15) |
| 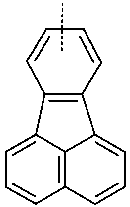 | 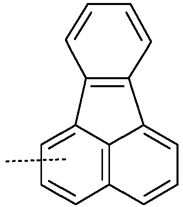 | 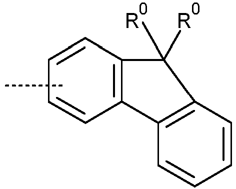 |
| (RL-16) | (RL-17) | (RL-18) |
| 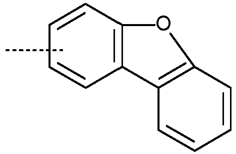 | 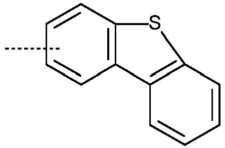 | 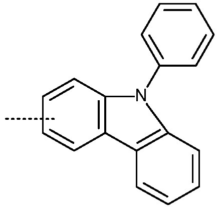 |
| (RL-19) | (RL-20) | (RL-21) |
| 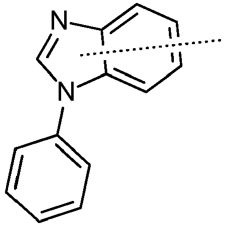 | 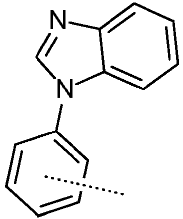 | 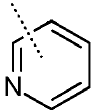 |
| (RL-22) | (RL-23) | (RL-24) |
| 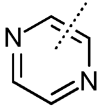 | 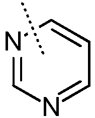 | 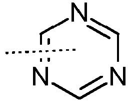 |
| (RL-25) | (RL-26) | (RL-27) |

where the dashed bond indicates the bonding to Ar¹ and where the group R⁰ has the same meaning as above; and where the groups of formulae (RL-1) to (RL-27) may be substituted at each free position by a group R, which has the same meaning as above.

Very particularly preferably, the group R^{L} is an aromatic or heteroaromatic ring system selected from the groups of formulae (RL-1) to (RL-27), each of which may be substituted by one or more radicals R at any free positions.

The compounds of formulae (2B), (3B) and (3C) may comprise a group ArL of formula (ArL-1), 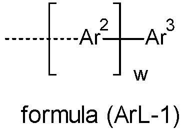 where the dashed bond in formula (ArL-1) indicates the bonding to the structure of formula (2B), (3B) or (4B).

In formula (ArL-1), it is preferred that the group Ar² is selected from the groups of formulae (Ar2-1) to (Ar2-25), 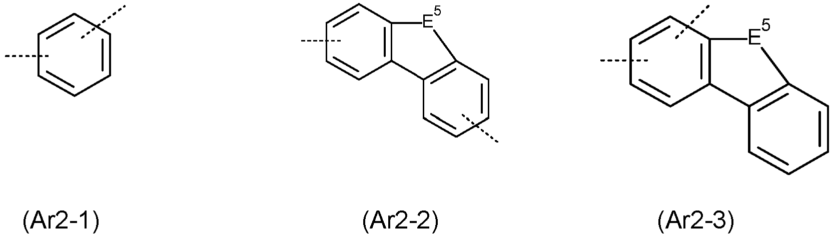 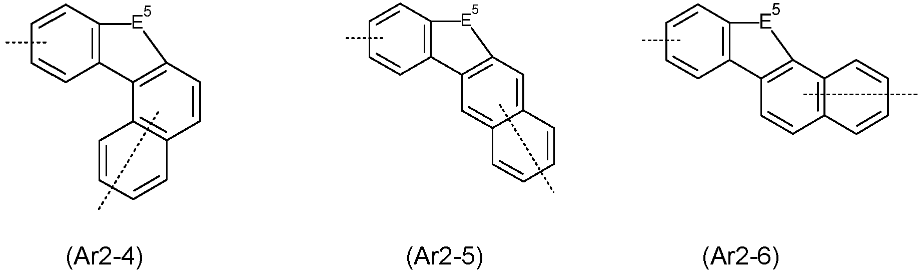 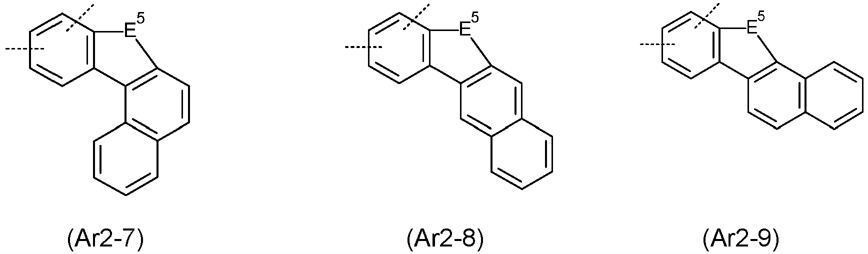 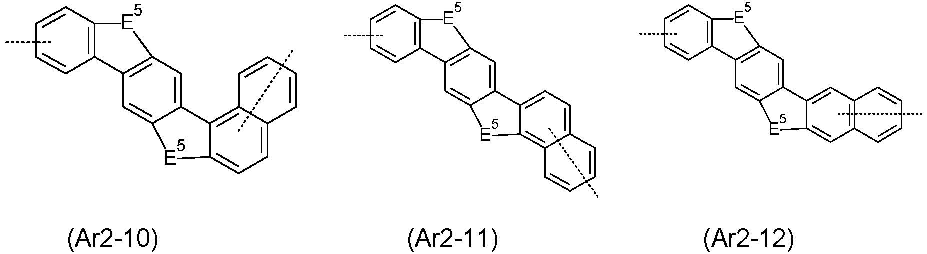 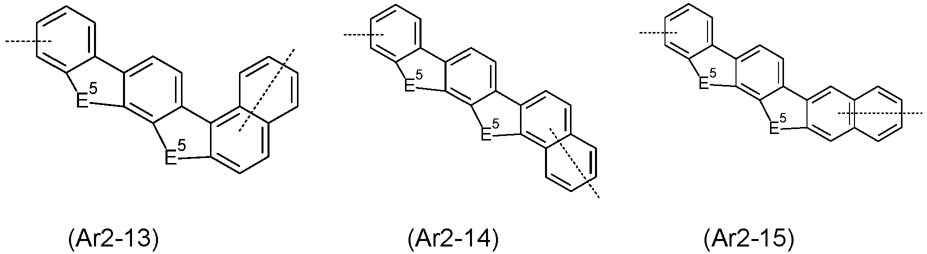 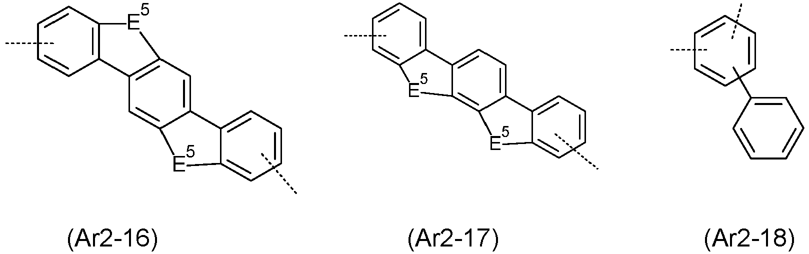 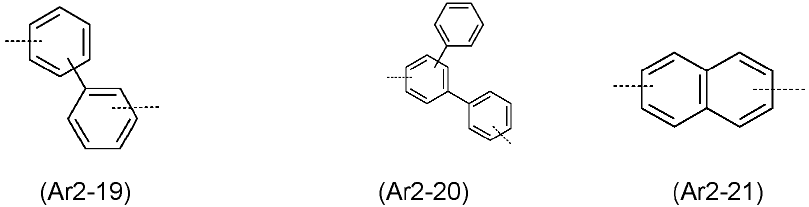 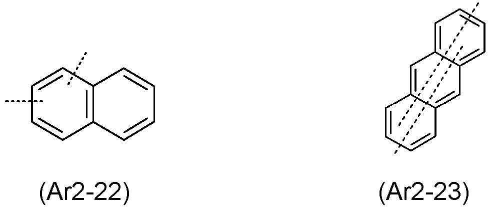 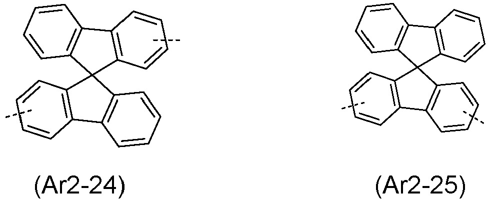
where the dashed bonds indicate the bonding to the structure of formula (2B), (3B) or (4B) and to a group Ar² or Ar³;
where the groups of formulae (Ar2-1) to (Ar2-25) may be substituted at each free position by a group R, which has the same meaning as above; and where
E⁵ is selected from -B(R⁰-), -C(R⁰)₂₋, -C(R⁰)₂-C(R⁰)₂-, -Si(R⁰)₂₋, -C(=O)-, - C(=NR⁰)-, -C=(C(R⁰))₂-, -O-, -S-, -S(=O)-, -SO₂-, -N(R⁰)-, -P(R⁰)- and - P((=O)R⁰)-, where the substituent R⁰ has the same meaning as above.

Preferably, E⁵ is selected from -C(R⁰)₂-, -Si(R⁰)₂-, -O-, -S- or -N(R⁰)-, where the substituent R⁰ has the same meaning as above

Among formulae (Ar2-1) to (Ar2-25), following formulae are preferred:
(Ar2-1), (Ar2-2), (Ar2-3), (Ar2-18), (Ar2-19), (Ar2-20), (Ar2-21), (Ar2-22) and (Ar2-25).

Furthermore, in formula (ArL-1), it is preferred that Ar³ is on each occurrence, identically or differently, selected from the group consisting of the groups of formulae (Ar3-1) to (Ar3-27), 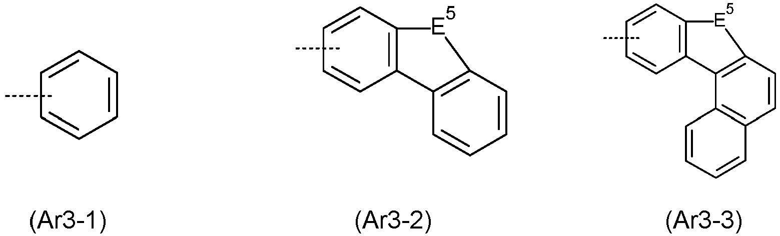 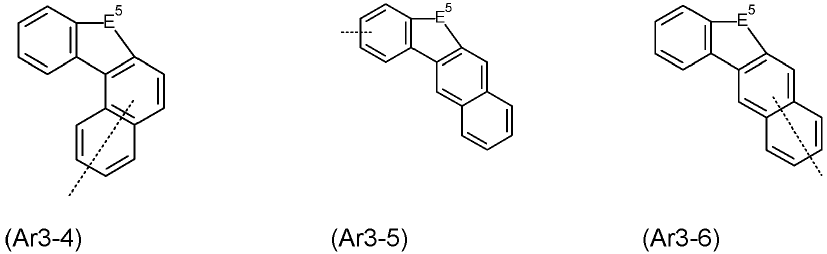 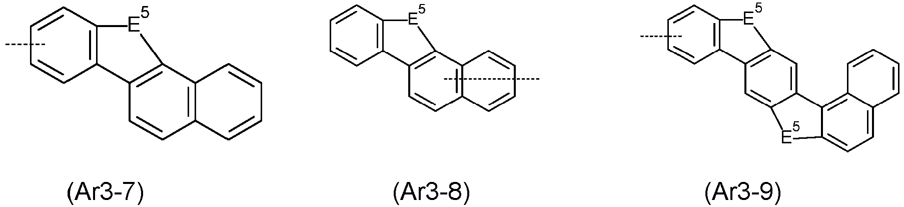 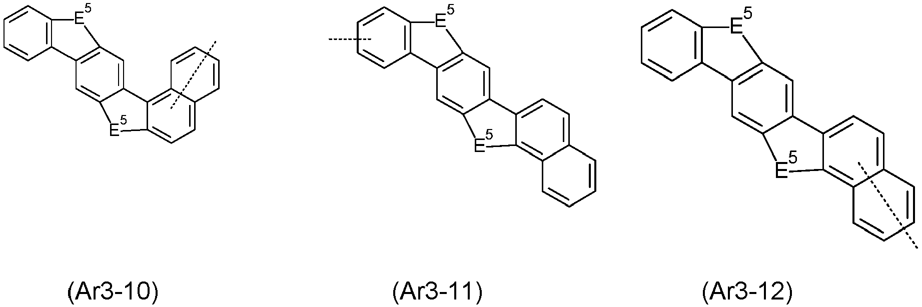 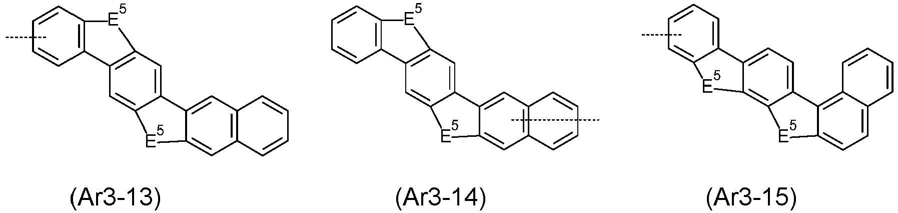 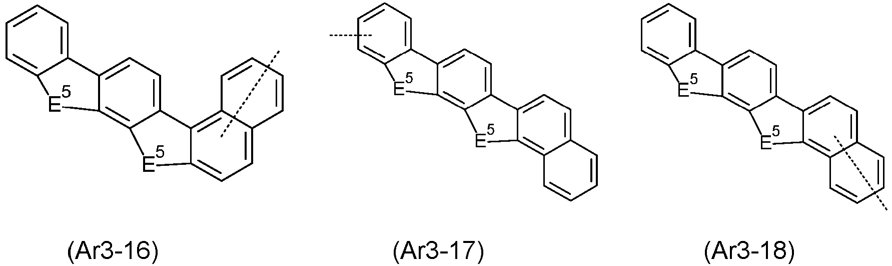 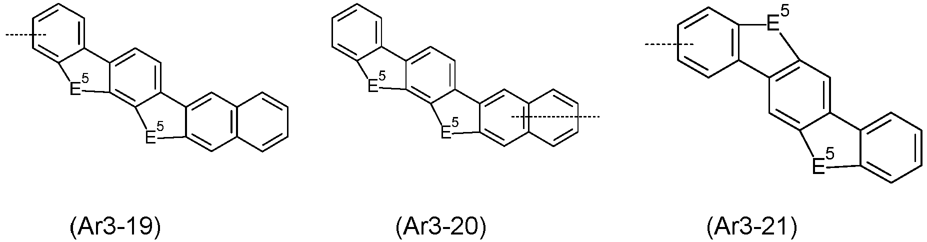 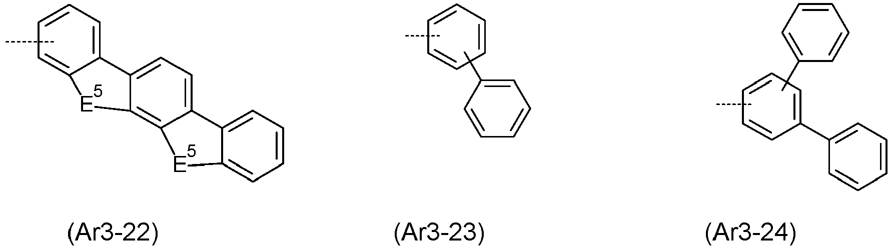 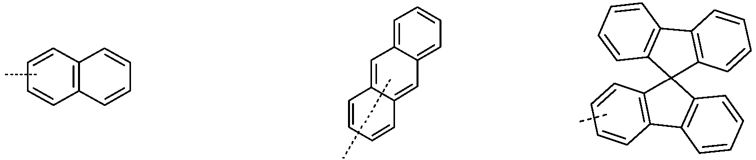 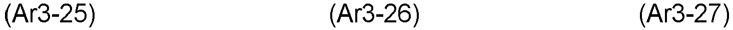
where the dashed bond indicates the bonding to Ar²;
where E⁵ has the same meaning as above; and
where the groups of formulae (Ar3-1) to (Ar3-27) may be substituted at each free position by a group R, which has the same meaning as above.

Among formulae (Ar3-1) to (Ar2-27), following formulae are preferred: (Ar3-1), (Ar3-2), (Ar3-23), (Ar3-24), (Ar3-25) and (Ar3-27).

In accordance with a preferred embodiment at least one group Ar² stands for a group of formula (Ar2-2) and/or at least one group Ar³ stands for a group of formula (Ar3-2), 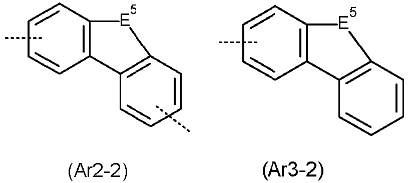 where
the dashed bonds in formula (Ar2-2) indicate the bonding to the structure of formula (2B), (3B) or (4B) and to a group Ar² or Ar³; and the dashed bond in formula (Ar3-2) indicates the bonding to Ar²; and E⁵ has the same meaning as in above; and the groups of formulae (Ar2-2) and (Ar3-2) may be substituted at each free position by a group R, which has the same meaning as above.

In accordance with a very preferred embodiment, at least one group Ar² stands for a group of formula (Ar2-2-1) and/or at least one group Ar³ stands for a group of formula (Ar3-2-1), 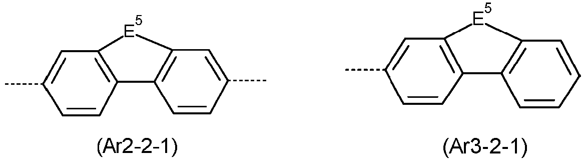 where
the dashed bonds in formula (Ar2-2-1) indicate the bonding to the structure of formula (2B), (3B9 or (4B) and to a group Ar² or Ar³;
the dashed bond in formula (Ar3-2-1) indicates the bonding to Ar²;
E⁵ has the same meaning as above; and
the groups of formulae (Ar2-2-1) and (Ar3-2-1) may be substituted at each free position by a group R, which has the same meaning as above.

In accordance with a particularly preferred embodiment, at least one group Ar² stands for a group of formula (Ar2-2-1b) and/or at least one group Ar³ stands for a group of formula (Ar3-2-1b), 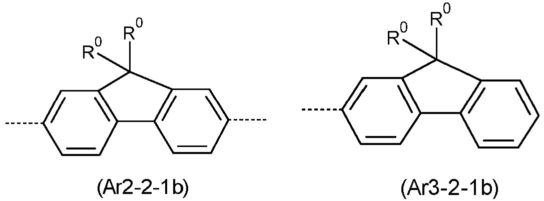 where
the dashed bonds in formula (Ar2-2-1b) indicate the bonding to the structure of formula (2B), (3B) or (4B) and to a group Ar² or Ar³;
the dashed bond in formula (Ar3-2-1b) indicates the bonding to Ar²;
R⁰ has the same meaning as above; and
the groups of formulae (Ar2-2-1b) and (Ar3-2-1b) may be substituted at each free position by a group R, which has the same meaning as above.

The following compounds are examples of compounds of the invention:

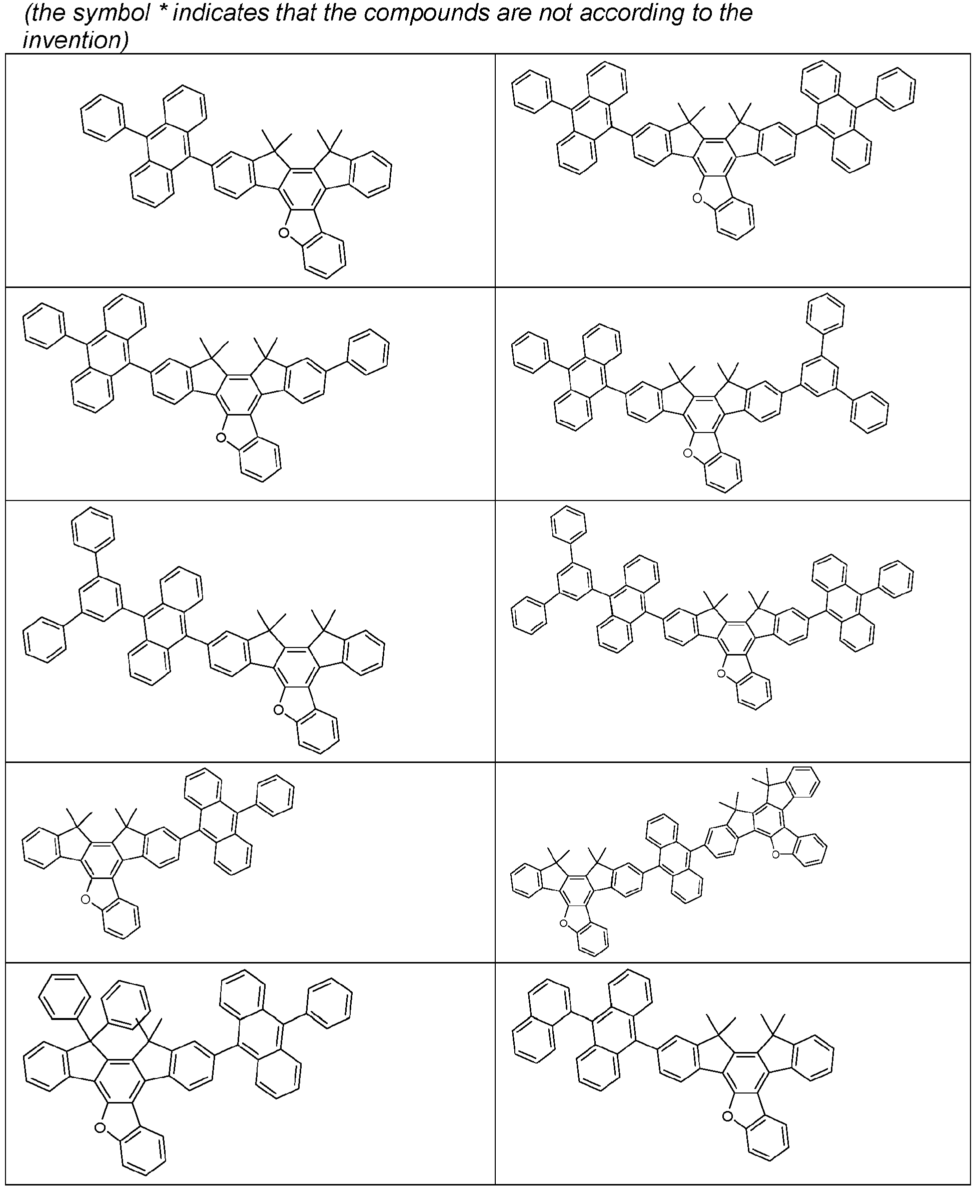

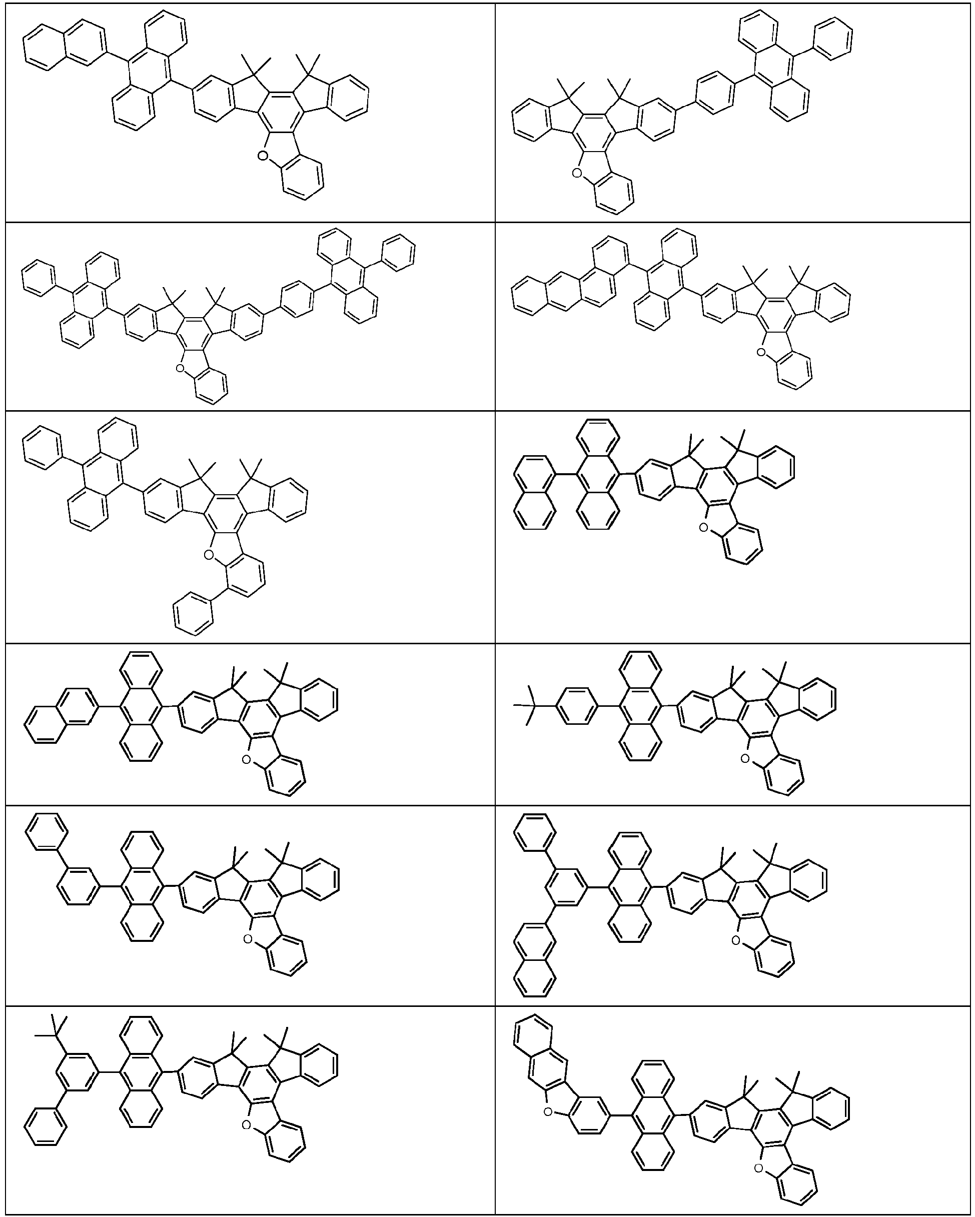

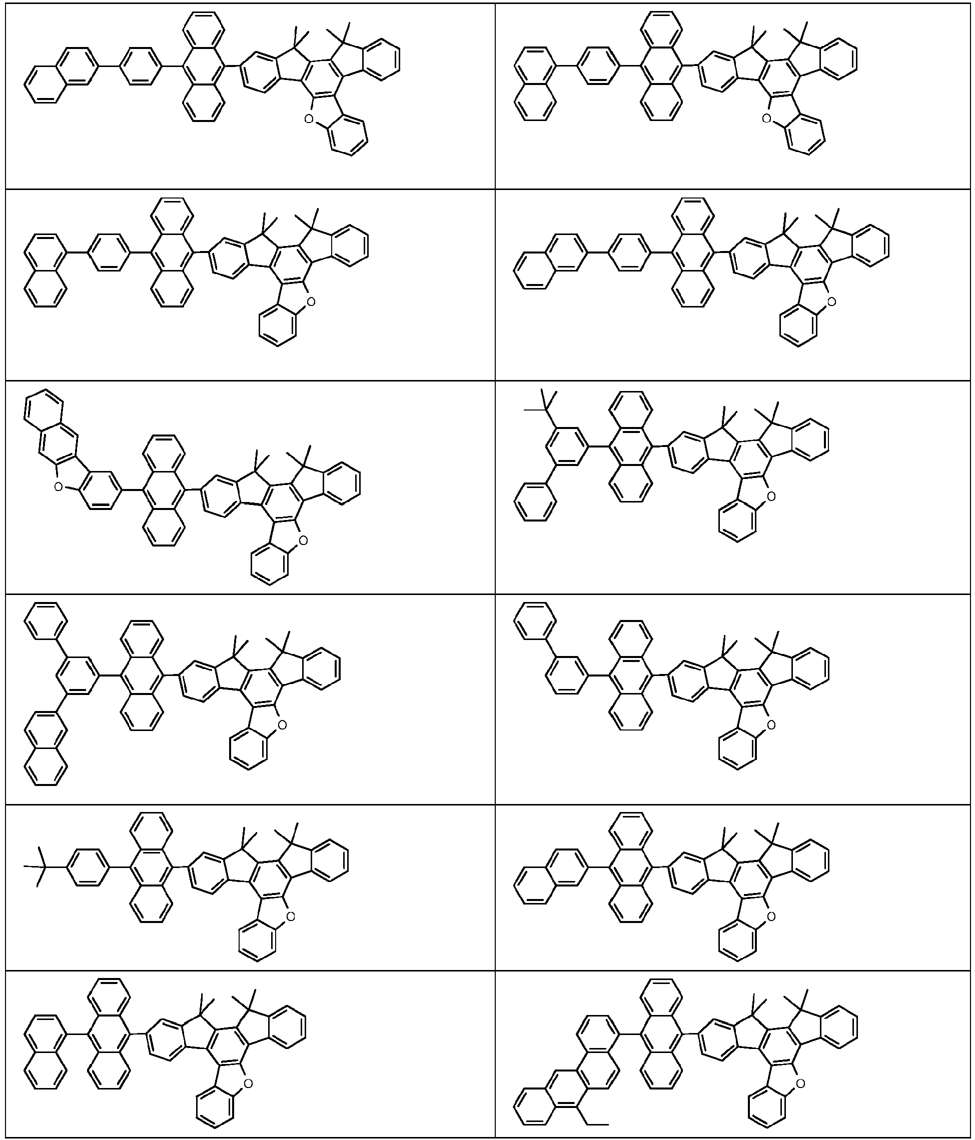

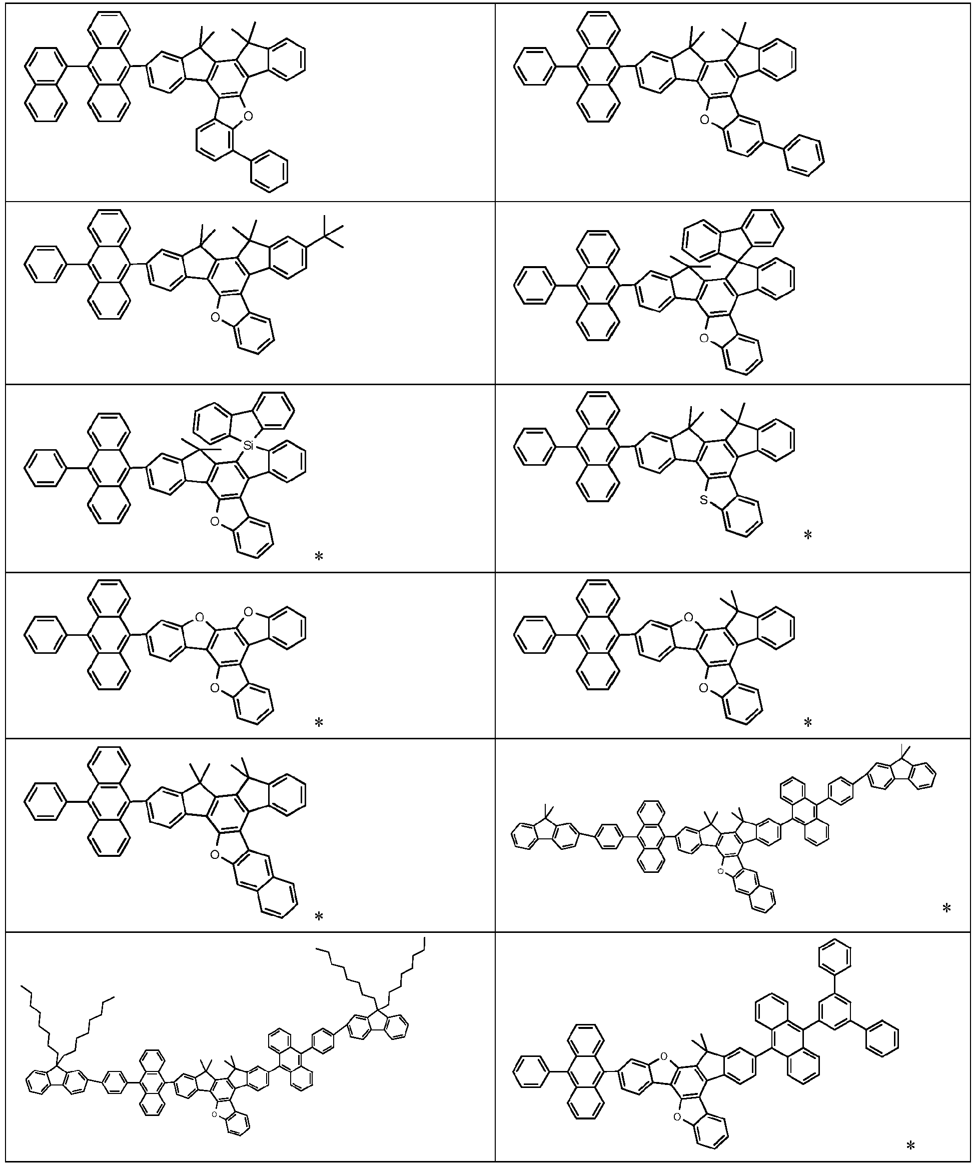

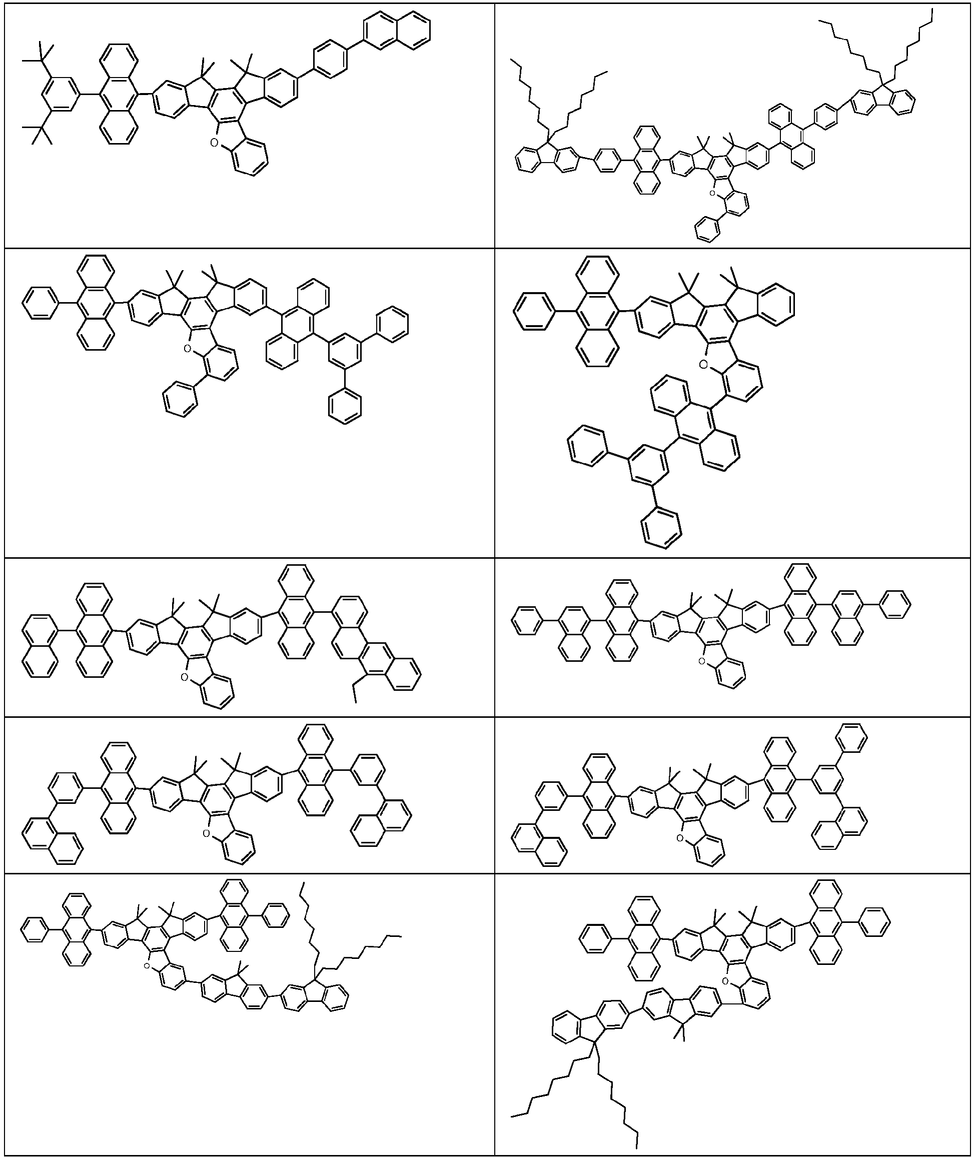

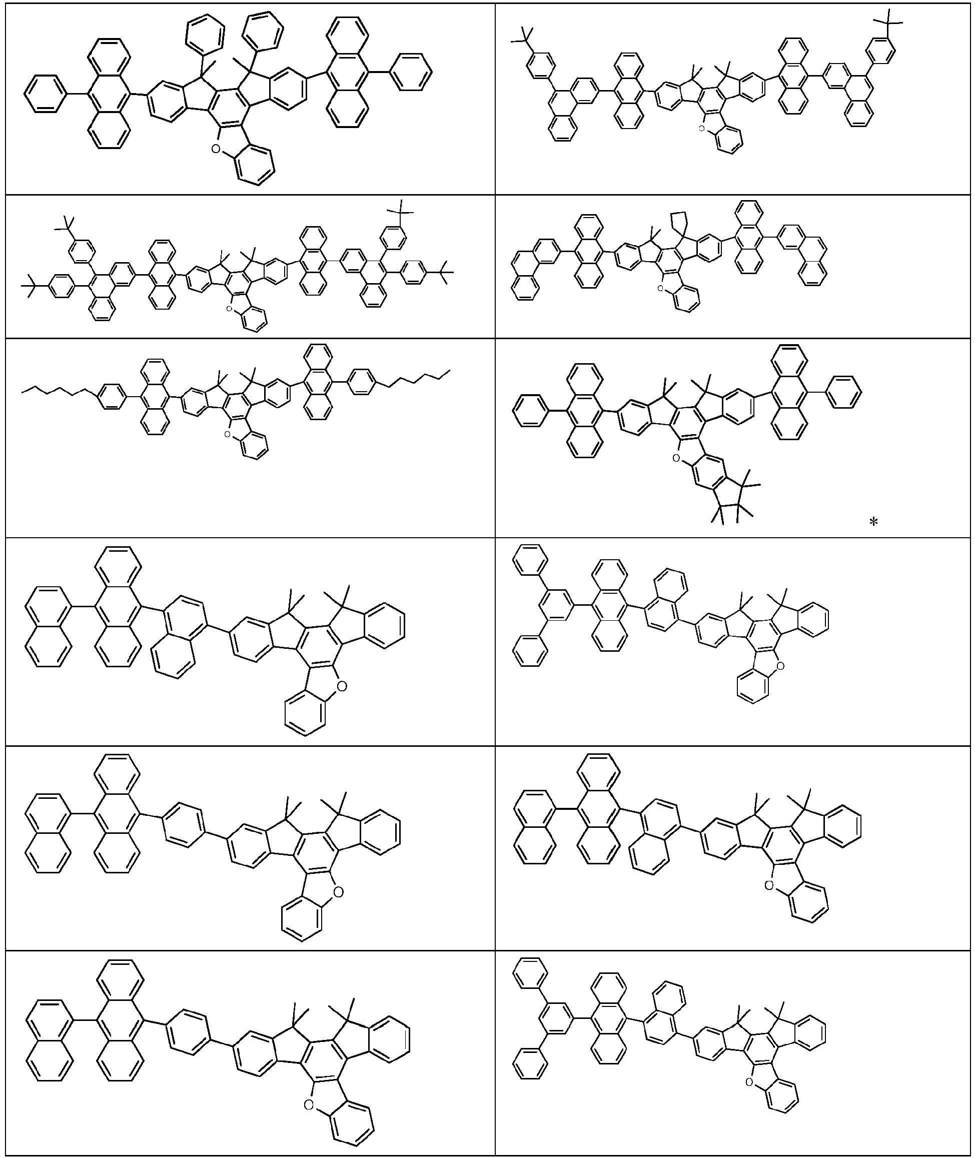

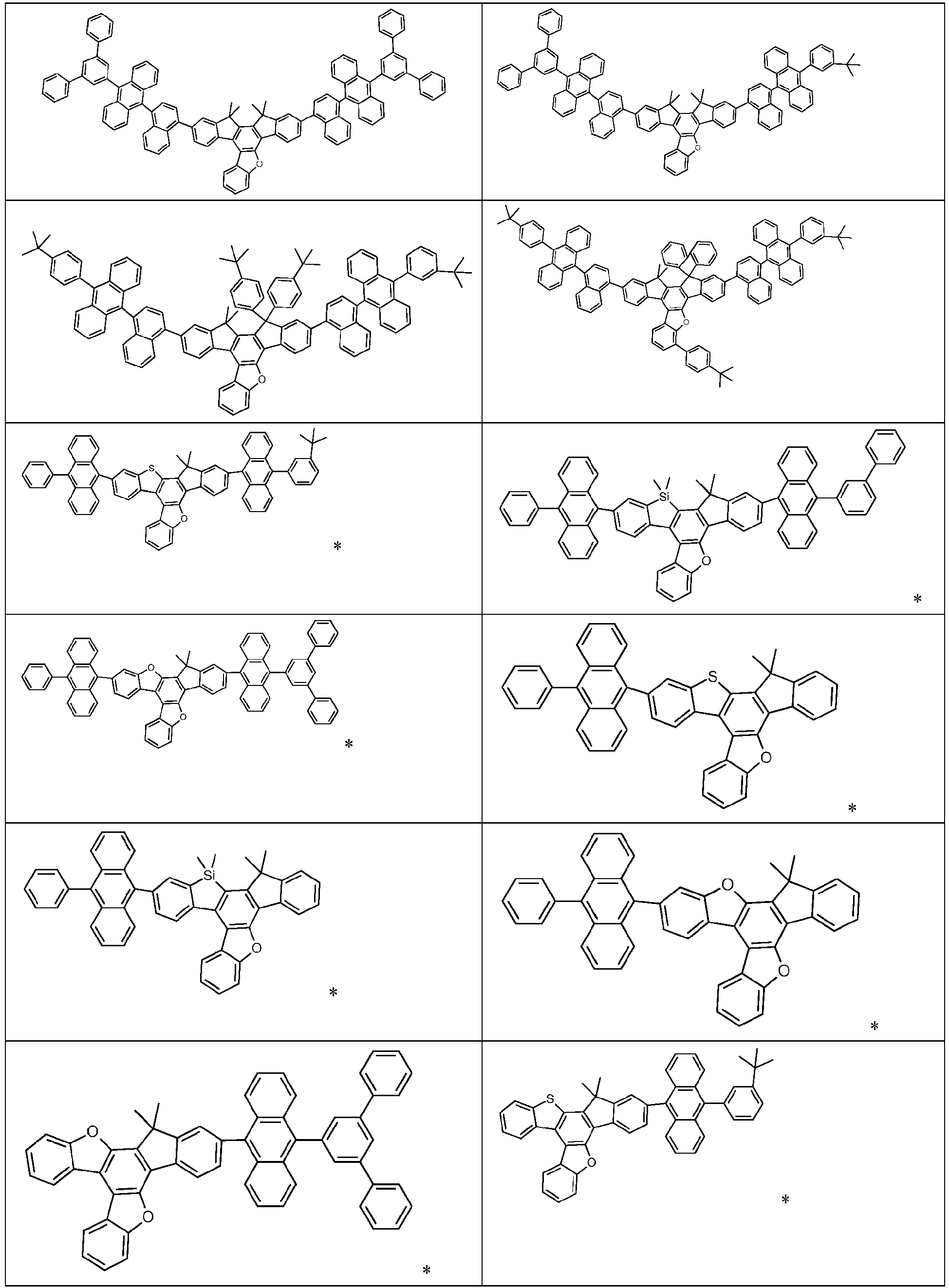

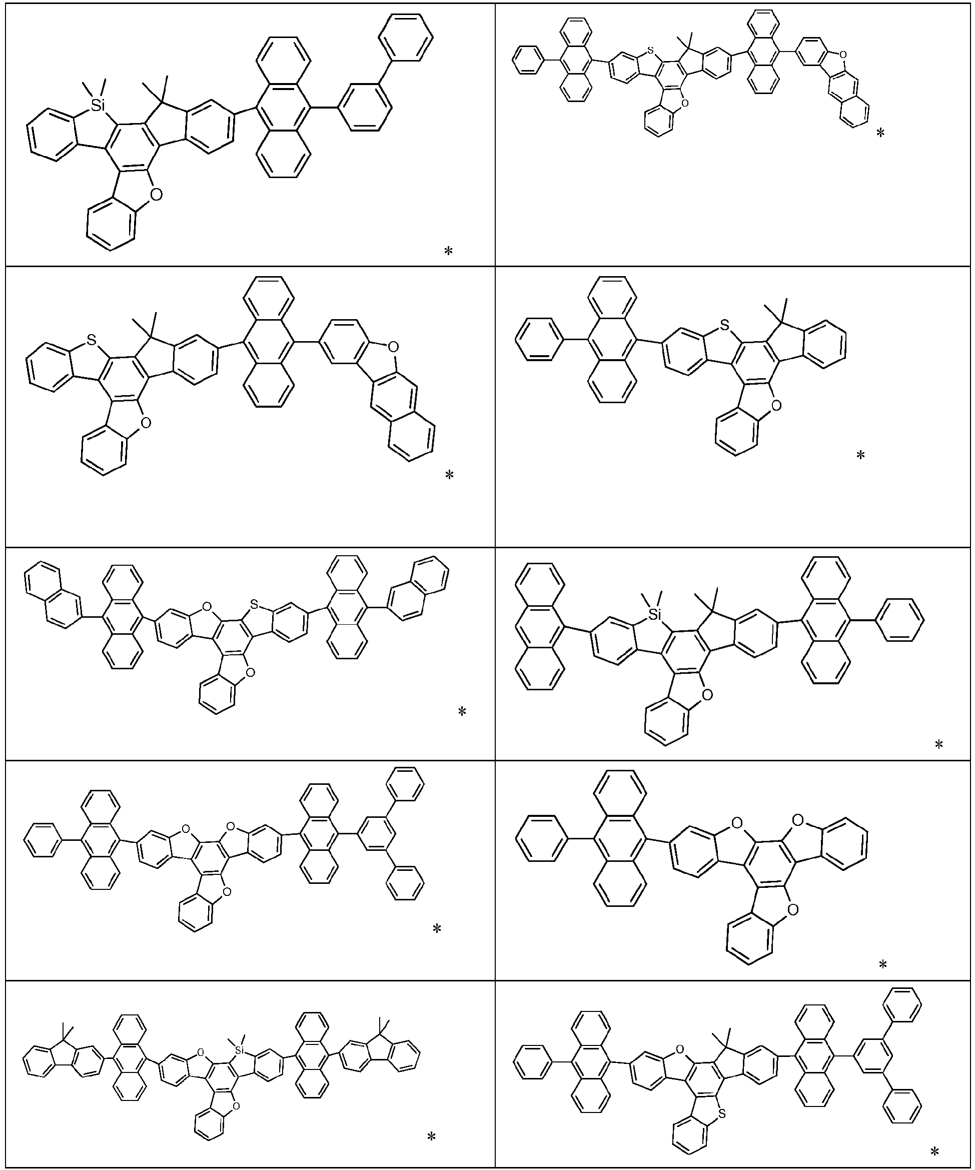

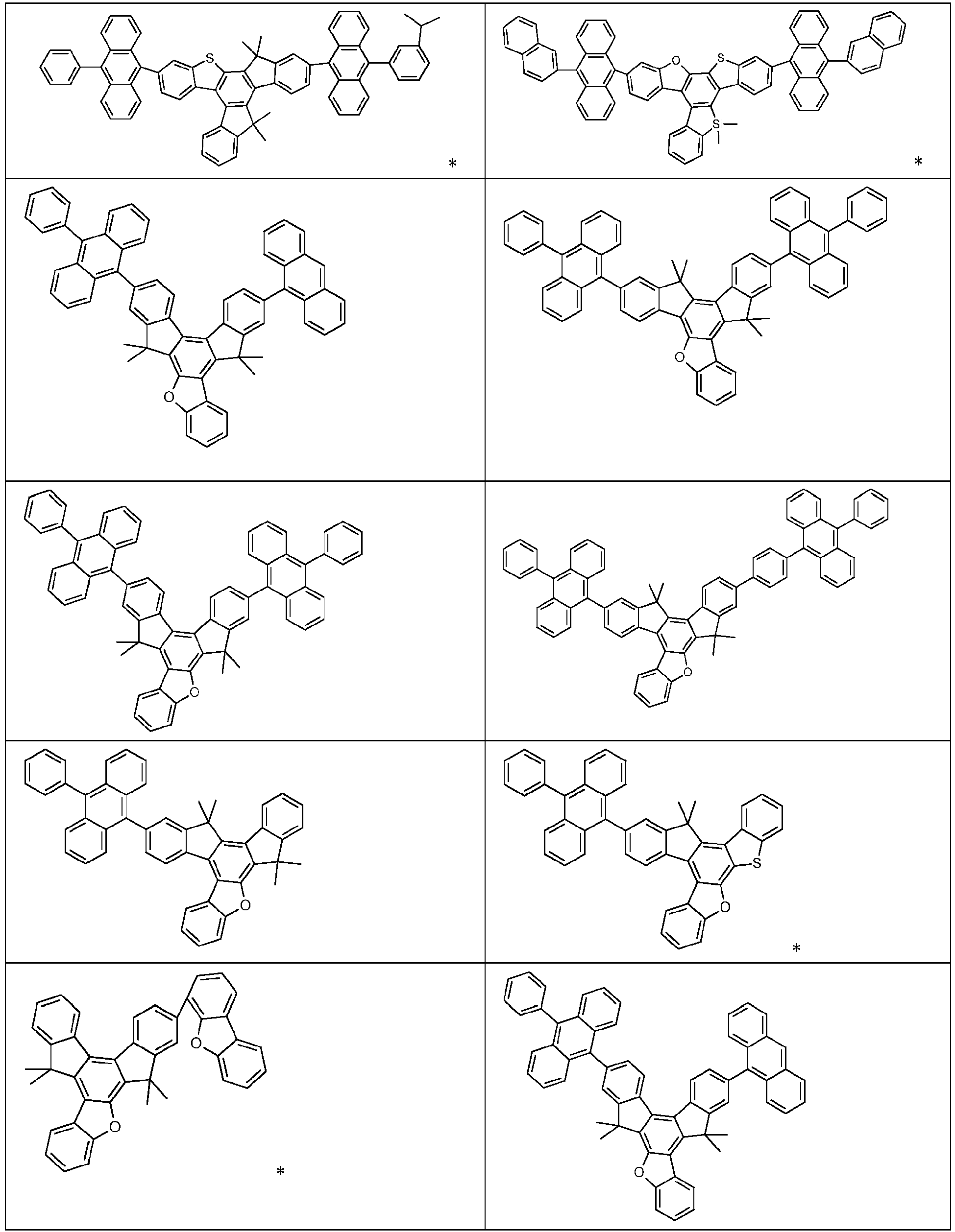

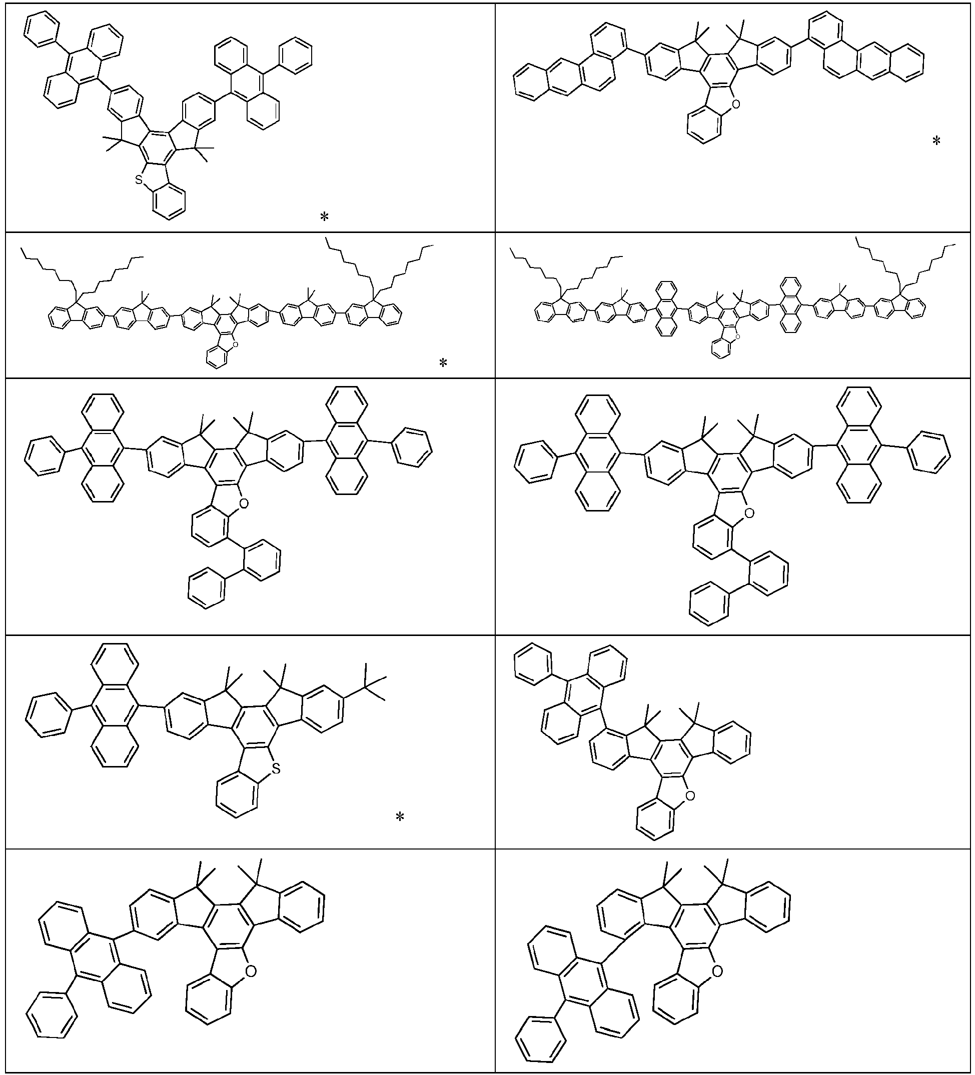

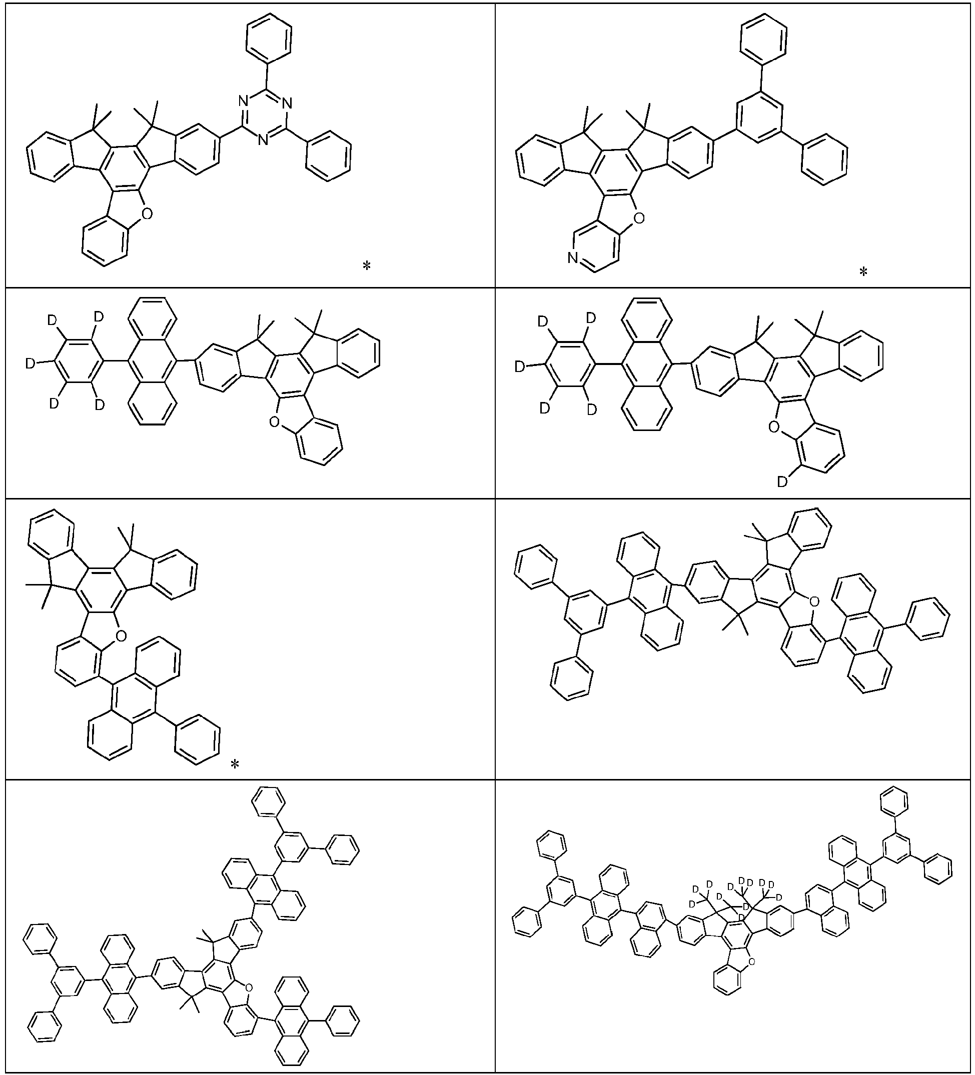

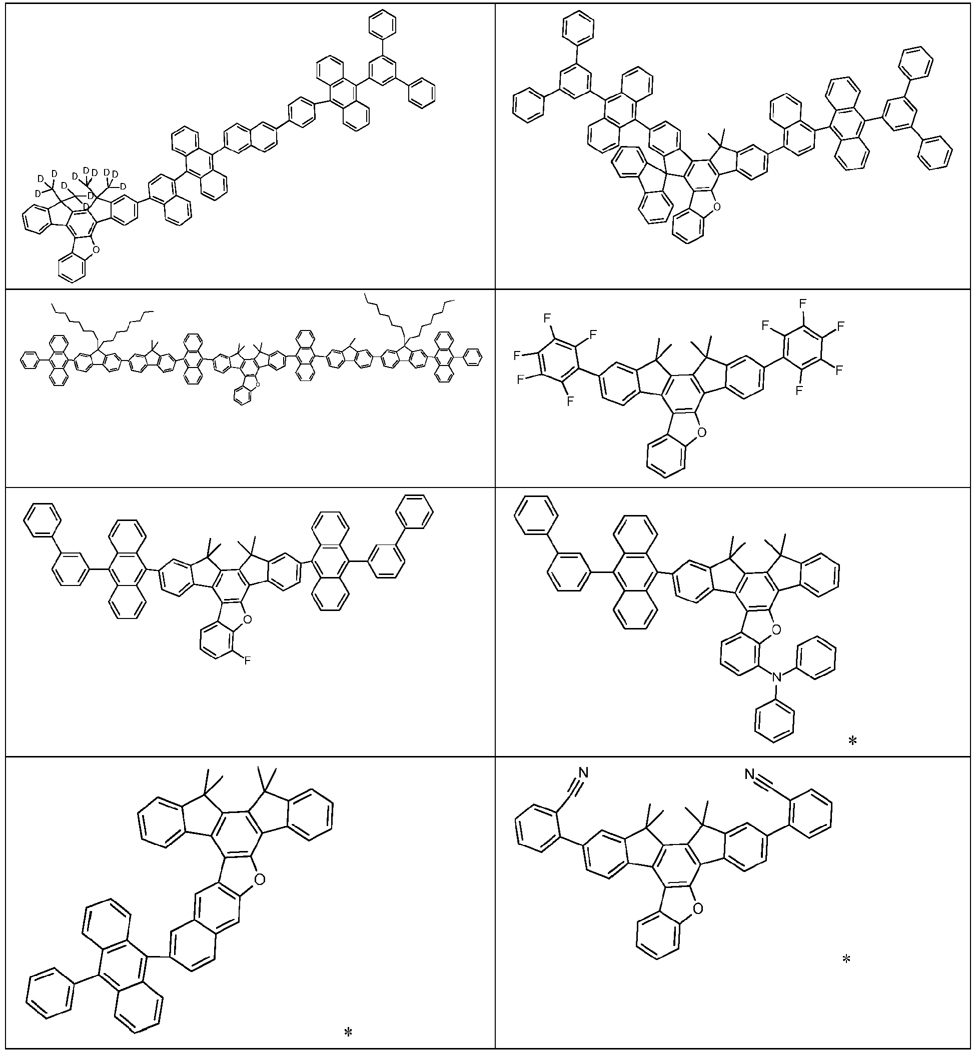

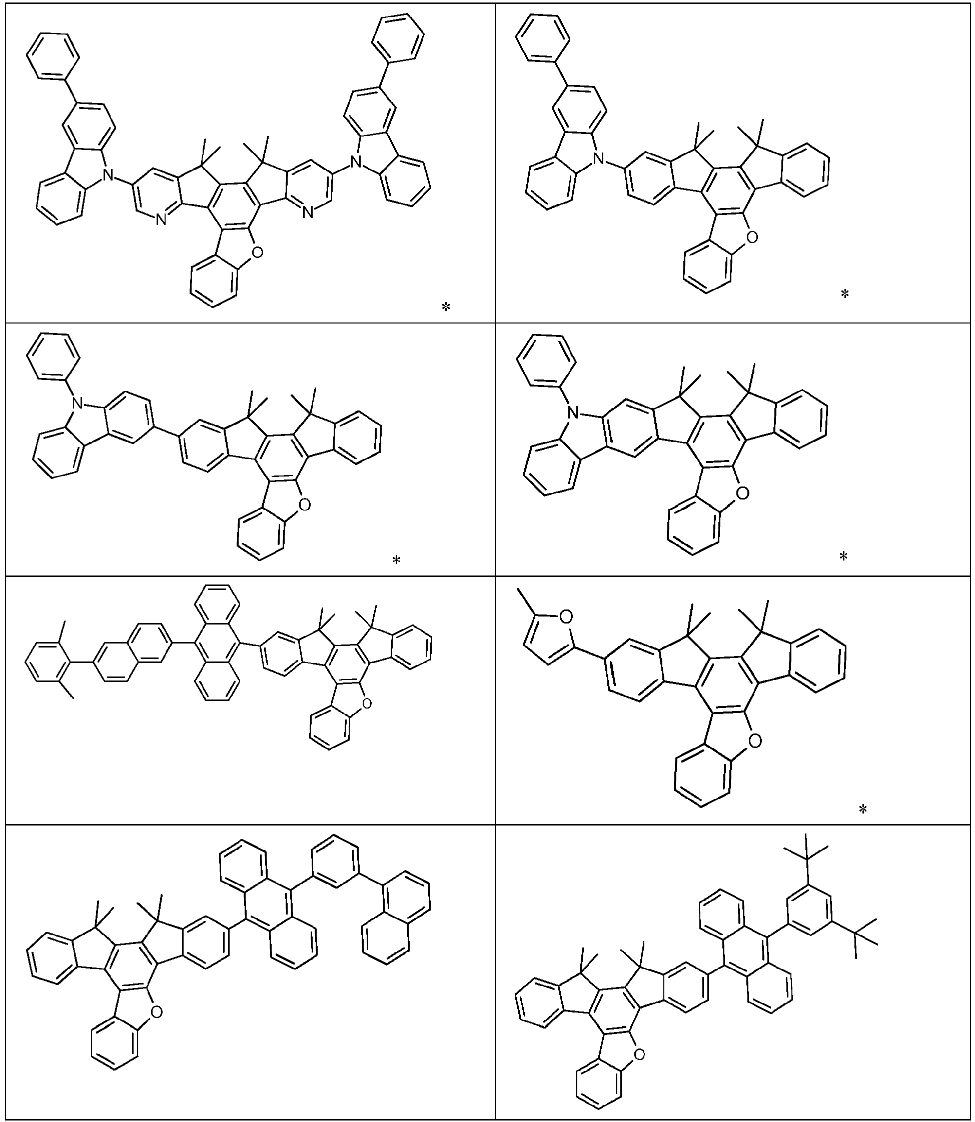

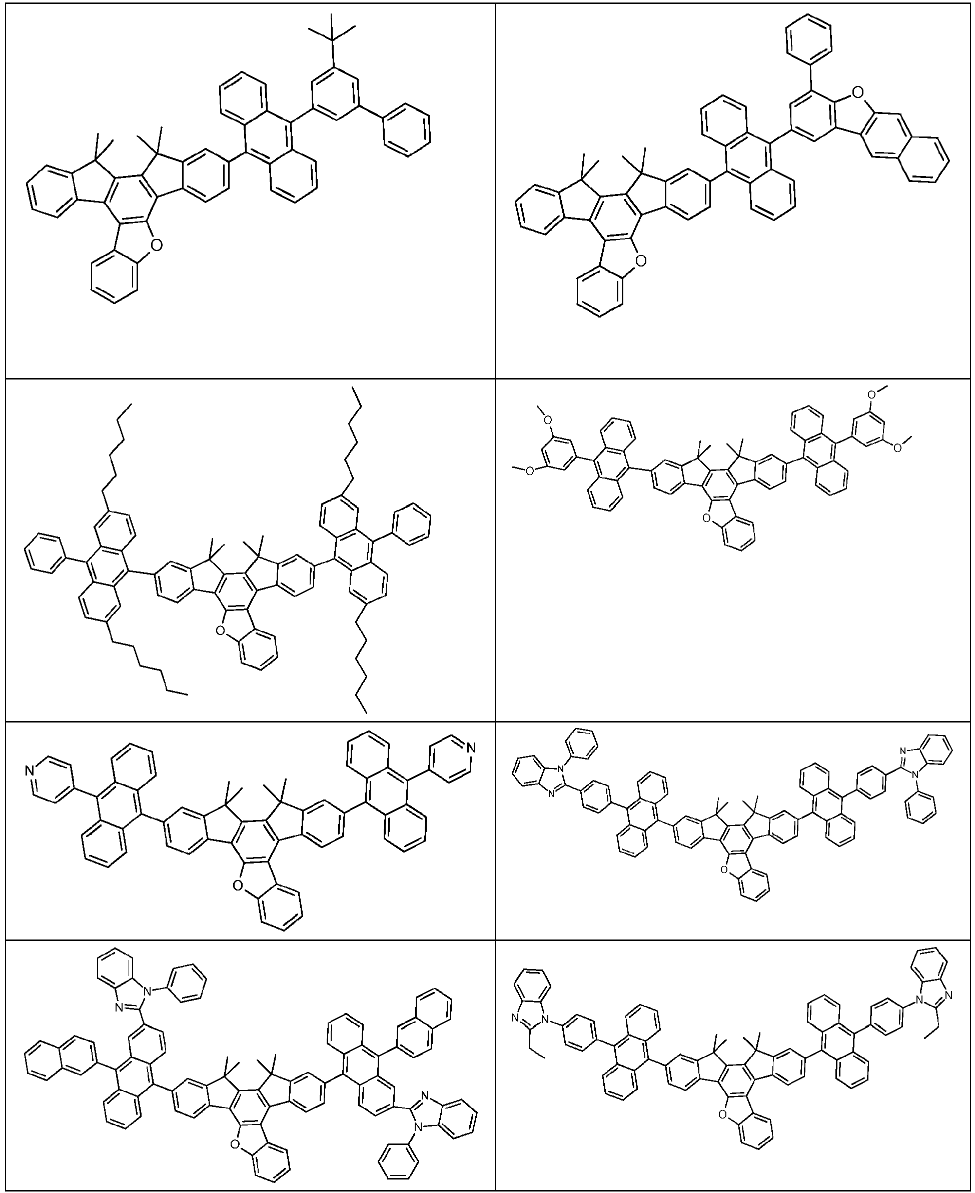

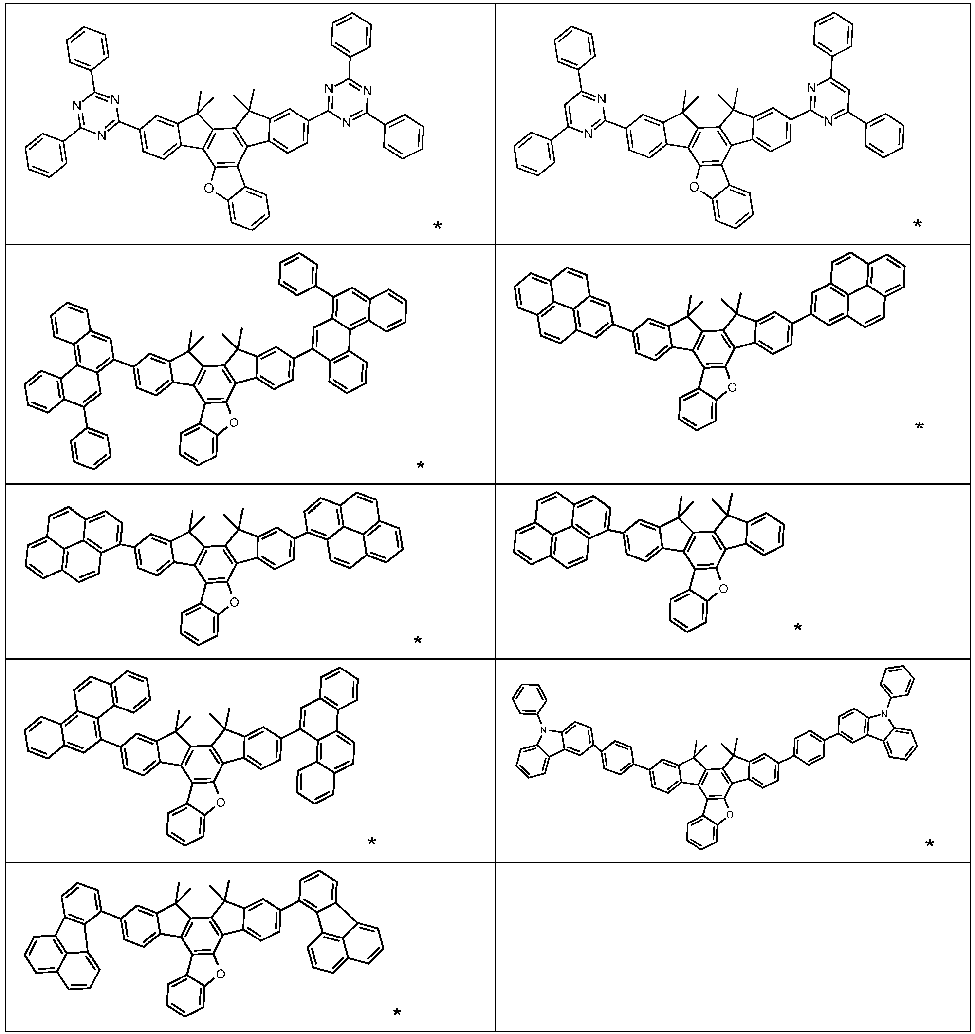

The compounds according to the invention can be prepared by synthesis steps known to the person skilled in the art, such as, for example, bromination, Suzuki coupling, Ullmann coupling, Hartwig-Buchwald coupling, etc. An example of a suitable synthesis process is depicted in general terms in Schemes 1 to 3 below. 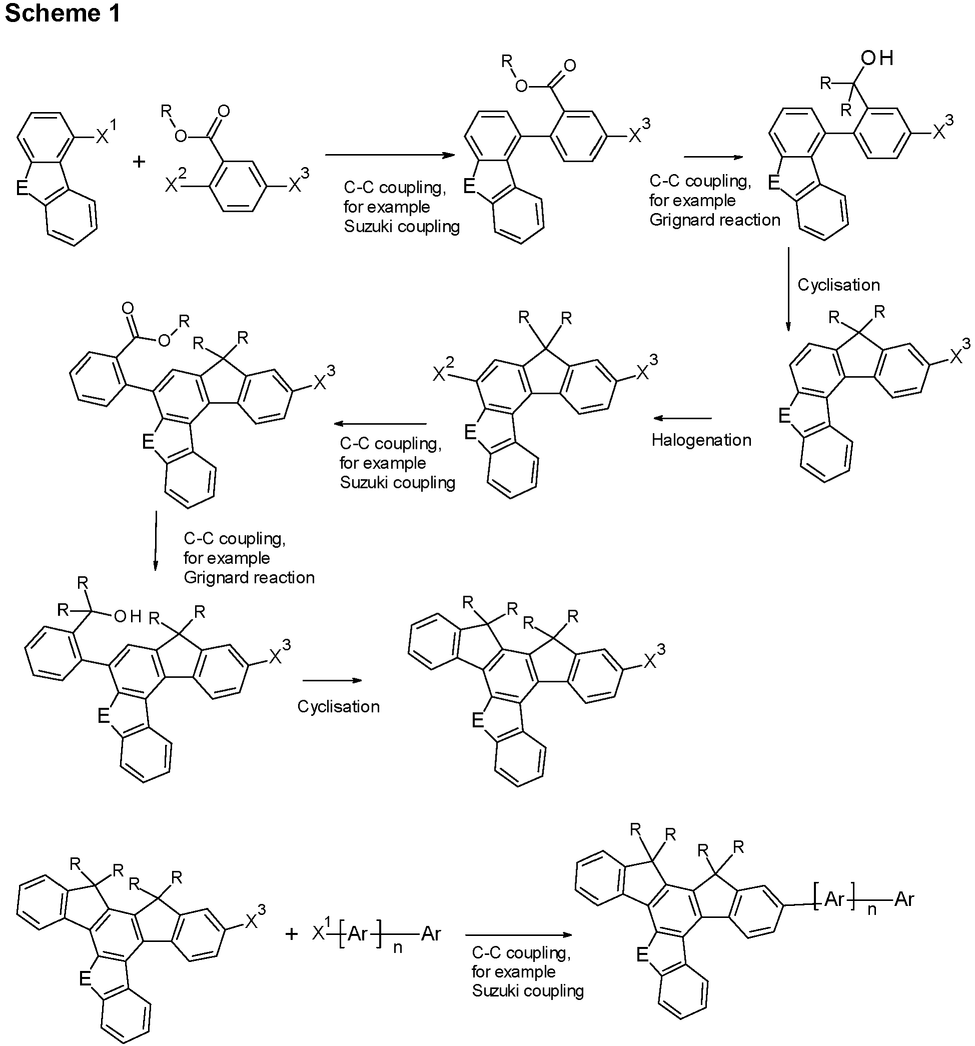
E is O or S or C(R)₂, where R is a substituent
X¹ and X³ are leaving groups (like halogens or boronic acid derivatives)
Ar, Ar' are an aromatic or heteroaromatic ring system (identical or different)
n is an integer like 0, 1, 2, 3 or 4

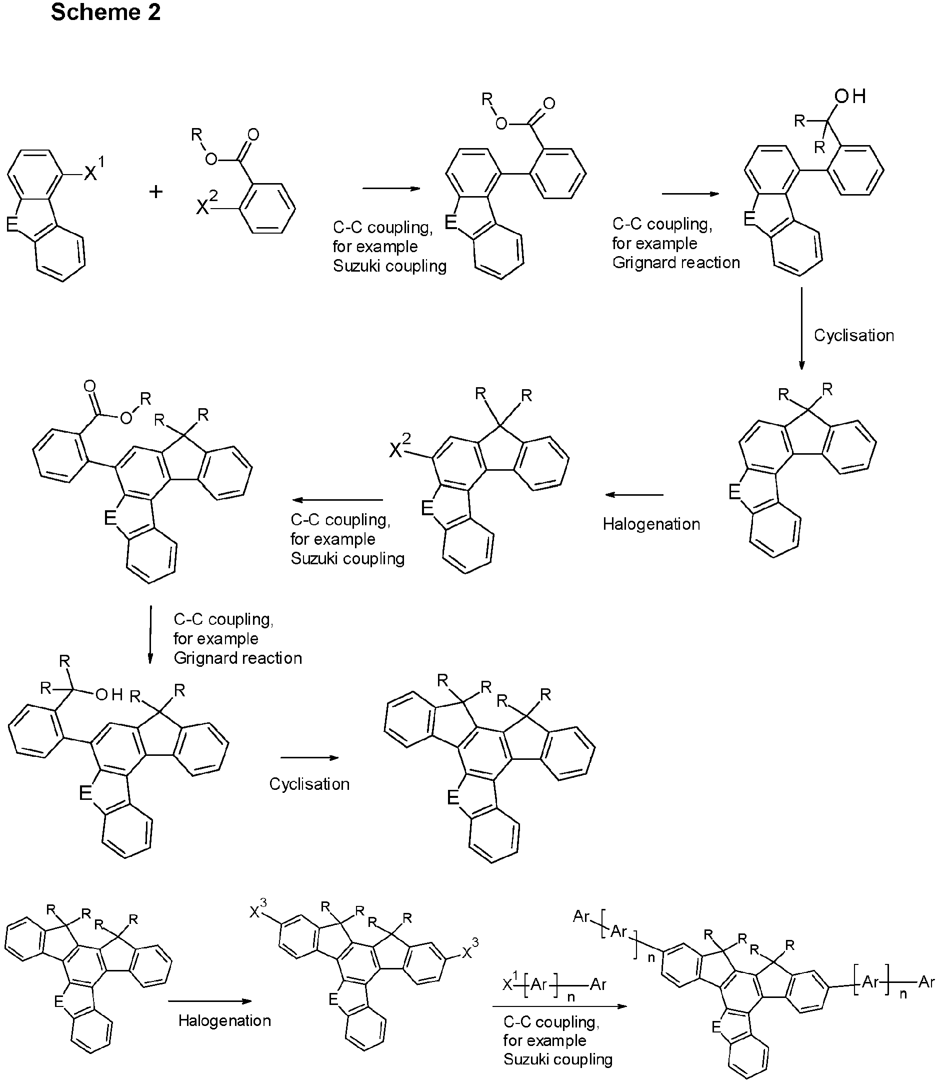

E is O or S or C(R)₂, where R is a substituent
X¹ and X³ are leaving groups (like halogens or boronic acid derivatives)
Ar, Ar' are an aromatic or heteroaromatic ring system (identical or different)
n is an integer like 0, 1, 2, 3 or 4

The compounds of the invention may be synthesized as described above, where a truxene derivative comprising a divalent bridge -O- or -S- is first synthesized using as a precursor a dibenzofuran or dibenzothiophene derivative comprising a reactive leaving group (like halogen or boronic acid) and at least one aromatic or heteroaromatic ring system is bonded to the truxene derivative via a C-C coupling reaction.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol - monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The present invention therefore furthermore relates to a formulation comprising a compound according to the invention and at least one further compound. The further compound may be, for example, a solvent, in particular one of the above-mentioned solvents or a mixture of these solvents. However, the further compound may also be at least one further organic or inorganic compound which is likewise employed in the electronic device, for example an emitting compound, in particular a phosphorescent dopant, and/or a further matrix material. Suitable emitting compounds and further matrix materials are indicated below in connection with the organic electroluminescent device. This further compound may also be polymeric.

The compounds and mixtures according to the invention are suitable for use in an electronic device. An electronic device here is taken to mean a device which comprises at least one layer which comprises at least one organic compound. However, the component here may also comprise inorganic materials or also layers built up entirely from inorganic materials.

The present invention therefore furthermore relates to the use of the compounds or mixtures according to the invention in an electronic device, in particular in an organic electroluminescent device.

The present invention again furthermore relates to an electronic device comprising at least one of the compounds or mixtures according to the invention mentioned above. The preferences stated above for the compound also apply to the electronic devices.

The electronic device is preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells, organic optical detectors, organic photoreceptors, organic field-quench devices (O-FQDs), light-emitting electrochemical cells (LECs), organic laser diodes (O-lasers) and "organic plasmon emitting devices" (D. M. Koller et al., Nature Photonics 2008, 1-4), preferably organic electroluminescent devices (OLEDs, PLEDs), in particular phosphorescent OLEDs.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers is present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

The compound according to the invention in accordance with the embodiments indicated above can be employed in various layers, depending on the precise structure and on the substitution. Preference is given to an organic electroluminescent device comprising a compound of the invention, or in accordance with the preferred embodiments, as fluorescent emitter, emitter showing TADF (Thermally Activated Delayed Fluorescence), or host (=matrix material) for fluorescent emitters. Particularly preferred is an organic electroluminescent device comprising a compound of the the invention, or in accordance with the preferred embodiments, as a host (=matrix material) for fluorescent emitters, more particularly for blue-emitting fluorescent compounds.

The compound of the invention is particularly suitable for use as a host for a blue-emitting fluorescent compound. The electronic device concerned may comprise a single emitting layer comprising the compound according to the invention or it may comprise two or more emitting layers. The further emitting layers here may comprise one or more compounds according to the invention or alternatively other compounds.

The compound of the invention is preferably employed as host for a fluorescent emitting compound in an emitting layer. It is preferably employed as a single host for a fluorescent emitting compound or in combination with one or more hosts. A host here is taken to mean a material which is present in the emitting layer, preferably as the principal component, and which does not emit light on operation of the device.

The proportion of the emitting compound in the mixture of the emitting layer is between 0.1 and 50.0%, preferably between 0.5 and 20.0%, particularly preferably between 1.0 and 10.0%. Correspondingly, the proportion of the host or hosts is between 50.0 and 99.9%, preferably between 80.0 and 99.5%, particularly preferably between 90.0 and 99.0%.

The specifications of the proportions in % are, for the purposes of the present application, taken to mean % by vol. if the compounds are applied from the gas phase and % by weight if the compounds are applied from solution.

Preferred hosts for use in combination with the compounds of the invention as co-host are selected from the classes of the oligoarylenes (for example 2,2',7,7'-tetraphenylspirobifluorene in accordance with EP 676461 or dinaphthylanthracene), in particular the oligoarylenes containing condensed aromatic groups, the oligoarylenevinylenes (for example DPVBi or spiro-DPVBi in accordance with EP 676461), the polypodal metal complexes (for example in accordance with WO 2004/081017), the hole-conducting compounds (for example in accordance with WO 2004/058911), the electron-conducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (for example in accordance with WO 2006/117052) or the benzanthracenes (for example in accordance with WO 2008/145239). Particularly preferred matrix materials are selected from the classes of the oligoarylenes, comprising naphthalene, anthracene, benzanthracene and/or pyrene or atropisomers of these compounds, the oligoarylenevinylenes, the ketones, the phosphine oxides and the sulfoxides. Very particularly preferred matrix materials are selected from the classes of the oligoarylenes, comprising anthracene, benzanthracene, benzophenanthrene and/or pyrene or atropisomers of these compounds. An oligoarylene in the sense of this invention is intended to be taken to mean a compound in which at least three aryl or arylene groups are bonded to one another.

Particularly preferred hosts for use in combination with the compounds of the the invention in the emitting layer are depicted in the following table.

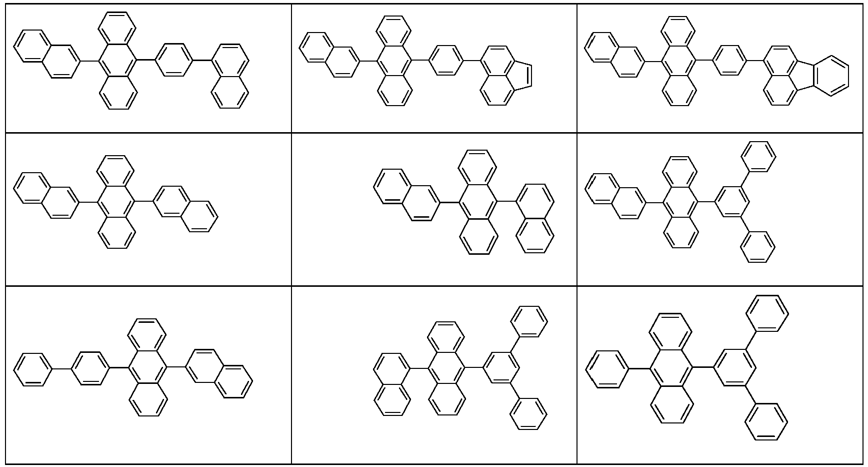

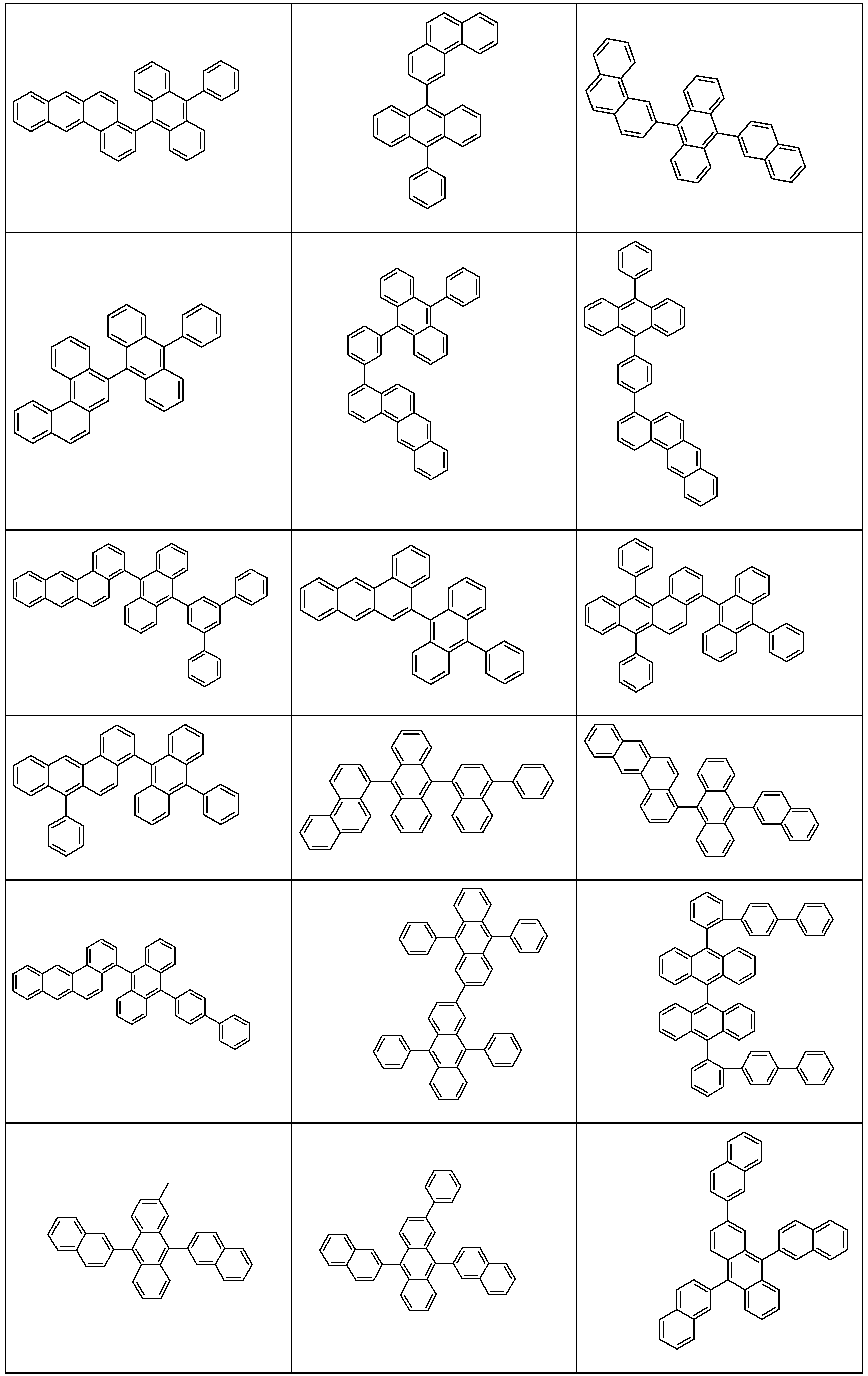

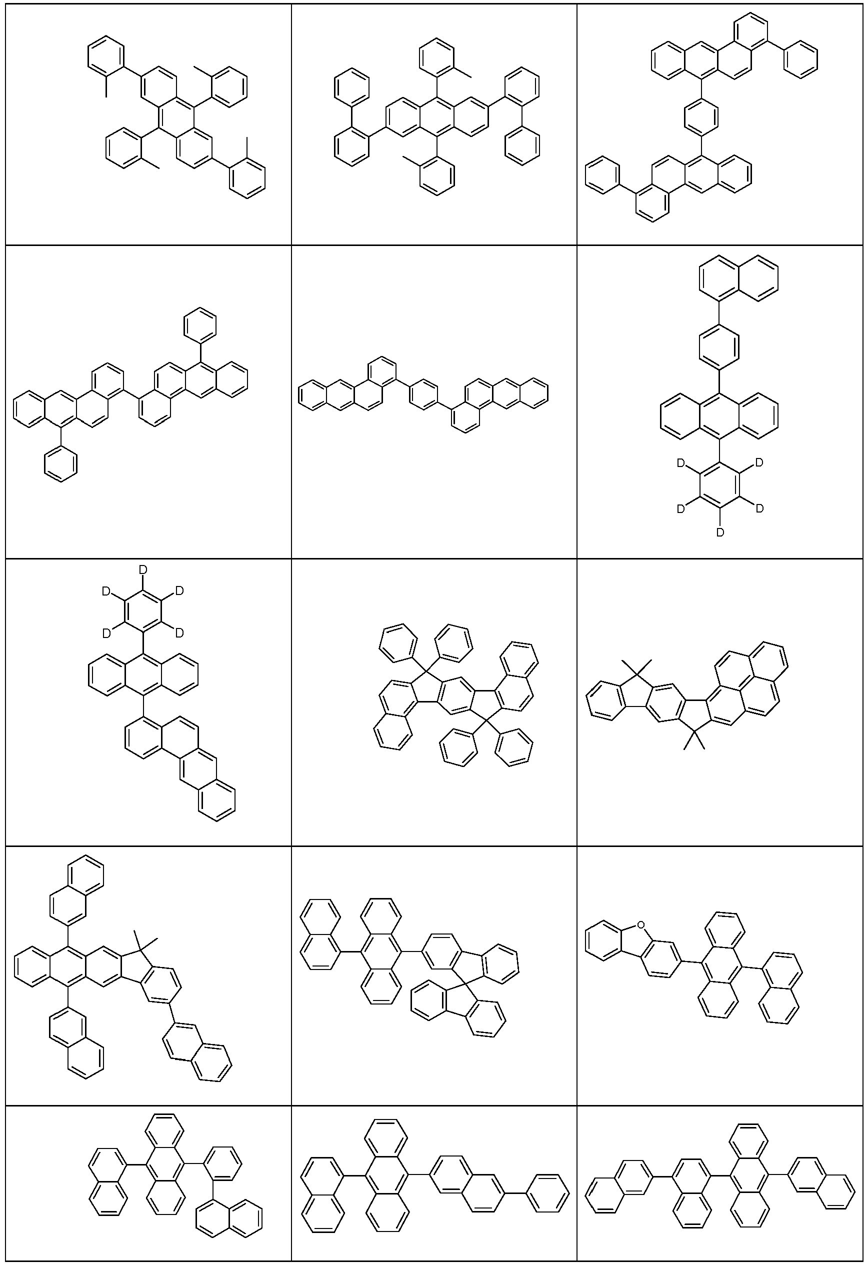

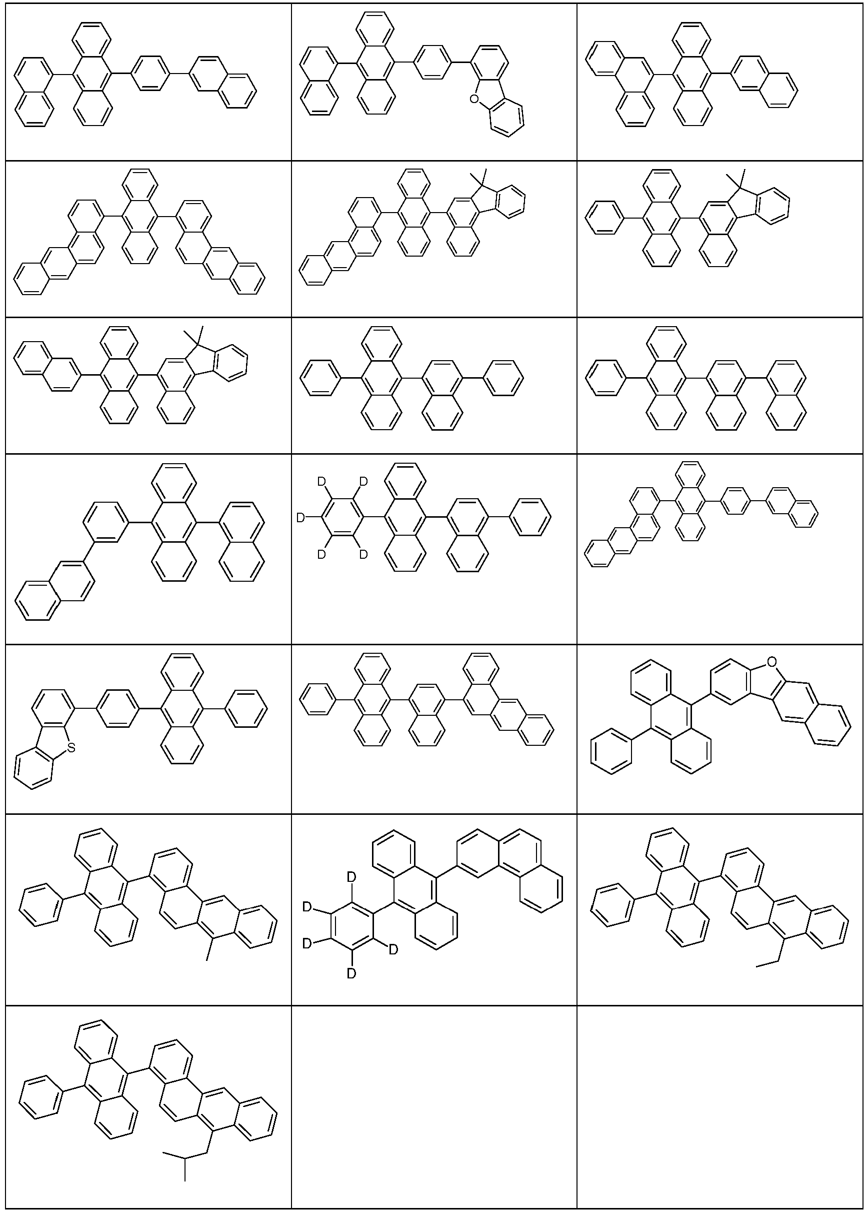

Preferred fluorescent emitters for use in combination with the compounds of the invention are selected from the class of the arylamines. An arylamine in the sense of this invention is taken to mean a compound which contains three substituted or unsubstituted aromatic or heteroaromatic ring systems bonded directly to the nitrogen. At least one of these aromatic or heteroaromatic ring systems is preferably a condensed ring system, particularly preferably having at least 14 aromatic ring atoms. Preferred examples thereof are aromatic anthracenamines, aromatic anthracenediamines, aromatic pyrenamines, aromatic pyrenediamines, aromatic chrysenamines or aromatic chrysenediamines. An aromatic anthracenamine is taken to mean a compound in which one diarylamino group is bonded directly to an anthracene group, preferably in the 9-position. An aromatic anthracene-diamine is taken to mean a compound in which two diarylamino groups are bonded directly to an anthracene group, preferably in the 9,10-position. Aromatic pyrenamines, pyrenediamines, chrysenamines and chrysenediamines are defined analogously thereto, where the diarylamino groups are preferably bonded to the pyrene in the 1-position or in the 1,6-position. Further preferred emitters are indenofluorenamines or indenofluorene-diamines, for example in accordance with WO 2006/108497 or WO 2006/ 122630, benzoindenofluorenamines or benzoindenofluorenediamines, for example in accordance with WO 2008/006449, and dibenzoindenofluorenamines or dibenzoindenofluorenediamines, for example in accordance with WO 2007/140847, and the indenofluorene derivatives containing condensed aryl groups which are disclosed in WO 2010/012328. Still further preferred emitters are benzanthracene derivatives as disclosed in WO 2015/158409, anthracene derivatives as disclosed in WO 2017/036573, fluorene dimers like in WO 2016/150544 or phenoxazine derivatives as disclosed in WO 2017/028940 and WO 2017/028941. Preference is likewise given to the pyrenarylamines disclosed in WO 2012/048780 and WO 2013/185871. Preference is likewise given to the benzoindenofluorenamines disclosed in WO 2014/037077, the benzofluorenamines disclosed in WO 2014/106522 and the indenofluorenes disclosed in WO 2014/111269 or WO 2017/036574.

Examples of preferred fluorescent emitting compounds, besides the compounds according to the invention, which can be used in combination with the compounds of the invention in an emitting layer or which can be used in another emitting layer of the same device are depicted in the following table:

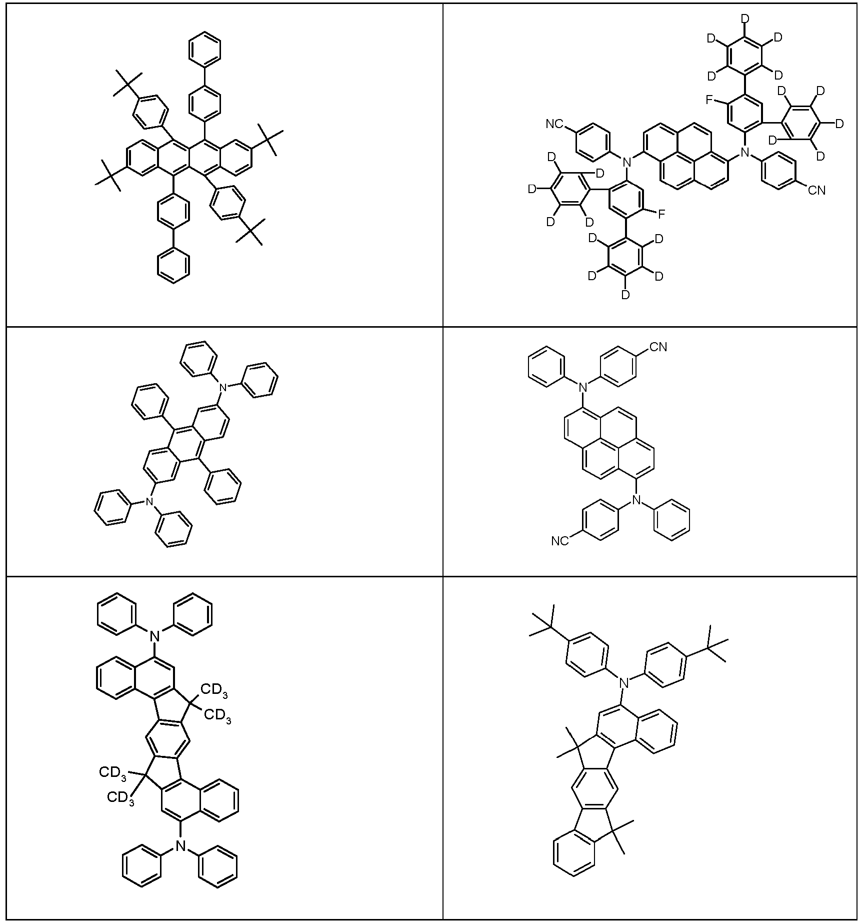

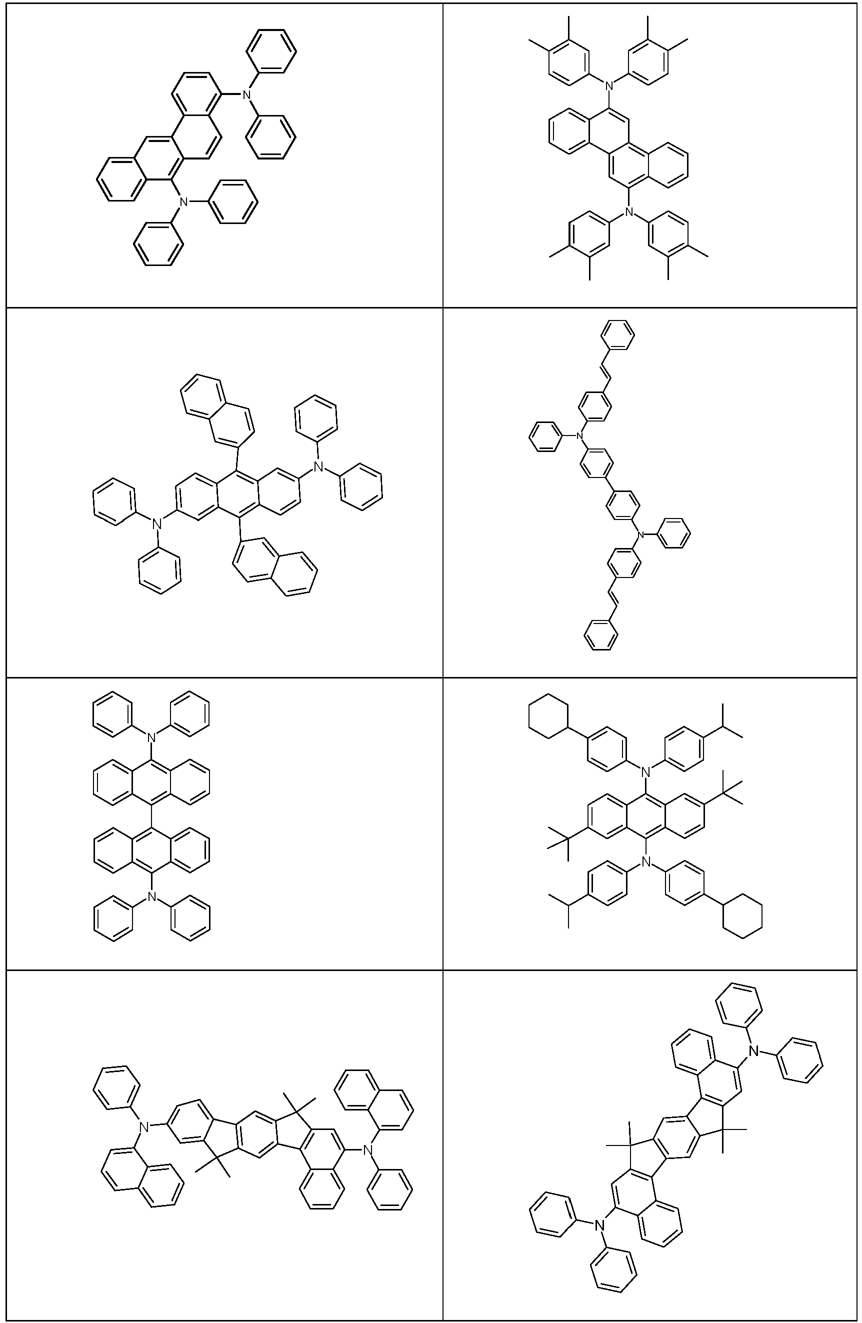

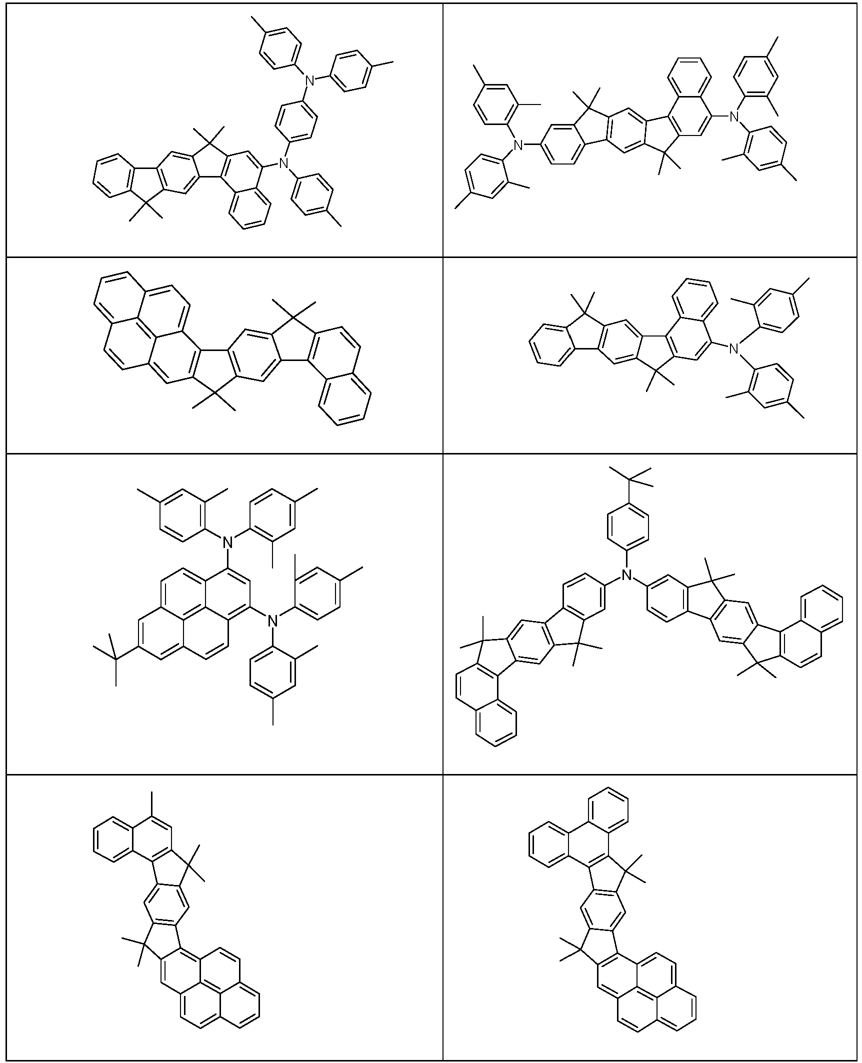

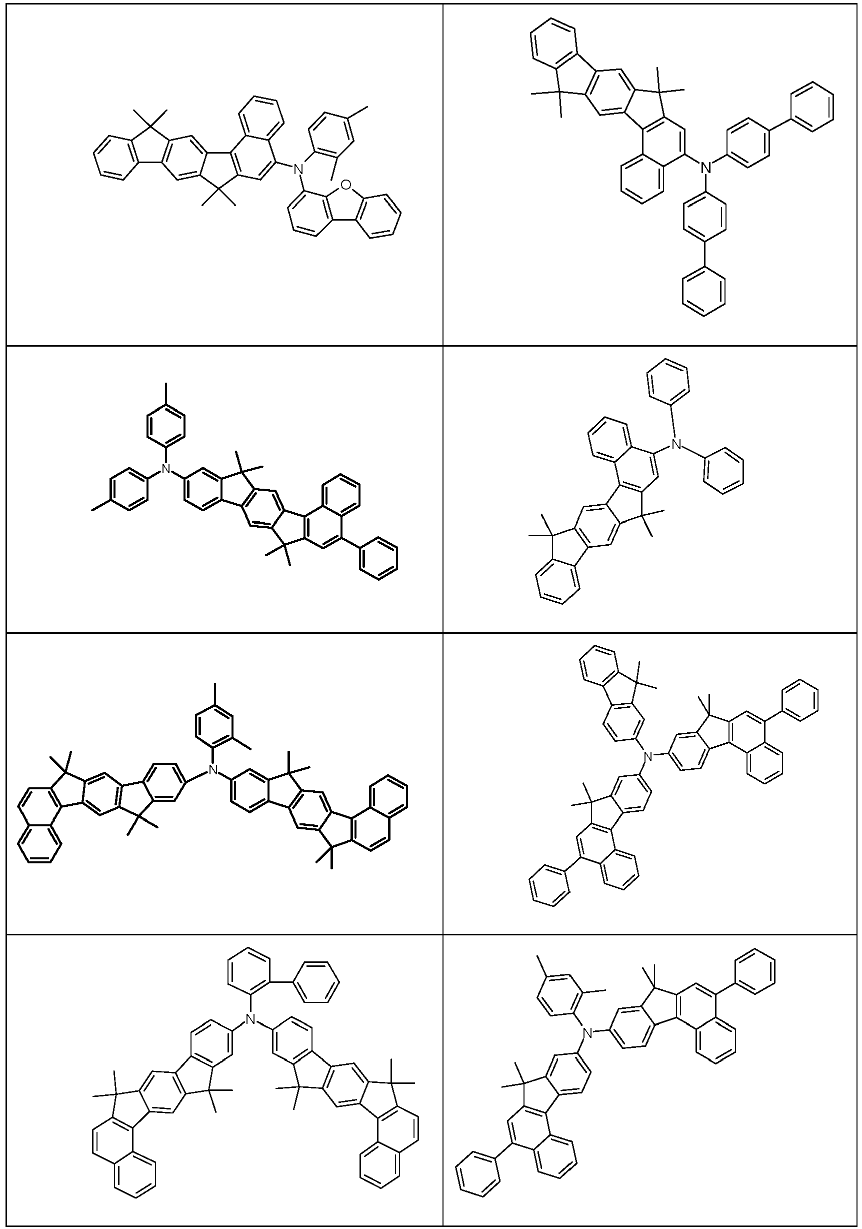

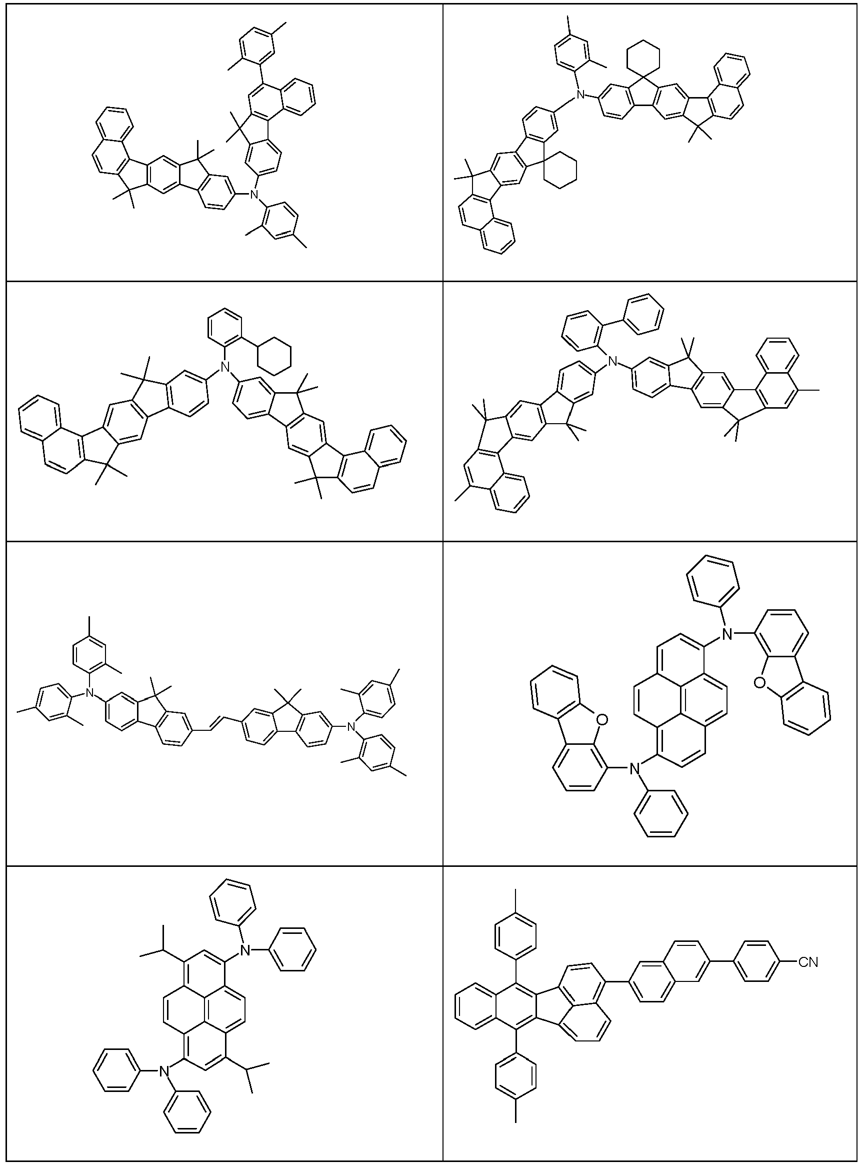

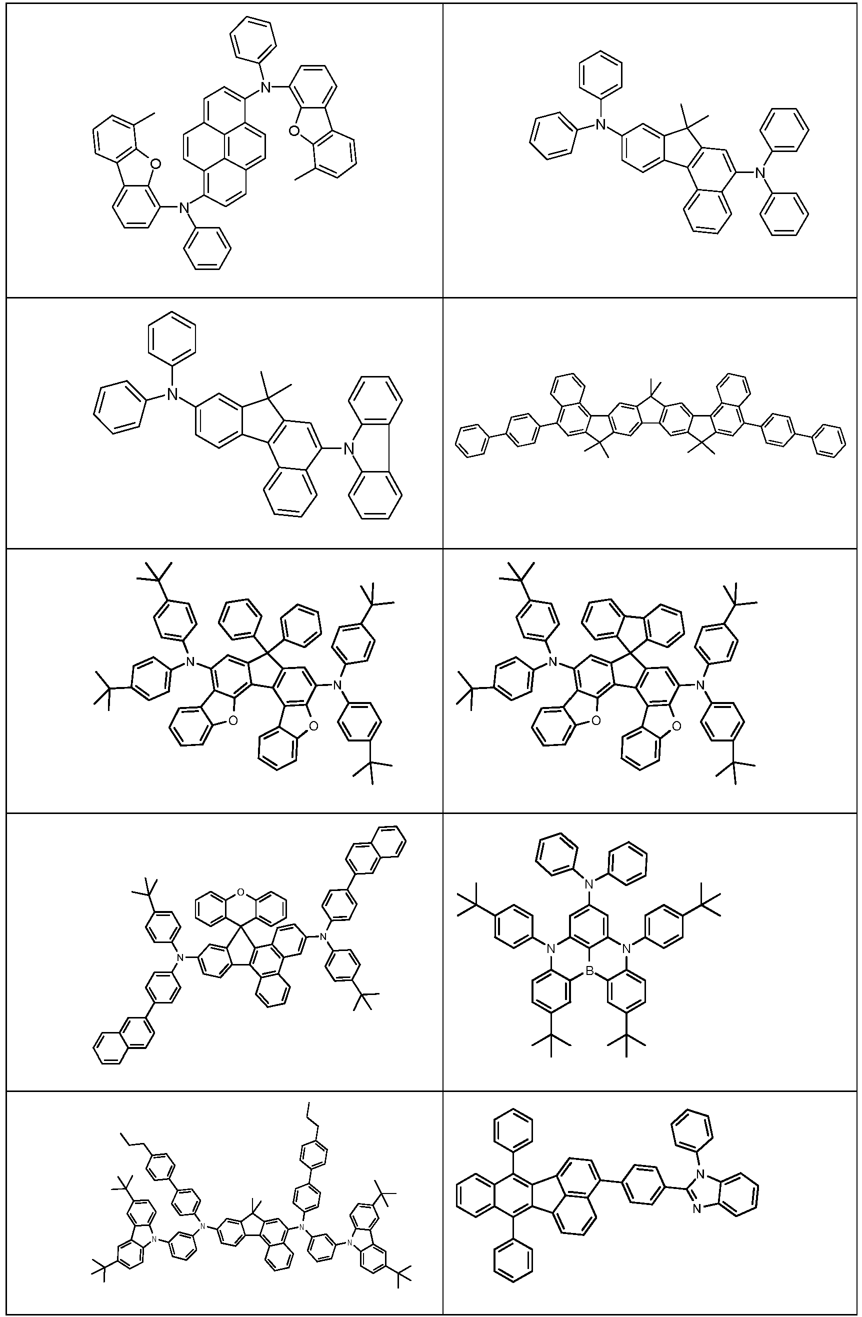

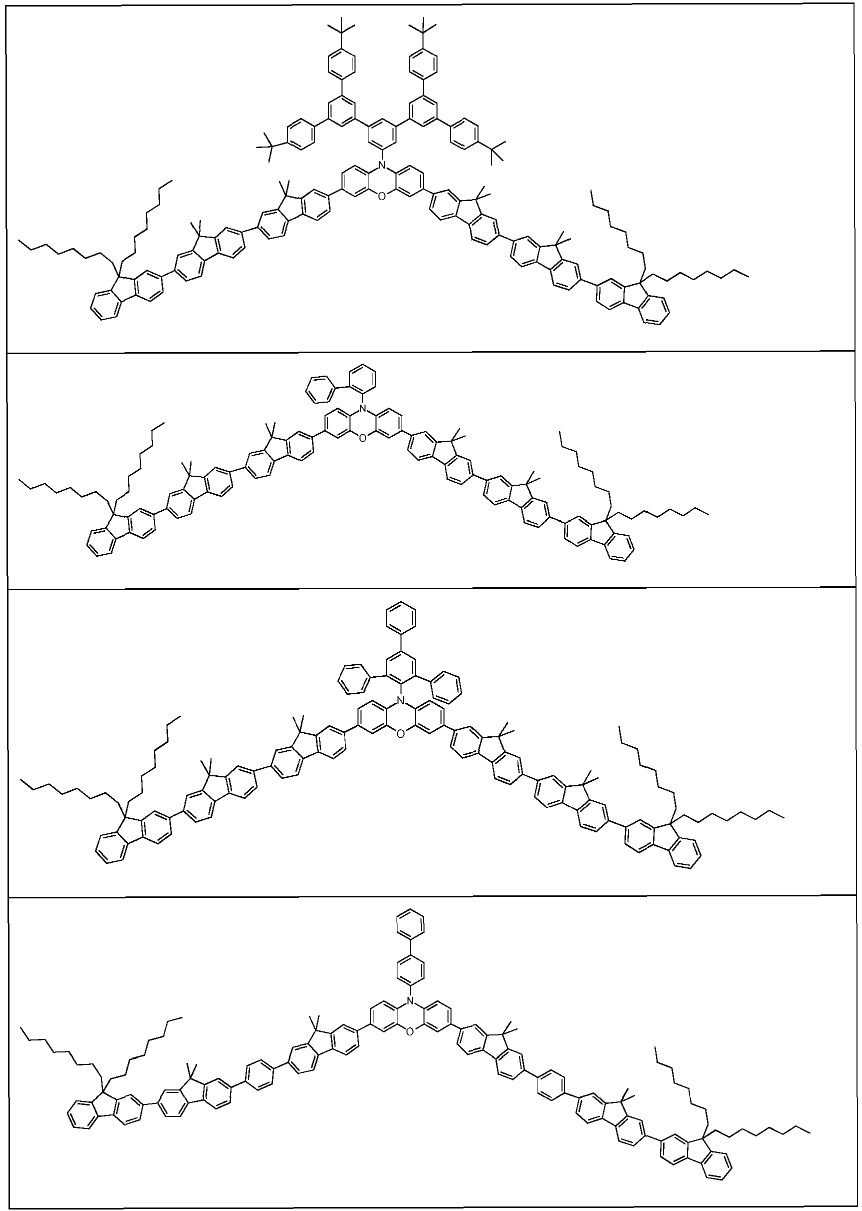

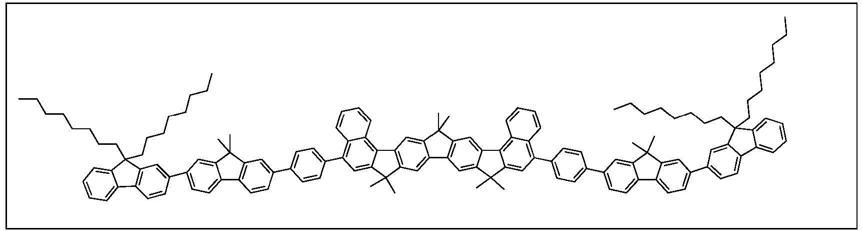

The compounds according to the invention can also be employed in other layers, for example as hole-transport materials in a hole-injection or hole-transport layer or electron-blocking layer or as matrix materials for phosphorescent emitters in an emitting layer.

If the compound of the invention is employed as hole-transport material in a hole-transport layer, a hole-injection layer or an electron-blocking layer, the compound can be employed as pure material, i.e. in a proportion of 100%, in the hole-transport layer, or it can be employed in combination with one or more further compounds. According to a preferred embodiment, the organic layer comprising the compound of the invention then additionally comprises one or more p-dopants. The p-dopants employed in accordance with the present invention are preferably organic electron-acceptor compounds which are able to oxidise one or more of the other compounds of the mixture.

Particularly preferred embodiments of p-dopants are the compounds disclosed in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 and WO 2012/095143.

If the compound of the invention is employed as matrix material in combination with a phosphorescent emitter in an emitting layer, the phosphorescent emitter is preferably selected from the classes and embodiments of phosphorescent emitters indicated below. Furthermore, one or more further matrix materials are preferably present in the emitting layer in this case. So-called mixed-matrix systems of this type preferably comprise two or three different matrix materials, particularly preferably two different matrix materials. It is preferred here for one of the two materials to be a material having hole-transporting properties and for the other material to be a material having electron-transporting properties. The compound of the invention is preferably the material having hole-transporting properties.

However, the desired electron-transporting and hole-transporting properties of the mixed-matrix components may also be combined mainly or completely in a single mixed-matrix component, where the further mixed-matrix component or components satisfy other functions. The two different matrix materials may be present here in a ratio of 1:50 to 1:1, preferably 1:20 to 1:1, particularly preferably 1:10 to 1:1 and very particularly preferably 1:4 to 1:1. Mixed-matrix systems are preferably employed in phosphorescent organic electroluminescent devices. Further details on mixed-matrix systems are contained, inter alia, in the application WO 2010/108579.

Particularly suitable matrix materials which can be used as matrix components of a mixed-matrix system in combination with the compounds according to the invention are selected from the preferred matrix materials for phosphorescent emitters indicated below or the preferred matrix materials for fluorescent emitters, depending on what type of emitter compound is employed in the mixed-matrix system.

Generally preferred classes of material for use as corresponding functional materials in the organic electroluminescent devices according to the invention are indicated below.

Suitable phosphorescent emitters are, in particular, compounds which emit light, preferably in the visible region, on suitable excitation and in addition contain at least one atom having an atomic number greater than 20, preferably greater than 38 and less than 84, particularly preferably greater than 56 and less than 80. The phosphorescent emitters used are preferably compounds which contain copper, molybdenum, tungsten, rhenium, ruthenium, osmium, rhodium, iridium, palladium, platinum, silver, gold or europium, in particular compounds which contain iridium, platinum or copper.

For the purposes of the present invention, all luminescent iridium, platinum or copper complexes are regarded as phosphorescent compounds.

Examples of the phosphorescent emitters described above are revealed by the applications WO 2000/70655, WO 2001/41512, WO 2002/02714, WO 2002/15645, EP 1191613, EP 1191612, EP 1191614, WO 2005/ 033244, WO 2005/019373 and US 2005/0258742. In general, all phosphorescent complexes as used in accordance with the prior art for phosphorescent OLEDs and as are known to the person skilled in the art in the area of organic electroluminescent devices are suitable for use in the devices according to the invention. The person skilled in the art will also be able to employ further phosphorescent complexes without inventive step in combination with the compounds according to the invention in OLEDs. Preferred matrix materials for phosphorescent emitters are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example in accordance with WO 2007/063754 or WO 2008/056746, indenocarbazole derivatives, for example in accordance with WO 2010/136109, WO 2011/ 000455 or WO 2013/041176, azacarbazole derivatives, for example in accordance with EP 1617710, EP 1617711, EP 1731584, JP 2005/347160, bipolar matrix materials, for example in accordance with WO 2007/137725, silanes, for example in accordance with WO 2005/111172, azaboroles or boronic esters, for example in accordance with WO 2006/117052, triazine derivatives, for example in accordance with WO 2010/015306, WO 2007/063754 or WO 2008/056746, zinc complexes, for example in accordance with EP 652273 or WO 2009/062578, diazasilole or tetraazasilole derivatives, for example in accordance with WO 2010/054729, diazaphosphole derivatives, for example in accordance with WO 2010/054730, bridged carbazole derivatives, for example in accordance with US 2009/0136779, WO 2010/050778, WO 2011/042107, WO 2011/088877 or WO 2012/ 143080, triphenylene derivatives, for example in accordance with WO 2012/048781, or lactams, for example in accordance with WO 2011/116865 or WO 2011/137951.

Besides the compounds according to the invention, suitable charge-transport materials, as can be used in the hole-injection or hole-transport layer or electron-blocking layer or in the electron-transport layer of the electronic device according to the invention, are, for example, the compounds disclosed in Y. Shirota et al., Chem. Rev. 2007, 107(4), 953-1010, or other materials as are employed in these layers in accordance with the prior art.

Materials which can be used for the electron-transport layer are all materials as are used in accordance with the prior art as electron-transport materials in the electron-transport layer. Particularly suitable are aluminium complexes, for example Alq₃, zirconium complexes, for example Zrq₄, lithium complexes, for example Liq, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Furthermore suitable materials are derivatives of the above-mentioned compounds, as disclosed in JP 2000/053957, WO 2003/060956, WO 2004/028217, WO 2004/080975 and WO 2010/072300.

Preferred hole-transport materials which can be used in a hole-transport, hole-injection or electron-blocking layer in the electroluminescent device according to the invention are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluorenamines (for example in accordance with WO 08/006449), dibenzoindenofluorenamines (for example in accordance with WO 07/140847), spirobifluorenamines (for example in accordance with WO 2012/034627 or WO 2013/120577), fluorenamines (for example in accordance with the as yet unpublished applications EP 12005369.9, EP 12005370.7 and EP 12005371.5), spirodibenzopyranamines (for example in accordance with WO 2013/083216) and dihydroacridine derivatives (for example in accordance with WO 2012/150001). The compounds according to the invention can also be used as hole-transport materials.

The cathode of the organic electroluminescent device preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys comprising an alkali metal or alkaline-earth metal and silver, for example an alloy comprising magnesium and silver. In the case of multilayered structures, further metals which have a relatively high work function, such as, for example, Ag or Al, can also be used in addition to the said metals, in which case combinations of the metals, such as, for example, Ca/Ag, Mg/Ag or Ag/Ag, are generally used. It may also be preferred to introduce a thin interlayer of a material having a high dielectric constant between a metallic cathode and the organic semiconductor. Suitable for this purpose are, for example, alkali metal fluorides or alkaline-earth metal fluorides, but also the corresponding oxides or carbonates (for example LiF, Li₂O, BaF₂, MgO, NaF, CsF, Cs₂CO₃, etc.). Furthermore, lithium quinolinate (LiQ) can be used for this purpose. The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOₓ, Al/PtOₓ) may also be preferred. For some applications, at least one of the electrodes must be transparent or partially transparent in order to facilitate either irradiation of the organic material (organic solar cells) or the coupling-out of light (OLEDs, O-lasers). Preferred anode materials here are conductive mixed metal oxides. Particular preference is given to indium tin oxide (ITO) or indium zinc oxide (IZO). Preference is furthermore given to conductive, doped organic materials, in particular conductive doped polymers.

The device is appropriately (depending on the application) structured, provided with contacts and finally sealed, since the lifetime of the devices according to the invention is shortened in the presence of water and/or air.

In a preferred embodiment, the organic electroluminescent device according to the invention is characterised in that one or more layers are coated by means of a sublimation process, in which the materials are applied by vapour deposition in vacuum sublimation units at an initial pressure of less than 10⁻⁵ mbar, preferably less than 10⁻⁶ mbar. However, it is also possible here for the initial pressure to be even lower, for example less than 10⁻⁷ mbar.

Preference is likewise given to an organic electroluminescent device, characterised in that one or more layers are coated by means of the OVPD (organic vapour phase deposition) process or with the aid of carrier-gas sublimation, in which the materials are applied at a pressure of between 10⁻⁵ mbar and 1 bar. A special case of this process is the OVJP (organic vapour jet printing) process, in which the materials are applied directly through a nozzle and are thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, screen printing, flexographic printing, nozzle printing or offset printing, but particularly preferably LITI (light induced thermal imaging, thermal transfer printing) or ink-jet printing. Soluble compounds according to the invention are necessary for this purpose. High solubility can be achieved through suitable substitution of the compounds.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition.

These processes are generally known to the person skilled in the art and can be applied by him without inventive step to organic electroluminescent devices comprising the compounds according to the invention.

In accordance with the invention, the electronic devices comprising one or more compounds according to the invention can be employed in displays, as light sources in lighting applications and as light sources in medical and/or cosmetic applications (for example light therapy).

The invention will now be explained in greater detail by the following examples, without wishing to restrict it thereby.

### A) Syntheses Examples

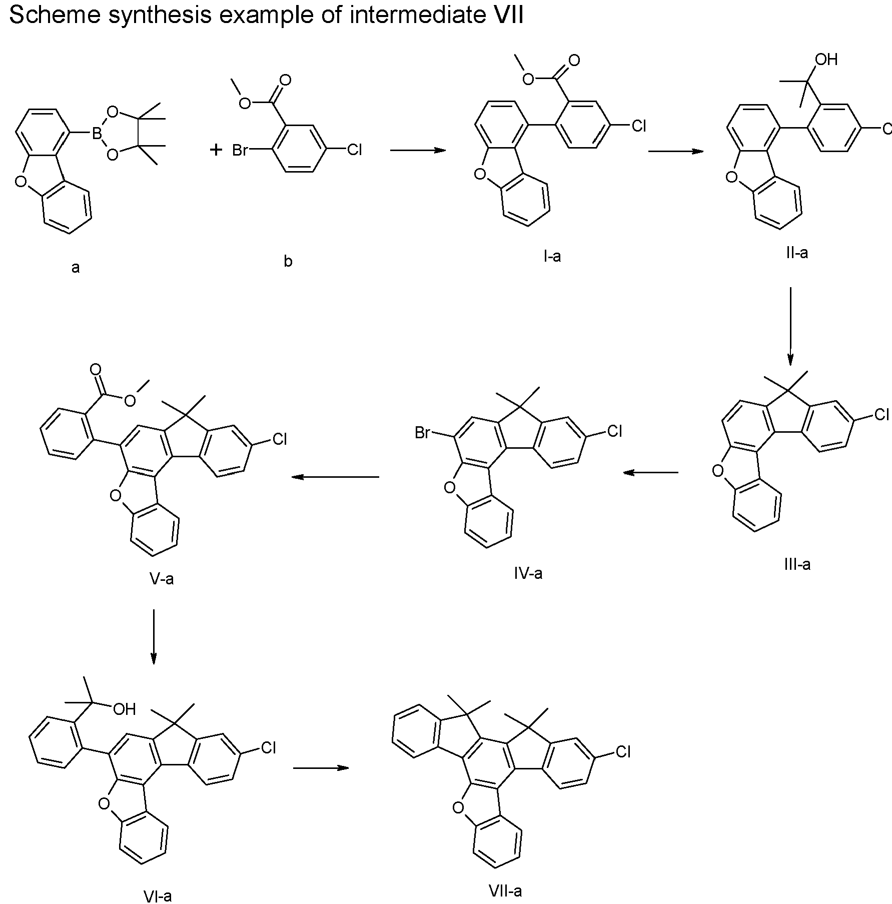

### Synthesis Intermediate I-a:

117.9 g (401 mmol) starting material **a,** 100 g (401 mmol) starting material **b** and 203.1 g (882 mmol) potassium phosphate monohydrate are mixed in 1.6 L toluene/water/dioxane (2:1:1) and degassed. To the mixture, palladium acetate (0.9 g, 4 mmol) and tri-ortho-tolylphosphine (2.44 g, 8 mmol) are added and the mixture is stirred at reflux for 16 h. After cooling the mixture to room temperature, the phases are separated. The aqueous phase is further extracted with ethyl acetate (2 x 300 mL). The combined organic phases are washed multiple times with water, dried over sodium sulfate and finally removed in vacuum. The crude is filtered over a plug of SiO₂/Al₂O₃ using ethyl acetate as solvent. After removing the solvent in vacuum, an oil is obtained in quantitative yield.

In a similar manner, the following compounds can also be prepared:

| Example | Starting material | Starting material | Intermediate I |
|---|---|---|---|
| I-b | 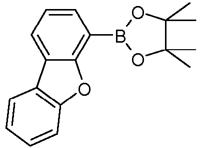 | 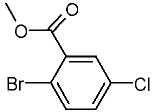 | 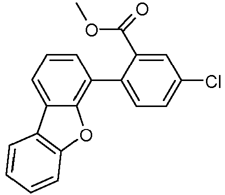 |
| I-c | 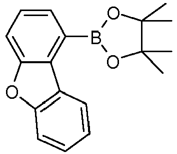 | 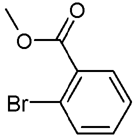 | 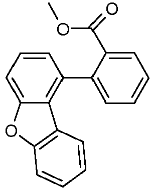 |

### Synthesis Intermediate II-a:

MeMgCl (461 mL, 3 M in THF, 1.38 mol) is added dropwise to a pre-cooled THF suspension (0 °C, 1.5 L) of compound **I-a** (135 g, 0.4 mol) and CeCl₃ (199 g, 0.8 mol). After completion of the reaction, a saturated aqueous solution of NH₄Cl is added to quench the excess of MeMgCl, and the organic phase is extracted three times with ethyl acetate. The organic fractions are combined and washed with water and brine, successively. The volatiles were removed in vacuum to yield the desired product. 129 g (96 %).

In a similar manner, the following compounds can also be prepared:

| Example | Intermediate I | Intermediate II |
|---|---|---|
| II-b | 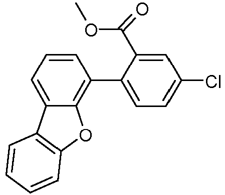 | 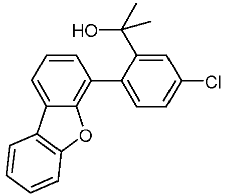 |
| II-c | 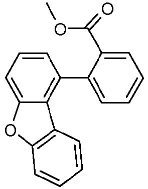 | 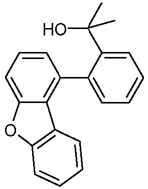 |

### Synthesis Intermediate III-a:

To a solution of compound **II-a** (129 g, 383 mmol) in toluene (1 L), 50 g of Amberlyst-15 are added. The mixture is stirred at reflux overnight. The mixture is cooled down to room temperature and the Amberlyst-15 filtered off. The solvent is removed in vacuum and the crude product is purified by column chromatography (SiO₂, heptane). Yield: 106.2 g (87 %).

In a similar manner, the following compounds can also be prepared:

| Example | Intermediate II | Intermediate III |
|---|---|---|
| III-b | 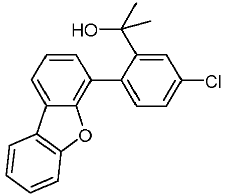 | 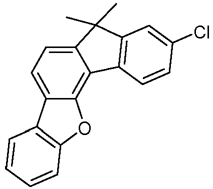 |
| III-c | 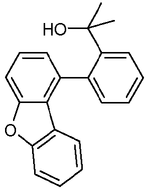 | 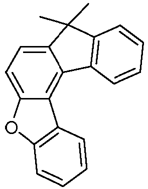 |

### Synthesis Intermediate IV-a:

To a solution of compound **III-a** (100 g, 314 mmol) in CH₂Cl₂ (1.2 L), N-bromosuccinimide (55.83 g, 314 mmol) and HBr (32 % solution in acetic acid, 0.5 mL) are added. The reaction is heated at 30 °C for 4 days. After completion of the reaction, Na₂S₂O₃ (300 mL, saturated aqueous solution) is added and the mixture is stirred vigorously for 30 minutes. The phases are separated and the organic phase is washed several times with water. The solvent is removed in vacuum and the crude product vigorously stirred with ethanol to yield a white solid. Yield: 119.8 g (96%).

In a similar manner, the following compounds can also be prepared:

| Example | Intermediate III | Intermediate IV |
|---|---|---|
| IV-b | 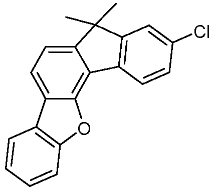 | 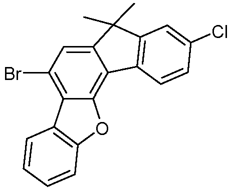 |
| IV-c | 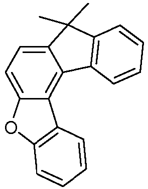 | 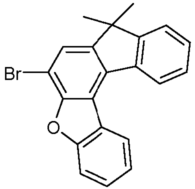 |

### Synthesis Intermediate V-a:

27.5 g (69.1 mmol) **IV-a,** 13.1 g (72.6 mmol) (2-methoxycarbonylphenyl)boronic acid and 35.0 g (152.1 mmol) potassium phosphate monohydrate are mixed in 300 mL toluene and degassed. To the mixture, 2-dicyclohexylphosphino-2',4',6'-triisopropyl-1,1'-biphenyl)[2-(2'-amino-1,1'-biphenyl)]palladium(II) methanesulfonate (1.18 g, 2.07 mmol) is added and the mixture is stirred at reflux for 3 h. After cooling the mixture to room temperature, 300 mL water are added and the phases are separated. The organic phase is washed multiple times with water and the solvent is removed in vacuum. Afterwards, the crude product is purified by column chromatography (SiO₂, toluene). Yield: 28.2 g (90 %).

In a similar manner, the following compounds can also be prepared:

| Exampl es | Intermediate IV | Intermediate V |
|---|---|---|
| V-b | 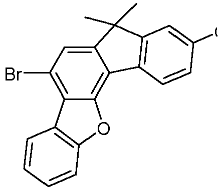 | 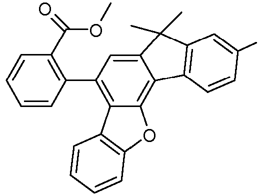 |
| V-c | 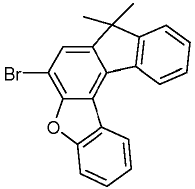 | 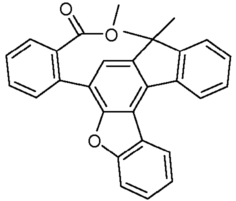 |

### Synthesis of compound V-d:

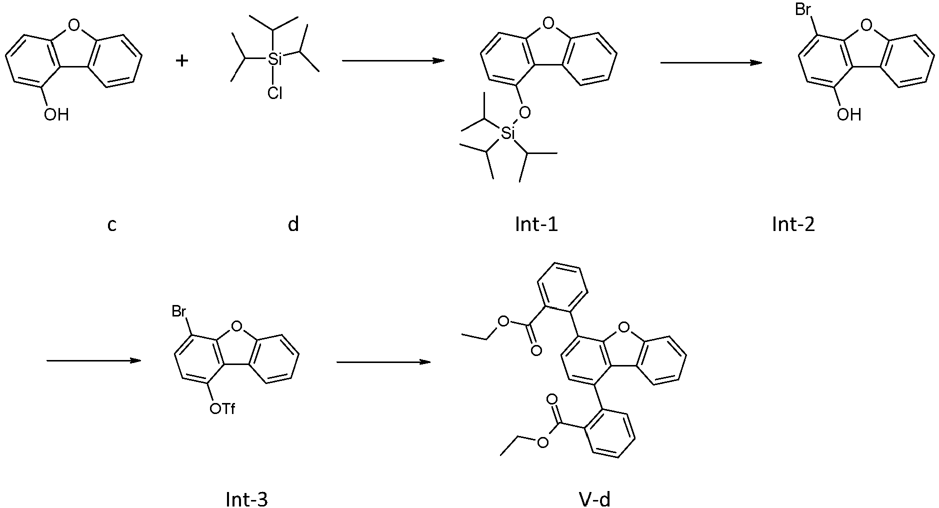

### Synthesis Int-1

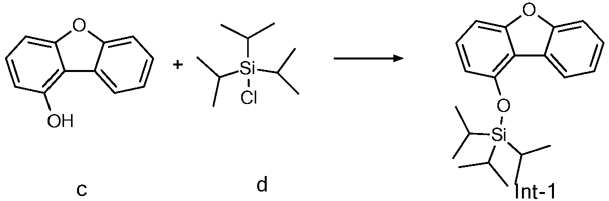

82.0 g (439 mmol) starting material c (CAS registry number 33483-06-6) are dissolved in anhydrous CH₂Cl₂ (2.00 L). Imidazole (59.7 g, 877 mmol) is added, followed by 105 mL (479 mmol) starting material **d** (CAS registry number 13154-24-0). The reaction is stirred at room temperature for 21 h. Afterwards, the reaction mixture is washed with water once, with 20 % NaOH twice and once again with water. The organic layers are dried over anhydrous sodium sulfate, filtered and concentrated in vacuo. **Int-1** is received as an oil in quantitative yield.

### Synthesis Int-2

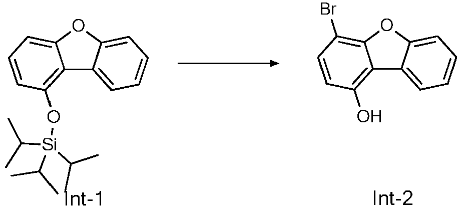

78.6 g (231 mmol) **Int-1** are dissolved in anhydrous THF (2.00 L). 41.1 g (231 mmol) NBS are added at room temperature. The reaction is stirred at room temperature for 17 h. 91.2 g (600 mmol) CsF are added at room temperature and the mixture is stirred for 18 h. The solvent is removed in vacuo and toluene and water are added. The aqueous layer is acidified with 1M HCl to pH 2-3 and extracted with toluene twice. The combined organic layer is washed with water twice, then dried over anhydrous sodium sulfate, filtered and concentrated. The crude product is suspended in cyclohexane (300 ml) and stirred at room temperature overnight. The solid is collected by filtration, washed with cyclohexane and dried in vacuo to give **Int-2** (53.5 g ; 88 %).

### Synthesis Int-3

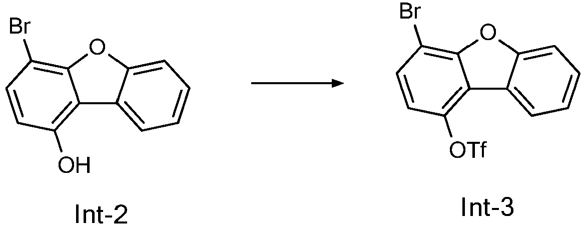

42.0 g (159 mmol) **Int-2** are suspended in anhydrous CH₂Cl₂ (1.00 l). 66.4 ml (478 mmol) triethylamine are added. The solution is cooled to an inner temperature of 4 °C. 34.3 mL (207 mmol) trifluoromethanesulfonic anhydride are added dropwise. The mixture is allowed to warm to room temperature over night. For an aqueous work-up, the mixture is washed with water once, with 1M HCl twice and with water twice. The organic layer is dried over anhydrous sodium sulfate, filtered and concentrated in vacuo to give the crude product as an oil. The crude product is filtered over silica gel (Heptane/THF 9:1) and concentrated to give **Int-3** as a solid (59.7 g; 95 %).

### Synthesis of V-d

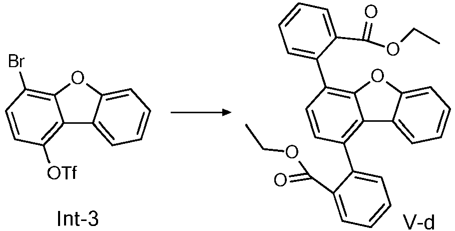

10.0 g (25.3 mmol) **Int-3,** 10.3 g (53.1 mmol) (2-ethoxycarbonylphenyl)-boronic acid (CAS registry number 380430-53-5) and 13.4 g (88 mmol) CsF are suspended in anhydrous THF (250 mL). The mixture is degassed, followed by the addition of 227 mg (1.01 mmol) Pd(OAc)₂ and 831 mg (2.02 mmol) S-Phos. The mixture is stirred at reflux for 36 h. After cooling to room temperature, the solvent is removed and the residue is dissolved in toluene and water. The organic layer is washed with water three times, then once with brine. The organic layer is dried over anhydrous sodium sulfate, filtered and concentrated. The crude product is purified by column chromatography (SiO₂, Heptane/THF) to give **Int-4** (8.2 g; 70 %).

### Synthesis Intermediate VI-a:

Intermediate **VI-a** can be prepared in a similar manner as intermediate **II-a** starting from **V-a** (28.0 g, 61.8 mmol), MeMgCl (71 mL, 3 M in THF, 213 mmol) and CeCl₃ (30.5 g, 124 mmol) in 350 mL of THF. Yield: 25.5 g (91 %). In a similar manner, the following compounds can also be prepared:

| Examples | Intermediate V | Intermediate VI |
|---|---|---|
| **VI-b** | 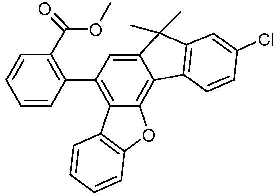 | 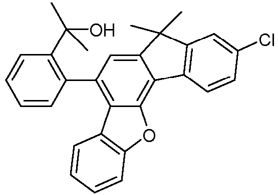 |
| **VI-c** | 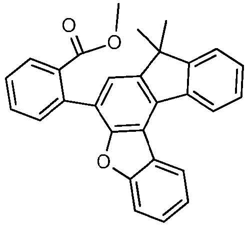 | 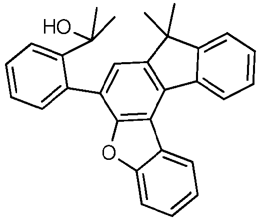 |
| **VI-d*** | 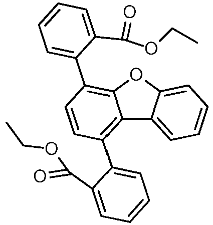 | 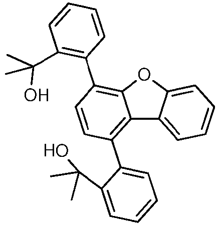 |
| * The reaction conditions are adapted and 8 eq. of MeMgCl and 4 eq of CeCl₃ are used. | | |
| **VI-e*** | 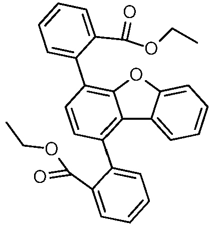 | 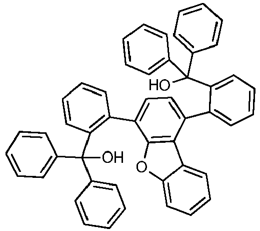 |
| *instead of MeMgCl 8 eq. Phenylmagnesium chloride (100-59-4) and 4 eq of CeCl₃ are used | | |
| **VI-f*** | 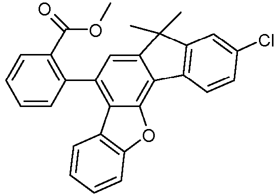 | 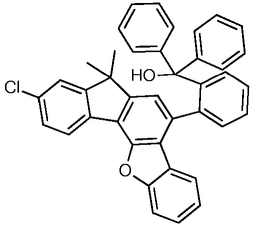 |
| *instead of MeMgCl Phenylmagnesium chloride (100-59-4) is used | | |

### Synthesis Intermediate VII-a:

Intermediate **VII-a** can be synthesized following the same procedure as intermediate **III-a** starting from 25 g (55 mmol) of **VI-a** and 12 g of Amberlyst-15 in 300 mL of toluene. Yield: 20.4 g (85%).

In a similar manner, the following compounds can also be prepared:

| Examples | Intermediate VI | Intermediate VII |
|---|---|---|
| **VII-b** | 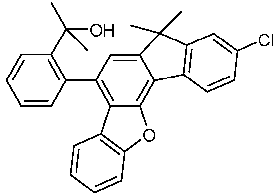 | 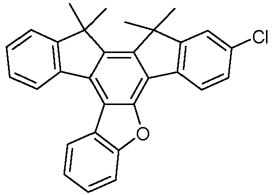 |
| **VII-c** | 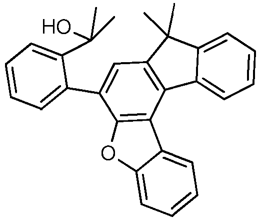 | 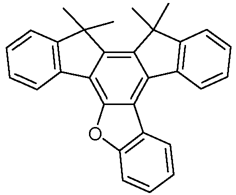 |
| **VII-c** (alternative synthesis) | 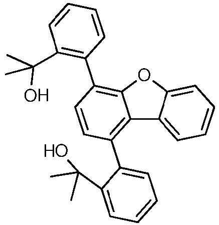 | 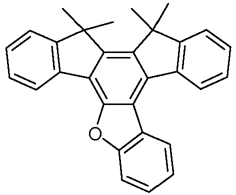 |
| **VII-d** | 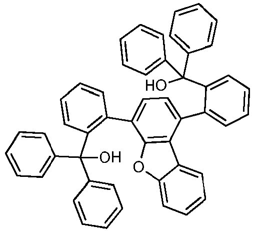 | 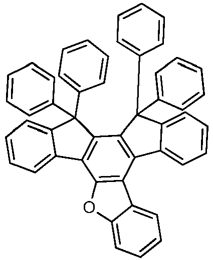 |
| **VII-e** | 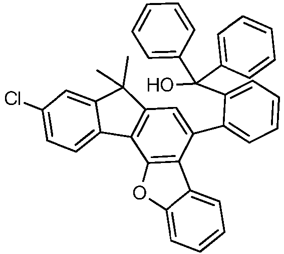 | 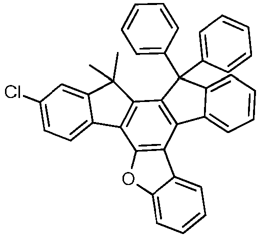 |

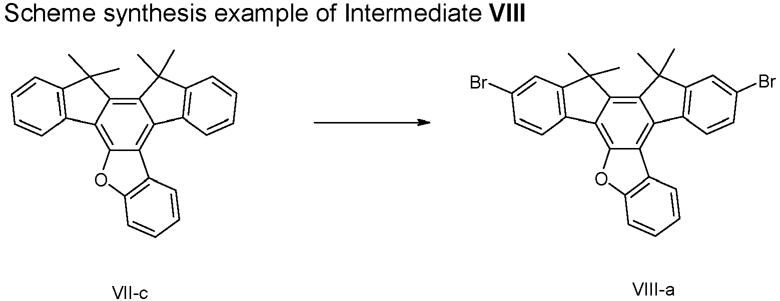

### Synthesis of Intermediate VIII-a:

Intermediate **VIII-a** can be synthesized following the same procedure as intermediate **IV-a** starting from 15 g (37.5 mmol) of **VII-c** and 13.3 g (75 mmol) of N-bromosuccinimide in 300 mL CH₂Cl₂. Yield: 15.3 g (73%) In a similar manner, the following compound can also be prepared:

| Examples | Intermediate VII | Intermediate VIII |
|---|---|---|
| **VIII-b*** | 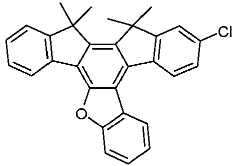 | 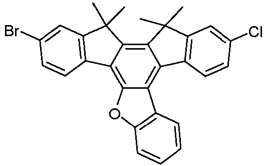 |
| * The reaction conditions are adapted and 1 eq. of NBS is used | | |
| **VIII-c** | 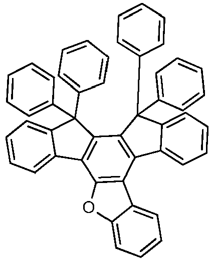 | 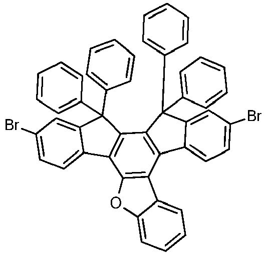 |

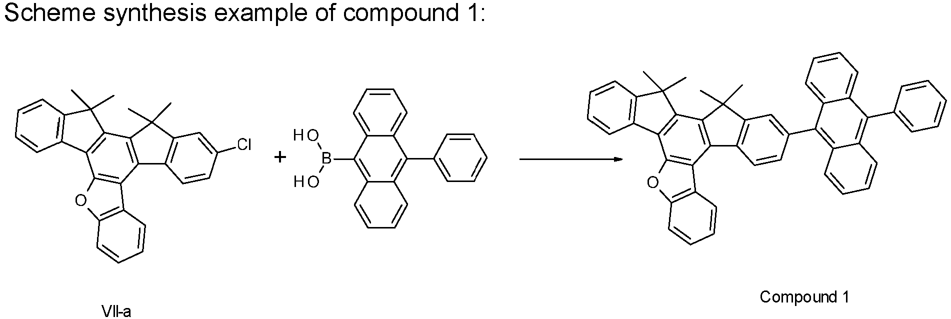

### Synthesis of compound 1

21.7 g (50 mmol) of **VII-a,** 21.0 g (55 mmol) of (10-phenylanthracen-9-yl)boronic acid and 23.0 g (100 mmol) potassium phosphate are mixed in 600 mL THF/water (2:1). After adding 1 g (1.2 mmol) XPhos-Pd-G3 the reaction is stirred at 60 °C until complete conversion. After cooling the mixture to room temperature, 200 mL water and 500 mL toluene are added and the phases are separated. The organic phase is washed multiple times with water and the solvent is removed in vacuum. Afterwards, the crude product is purified by filtration through silica (toluene) and by several recrystalisations from toluene/heptane. Yield: 24.2 g (74 %).

In a similar manner, the next compounds can be prepared.

| Compound | Intermediate | Boronate | Product |
|---|---|---|---|
| **1.2** | **VII-a** | 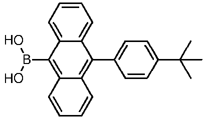 | 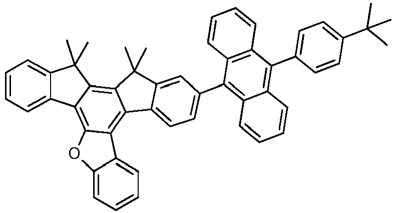 |
| **1.3** | **VII-a** | 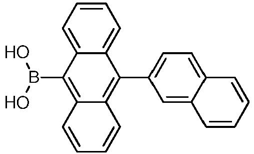 | 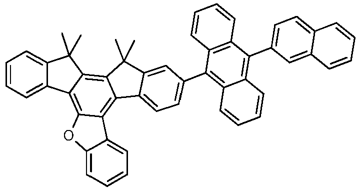 |
| **1.4** | **VII-a** | 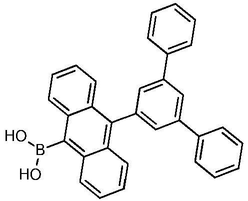 | 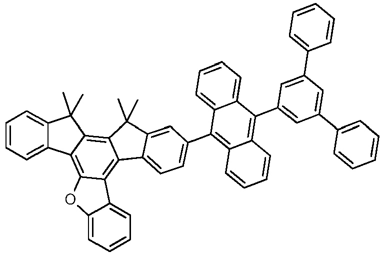 |
| **1.5** | **VIII-b** | 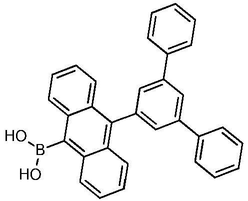 | 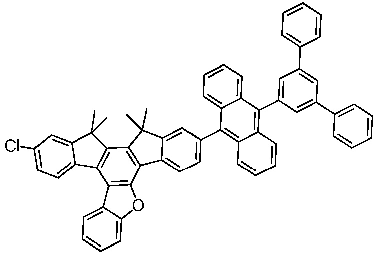 |
| **1.6** | **VIII-b** | 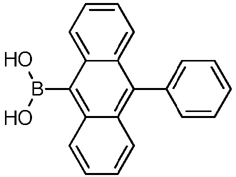 | 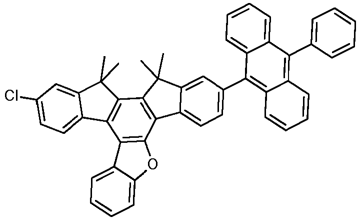 |
| **1.7** | **VII-e** | 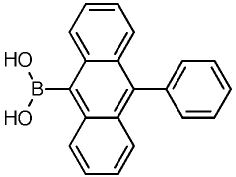 | 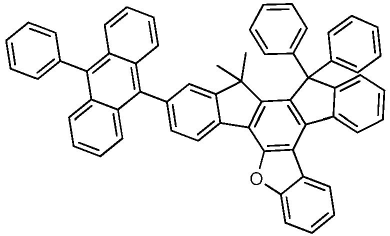 |
| **1.8** | **VII-b** | 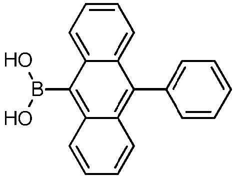 | 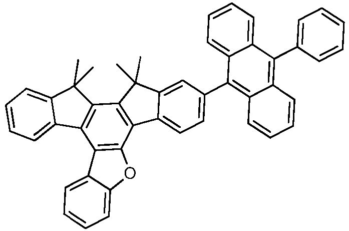 |

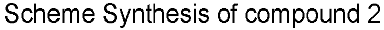

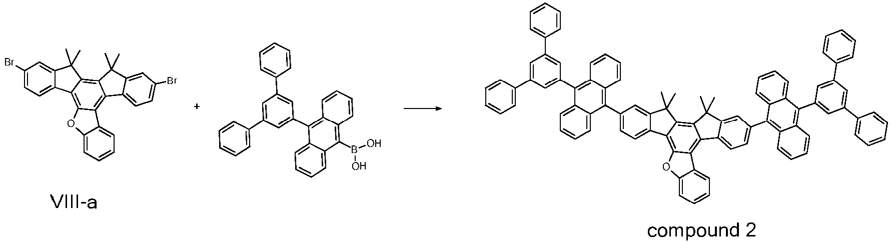

### Synthesis of compound 2

27.9 g (50 mmol) of **VIII-a,** 49.5 g (110 mmol) of (10-(5-phenyl-[1,1'-biphenyl]-3-yl) anthracen-9-yl)boronic acid and 46.0 g (200 mmol) potassium phosphate are mixed in 1000 ml THF/water (2:1). After adding 2 g (2.4 mmol) XPhos-Pd-G3, the reaction is stirred at 60 °C until complete conversion. After cooling the mixture to room temperature, 200 mL water and 500 mL toluene are added and the phases are separated. The organic phase is washed multiple times with water and the solvent is removed in vacuum. Afterwards, the crude product is purified by filtration through silica (toluene) and by several recrystalisations from toluene/heptane. Yield: 31.5 g (52 %).

In a similar manner, the next compounds can be prepared:

| Compound | Int. | Boronate | Product |
|---|---|---|---|
| **2.2** | **VIII-a** | 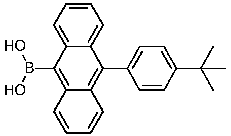 | 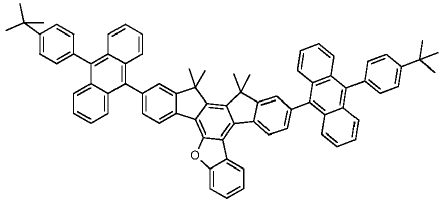 |
| **2.3** | **VIII-a** | 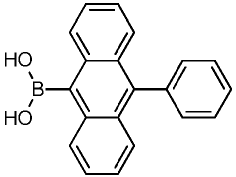 | 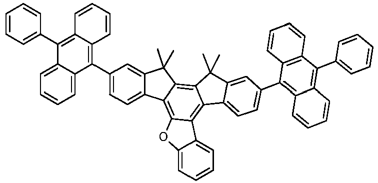 |
| **2.4** | **VIII-a** | 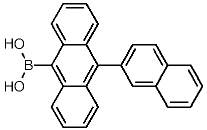 | 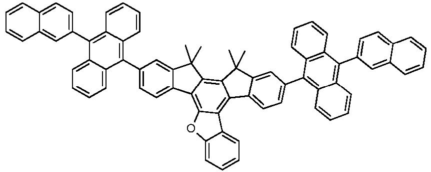 |
| **2.5** | **VIII-c** | 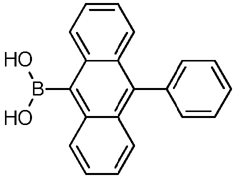 | 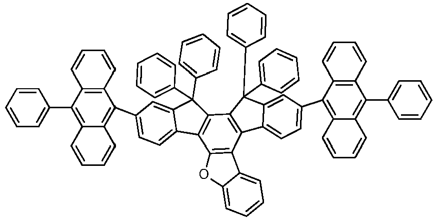 |

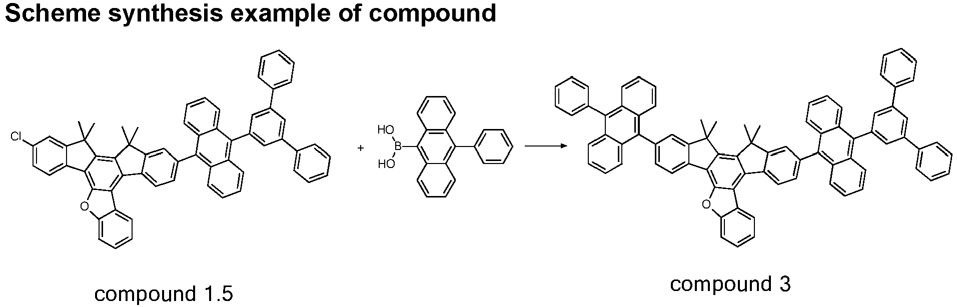

21.0 g (25 mmol) of compound **1.5,** 11.0 g (28 mmol) of (10-phenylanthracen-9-yl)boronic acid and 11.5 g (50 mmol) potassium phosphate are mixed in 500 mL THF/water (2:1). After adding 1 g (1.2 mmol) XPhos-Pd-G3 the reaction is stirred at 60 °C until complete conversion. After cooling the mixture to room temperature, 200 mL water and 500 mL toluene are added and the phases are separated. The organic phase is washed multiple times with water and the solvent is removed in vacuum. Afterwards, the crude product is purified by filtration through silica (toluene) and by several recrystalisation from toluene/heptane. Yield: 17.7 g (67 %).

| Compound | Int. | Boronate | Product |
|---|---|---|---|
| **3.2** | **1.5** | 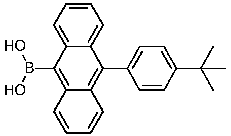 | 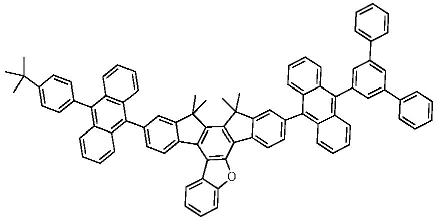 |
| **3.3** | **1.5** | 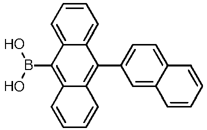 | 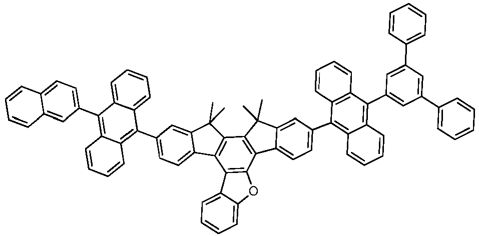 |
| **3.4** | **1.6** | 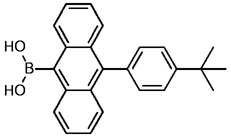 | 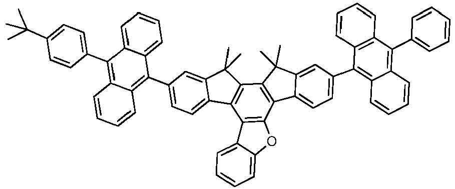 |
| **3.5** | **1.6** | 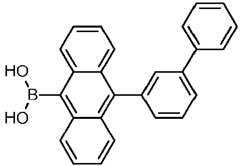 | 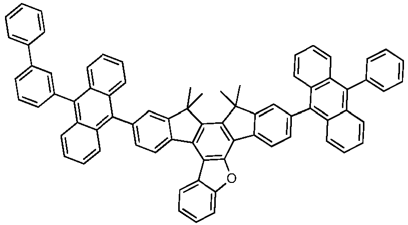 |

### B) Fabrication of OLEDs

### Fabrication of vapor processed OLED devices

The manufacturing of the OLED devices is performed accordingly to WO 04/05891 with adapted film thicknesses and layer sequences. The following examples V1, E1, E2 and E3 show data of various OLED devices.

### Substrate pre-treatment of examples V1, E1, E2 and E3:

Glass plates with structured ITO (50 nm, indium tin oxide) are coated with 20 nm PEDOT:PSS (Poly(3,4-ethylenedioxythiophene) poly(styrene-sulfonate, CLEVIOS^{™} P VP Al 4083 from Heraeus Precious Metals GmbH Germany, spin-coated from a water-based solution) to form the substrates on which the OLED devices are fabricated.

The OLED devices have in principle the following layer structure:
- Substrate,
- ITO (50 nm),
- Buffer (20 nm),
- Hole transporting layer (HTL),
- Interlayer (IL)
- Emissive layer (EML),
- Hole blocking layer (HBL),
- Electron transporting layer (ETL),
- Electron injection layer (EIL),
- Cathode.

The cathode is formed by an aluminium layer with a thickness of 100 nm. The detailed stack sequence is shown in table A. The materials used for the OLED fabrication are presented in table C.

All materials are applied by thermal vapour deposition in a vacuum chamber. The emission layer here always consists of at least one matrix material (host material=H) and an emitting dopant (emitter=D), which is mixed with the matrix material or matrix materials in a certain proportion by volume by co-evaporation. An expression such as H1:D1 (97%:3%) here means that material H1 is present in the layer in a proportion by volume of 97%, whereas D1 is present in the layer in a proportion of 3%. Analogously, the electron-transport layer may also consist of a mixture of two or more materials.

The OLED devices are characterised by standard methods. For this purpose, the electroluminescence spectra, the current efficiency (measured in cd/A), power efficiency (lm/W) and the external quantum efficiency (EQE, measured in % at 1000 cd/m²) are determined from current/voltage/luminance characteristic lines (IUL characteristic lines) assuming a Lambertian emission profile. The electroluminescence (EL) spectra are recorded at a luminous density of 1000 cd/m² and the CIE 1931 x and y coordinates are then calculated from the EL spectrum. U1000 is defined as the voltage at luminous density of 1000 cd/m². SE1000 represents the current efficiency, LE1000 the power efficiency at 1000 cd/m². EQE1000 is defined as the external quantum efficiency at luminous density of 1000 cd/m². The device data of various OLED devices are summarized in table B. The example V1 represents the comparative example according to the state-of-the-art. The examples E1, E2 and E3 show data of inventive OLED devices.

In the following section several examples are described in more detail to show the advantages of the inventive OLED devices.

### Use of inventive compounds as host material in fluorescent OLEDs

The inventive compounds are especially suitable as a host (matrix) when blended with a fluorescent blue dopant (emitter) to form the emissive layer of a fluorescent blue OLED device. The representative examples are H1, H2 and H3. Comparative compound for the state-of-the-art is represented by SdT1 (structures see table C). The use of the inventive compound as a host (matrix) in a fluorescent blue OLED device results in excellent device data, especially with respect to efficiency when compared to the state-of-the-art (compare E1 to E3 versus V1, see device data see table B).

**Table A: Device stack of vapor processed OLEDs**

| Ex. | HTL [nm] | IL [nm] | EBL [nm] | EML [nm] | HBL [nm] | ETL [nm] | EIL [nm] |
|---|---|---|---|---|---|---|---|
| V1 | SpA1 140nm | HAT-CN 5nm | SpMA1 20nm | SdT1:D1 (97%:3%) 20nm | HBM 10nm | ETM:LiQ (50%:50%) 20nm | LiQ 1nm |
| E1 | SpA1 140nm | HAT-CN 5nm | SpMA1 20nm | H1:D1 (97%:3%) 20nm | HBM 10nm | ETM:LiQ (50%:50%) 20nm | LiQ 1nm |
| E2 | SpA1 140nm | HAT-CN 5nm | SpMA1 20nm | H2:D1 (97%:3%) 20nm | HBM 10nm | ETM: LiQ (50%:50%) 20nm | LiQ 1nm |
| E3 | SpA1 140nm | HAT-CN 5nm | SpMA1 20nm | H3:D1 (97%:3%) 20nm | HBM 10nm | ETM: LiQ (50%:50%) 20nm | LiQ 1nm |

**Table B: Device data of vapor processed OLEDs**

| **Ex.** | U1000 (V) | SE1000 (cd/A) | LE1000 (lm/W) | EQE1000 (%) | CIE x/y @ 1000 cd/m² |
|---|---|---|---|---|---|
| **V1** | 4.4 | 3.7 | 2.6 | 5.7% | 0.15/0.07 |
| **E1** | 4.4 | 4.5 | 3.1 | 6.5% | 0.15/0.07 |
| **E2** | 4.5 | 4.7 | 3.3 | 6.3% | 0.15/0.08 |
| **E3** | 4.3 | 4.8 | 3.5 | 6.7% | 0.15/0.07 |

**Table C: Structural formulae of vapor processed OLED materials**

| | |
|---|---|
| 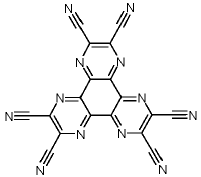 | 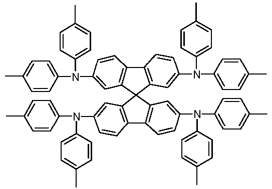 |
| HAT-CN | SpA1 |
| 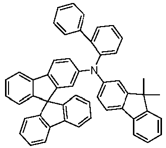 | 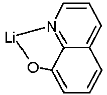 |
| SpMA1 | LiQ |
| 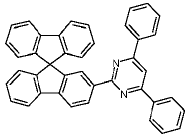 | 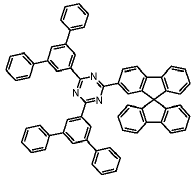 |
| HBM | ETM |
| 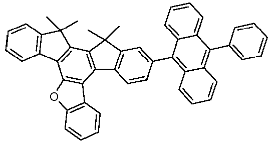 | 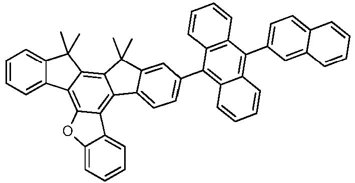 |
| H1 | H2 |
| 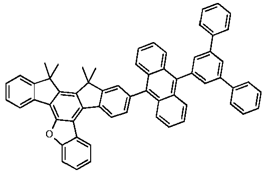 | 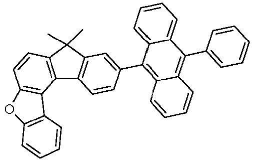 |
| H3 | SdT1 |
| 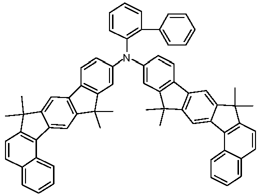 | |
| D1 | |

### Fabrication of solution processed OLED devices

The production of solution-based OLEDs has already been described many times in the literature, for example in WO 2004/037887 and WO 2010/097155. The process is adapted to the circumstances described below (layer-thickness variation, materials).

The inventive material combinations are used in the following layer sequence:
- substrate,
- ITO (50 nm),
- Buffer (40 nm),
- emission layer (EML) (40 nm),
- hole-blocking layer (HBL) (10 nm)
- electron-transport layer (ETL) (30 nm),
- cathode (Al) (100 nm).

Glass plates coated with structured ITO (indium tin oxide) in a thickness of 50 nm serve as substrate. These are coated with the buffer (PEDOT) Clevios P VP Al 4083 (Heraeus Clevios GmbH, Leverkusen). The spin coating of the buffer is carried out from water in air. The layer is subsequently dried by heating at 180°C for 10 minutes. The emission layers are applied to the glass plates coated in this way.

The emission layer (EML) is composed of the matrix material (host material) H4 and the emitting dopant (emitter) D2. Both materials are present in the emission layer in a proportion of 97 % by weight H4 and 3 % by weight D2. The mixture for the emission layer is dissolved in chlorobenzene. The solids content of such solutions is about 9 mg/ml if, as here, the layer thickness of 40 nm which is typical for a device is to be achieved by means of spin coating. The layers are applied by spin coating in an inert-gas atmosphere, and dried by heating at 150°C for 10 minutes. The materials used in the present case are shown in Table D.

**Table D: Structural formulae of the solution processed materials in the EML**

| | |
|---|---|
| 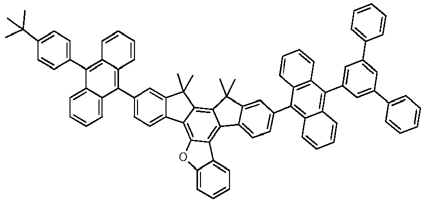 | 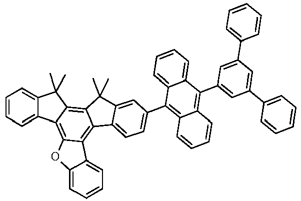 |
| H4 | H5 |
| 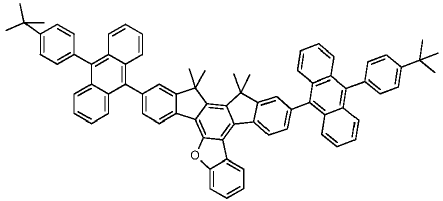 | 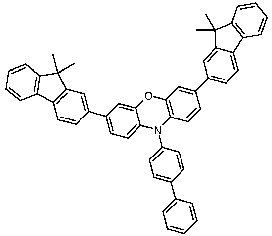 |
| H6 | D2 |

The materials for the hole-blocking layer and electron-transport layer are likewise applied by thermal vapour deposition in a vacuum chamber and are shown in Table C. The hole-blocking layer (HBL) consists of ETM. The electron-transport layer (ETL) consists of the two materials ETM and LiQ, which are mixed with one another in a proportion by volume of 50% each by co-evaporation. The cathode is formed by the thermal evaporation of an aluminium layer with a thickness of 100 nm.

The OLEDs are characterised by standard methods. For this purpose, the electroluminescence spectra are recorded, the current efficiency (measured in cd/A) and the external quantum efficiency (EQE, measured in percent) as a function of the luminous density assuming Lambert emission characteristics are calculated from current/voltage/luminous density characteristic lines (IUL characteristic lines). The electroluminescence spectra are recorded at a luminous density of 1000 cd/m², and the CIE 1931 x and y colour coordinates are calculated from this data. The term EQE1000 denotes the external quantum efficiency at an operating luminous density of 1000 cd/m².

The properties of the various OLEDs are summarised in table E. Examples E4, E5 and E6 show properties of OLEDs containing materials of the present invention.

**Table E: Device data of solution processed OLEDs**

| Example | EML host | EML dopant | EQE1000 [%] | CIE x/y @ 1000 cd/m² |
|---|---|---|---|---|
| E3 | H4 | D2 | 3.6 | 0.15 / 0.14 |
| E4 | H5 | D2 | 3.1 | 0.15 / 0.15 |
| E5 | H6 | D2 | 3.8 | 0.15 / 0.13 |

Table E shows that use of materials (H4, H5, H6) according to the present invention results in excellent device data when used as fluorescent blue host material.

## Claims

1. Compound of the formula (2B), (3B) or (4B), 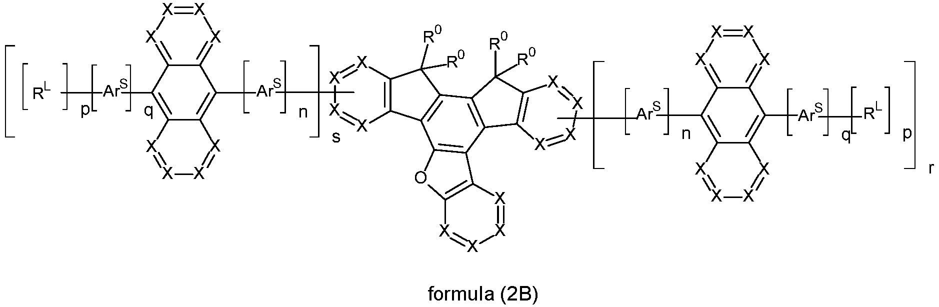 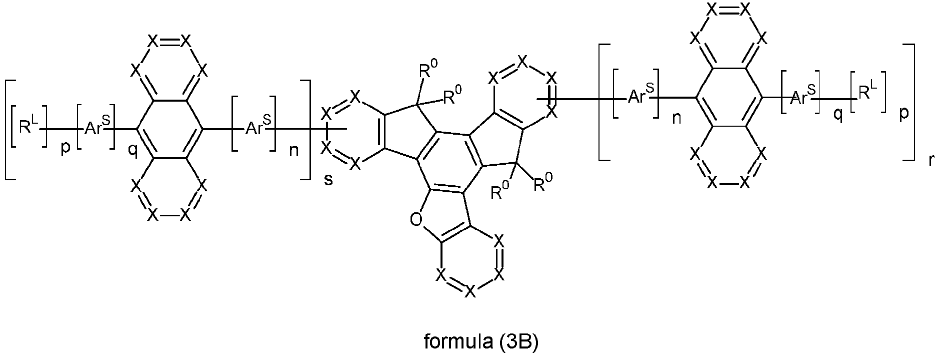 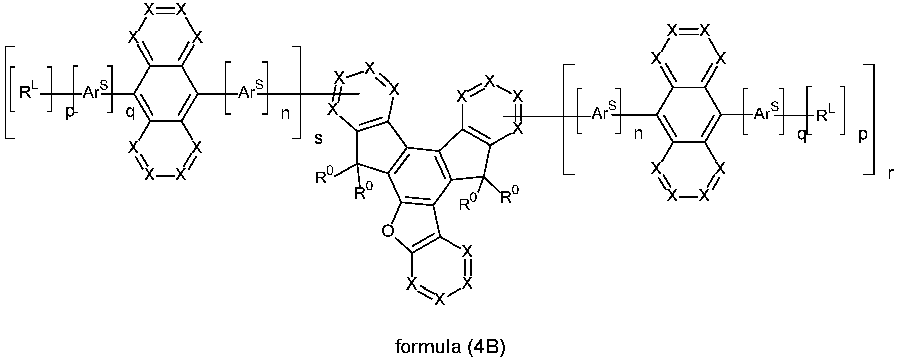 where the following applies to the symbols and indices used:
Ar^{S} stands on each occurrence, identically or differently, for an aromatic or heteroaromatic ring system having 5 to 25 aromatic ring atoms, which may in each case be substituted by one or more radicals R¹;
X stands for CR², N; or X stands for C, if it is bonded to an adjacent group;
R⁰, R¹, R² stand on each occurrence, identically or differently, for H, D, F, a straight-chain alkyl, alkoxy or thioalkyl groups having 1 to 20 C atoms or branched or a cyclic alkyl, alkoxy or thioalkyl groups having 3 to 20 C atoms, each of which may be substituted by one or more radicals R, where one or more H atoms may be replaced by D or F, an aromatic or heteroaromatic ring systems having 5 to 30 aromatic ring atoms, which may in each case be substituted by one or more radicals R, where two radicals R⁰ may form a ring system with one another, which may be substituted by one or more radicals R;
R^{L} stands on each occurrence, identically or differently, for H, D, F, a straight-chain alkyl group having 1 to 20 C atoms or branched or a cyclic alkyl group having 3 to 20 C atoms, each of which may be substituted by one or more radicals R, or an aromatic ring system having 5 to 25 aromatic ring atoms, which may in each case be substituted by one or more radicals R;
R stands on each occurrence, identically or differently, for H, D, F, a straight-chain alkyl group having 1 to 10 C atoms or branched or cyclic alkyl group having 3 to 20 C atoms, each of which may be substituted by one or more radicals R', where one or more H atoms may be replaced by D or F, or an aromatic ring system having 6 to 18 aromatic ring atoms;
Ar is an aromatic or heteroaromatic ring system having 5 to 24 aromatic ring atoms, which may in each case also be substituted by one or more radicals R';
R' stands on each occurrence, identically or differently, for H, D, F, a straight-chain alkyl group having 1 to 10 C atoms or branched or cyclic alkyl group having 3 to 10 C atoms, where one or more H atoms may be replaced by D or F, or an aromatic ring system having 5 to 18 C atoms;
n, q, p are identically or differently an integer selected from 0 or 1; and
r, s are identically or differently, equal to 0 or 1; with the proviso that r + s ≥ 1.

2. Compound according to claim 1, **characterized in that** n and q are, identically or differently, equal to 0 or 1 and p is equal to 1.

3. Compound according to claim 1 or 2, **characterized in that** the group Ar^{S} stands on each occurrence, identically or differently, for phenyl, biphenyl, fluorene, spirobifluorene, naphthalene, phenanthrene, anthracene, dibenzofuran, dibenzothiophene, carbazole, pyridine, pyrimidine, pyrazine, pyridazine, triazine, benzopyridine, benzopyridazine, benzopyrimidine and quinazoline, each of which may be substituted by one or more radicals R¹.

4. Compound according to one or more of the preceding claims, **characterized in that** the group R^{L} is an aromatic ring system selected from phenyl, biphenyl, terphenyl, quaterphenyl, fluorene, naphthalene, phenanthrene, triphenylene, fluoranthene, tetracene, chrysene, benzanthracene, benzophenanthracene, pyrene or perylene, each of which may be substituted by one or more radicals R at any free positions;.

5. Formulation comprising at least one compound according to one or more of the claims 1 to 4 and at least one solvent.

6. Electronic device comprising at least one compound according to one or more of claims 1 to 4, selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

7. Electronic device according to claim 6, which is an organic electroluminescent device, **characterized in that** the compound according to one or more of claims 1 to 4 is employed as a matrix material for a fluorescent emitter in an emitting layer.

## Patentansprüche

1. Verbindung der Formel (2B), (3B) oder (4B), 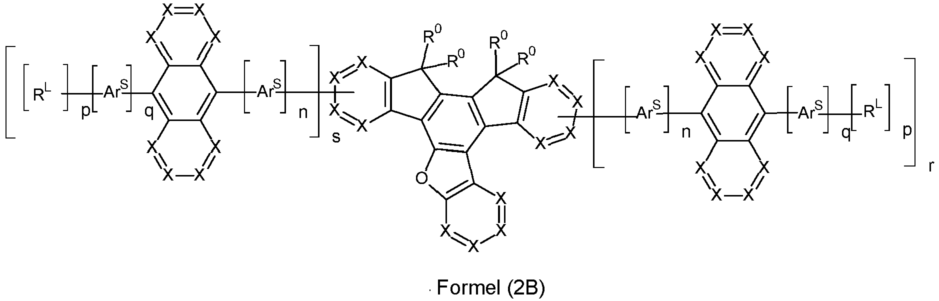 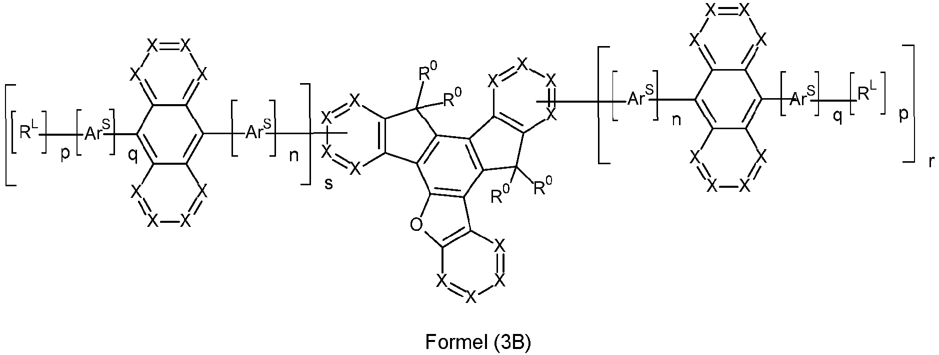 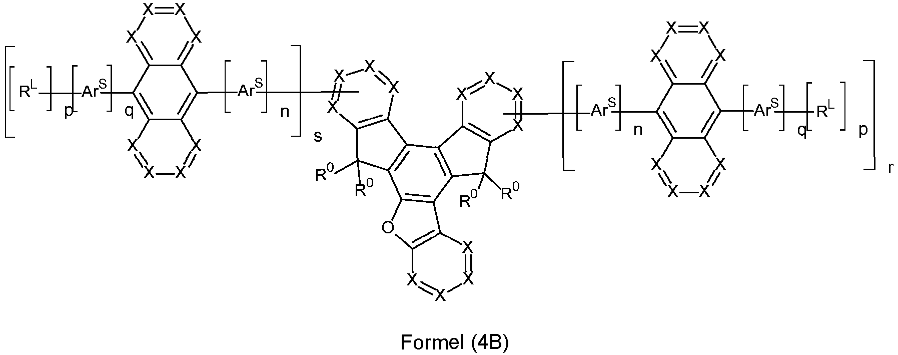 wobei für die verwendeten Symbole und Indizes Folgendes gilt:
Ar^{S} steht bei jedem Auftreten gleich oder verschieden für ein aromatisches oder heteroaromatisches Ringsystem mit 5 bis 25 aromatischen Ringatomen, das jeweils durch einen oder mehrere Reste R¹ substituiert sein kann;
X steht für CR², N; oder X steht für C, wenn es an eine benachbarte Gruppe gebunden ist;
R⁰, R¹, R² stehen bei jedem Auftreten gleich oder verschieden für H, D, F, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, die jeweils durch einen oder mehrere Reste R substituiert sein kann, wobei ein oder mehrere H-Atome durch D oder F ersetzt sein können, ein aromatisches oder heteroaromatisches Ringsystem mit 5 bis 30 aromatischen Ringatomen, das jeweils durch einen oder mehrere Reste R substituiert sein kann, wobei zwei Reste R⁰ miteinander ein Ringsystem bilden können, das durch einen oder mehrere Reste R substituiert sein kann;
R^{L} steht bei jedem Auftreten gleich oder verschieden für H, D, F, eine geradkettige Alkylgruppe mit 1 bis 20 C-Atomen oder eine verzweigte oder cyclische Alkylgruppe mit 3 bis 20 C-Atomen, die jeweils durch einen oder mehrere Reste R substituiert sein kann, oder ein aromatisches Ringsystem mit 5 bis 25 aromatischen Ringatomen, das jeweils durch einen oder mehrere Reste R substituiert sein kann;
R steht bei jedem Auftreten gleich oder verschieden für H, D, F, eine geradkettige Alkylgruppe mit 1 bis 10 C-Atomen oder verzweigte oder cyclische Alkylgruppe mit 3 bis 20 C-Atomen, die jeweils durch einen oder mehrere Reste R' substituiert sein kann, wobei ein oder mehrere H-Atome durch D oder F ersetzt sein können, oder ein aromatisches Ringsystem mit 6 bis 18 aromatischen Ringatomen;
Ar ist ein aromatisches oder heteroaromatisches Ringsystem mit 5 bis 24 aromatischen Ringatomen, das jeweils auch durch einen oder mehrere Reste R' substituiert sein kann;
R' steht bei jedem Auftreten gleich oder verschieden für H, D, F, eine geradkettige Alkylgruppe mit 1 bis 10 C-Atomen oder verzweigte oder cyclische Alkylgruppe mit 3 bis 10 C-Atomen, wobei ein oder mehrere H-Atome durch D oder F ersetzt sein können, oder ein aromatisches Ringsystem mit 5 bis 18 C-Atomen;
n, q, p sind gleich oder verschieden eine ganze Zahl, die ausgewählt ist aus 0 oder 1; und
r, s sind gleich oder verschieden gleich 0 oder 1; mit der Maßgabe, dass r + s ≥ 1.

2. Verbindung nach Anspruch 1, **dadurch gekennzeichnet, dass** n und q gleich oder verschieden gleich 0 oder 1 sind und p gleich 1 ist.

3. Verbindung nach Anspruch 1 oder 2, **dadurch gekennzeichnet, dass** die Gruppe Ar^{S} bei jedem Auftreten gleich oder verschieden für Phenyl, Biphenyl, Fluoren, Spirobifluoren, Naphthalin, Phenanthren, Anthracen, Dibenzofuran, Dibenzothiophen, Carbazol, Pyridin, Pyrimidin, Pyrazin, Pyridazin, Triazin, Benzopyridin, Benzopyridazin, Benzopyrimidin und Chinazolin steht, das jeweils durch einen oder mehrere Reste R¹ substituiert sein kann.

4. Verbindung nach einem oder mehreren der vorhergehenden Ansprüche, **dadurch gekennzeichnet, dass** die Gruppe R^{L} ein aromatisches Ringsystem ist, das ausgewählt ist aus Phenyl, Biphenyl, Terphenyl, Quaterphenyl, Fluoren, Naphthalin, Phenanthren, Triphenylen, Fluoranthen, Tetracen, Chrysen, Benzanthracen, Benzophenanthracen, Pyren oder Perylen, das an beliebigem freien Positionen jeweils durch einen oder mehrere Reste R substituiert sein kann.

5. Formulierung enthaltend mindestens eine Verbindung nach einem oder mehreren der Ansprüche 1 bis 4 sowie mindestens ein Lösungsmittel.

6. Elektronische Vorrichtung enthaltend mindestens eine Verbindung nach einem oder mehreren der Ansprüche 1 bis 4, ausgewählt aus der Gruppe bestehend aus organischen Elektrolumineszenzvorrichtungen, organischen integrierten Schaltungen, organischen Feldeffekttransistoren, organischen Dünnschichttransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und organischen Plasmon-emittierenden Vorrichtungen.

7. Elektronische Vorrichtung nach Anspruch 6, bei der es sich um eine organische Elektrolumineszenzvorrichtung handelt, **dadurch gekennzeichnet, dass** die Verbindung nach einem oder mehreren der Ansprüche 1 bis 4 als Matrix-Material für einen fluoreszierenden Emitter in einer Emitterschicht eingesetzt wird.

## Revendications

1. Composé de formule (2B), (3B) ou (4B), 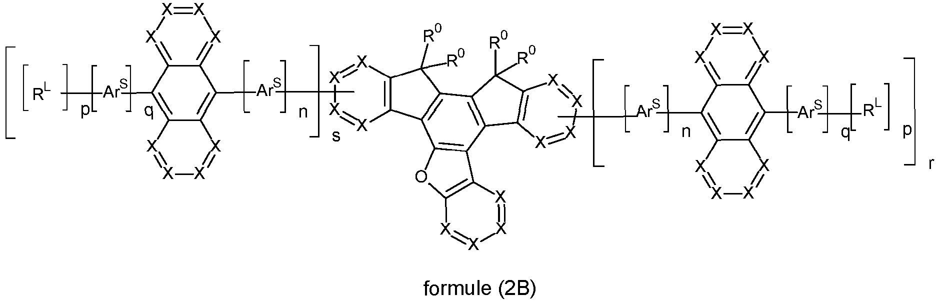 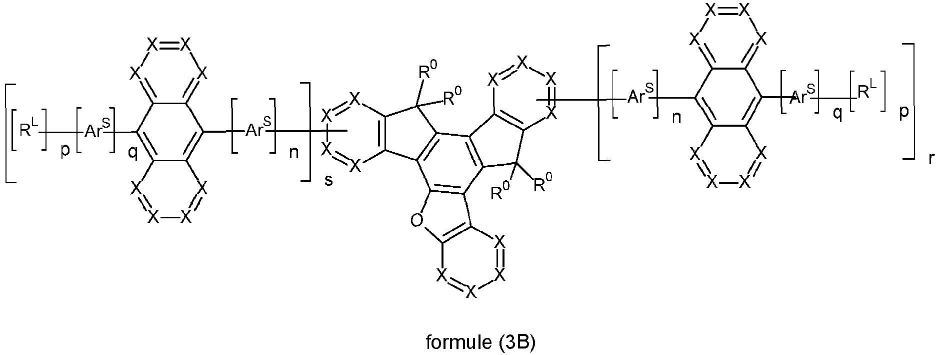 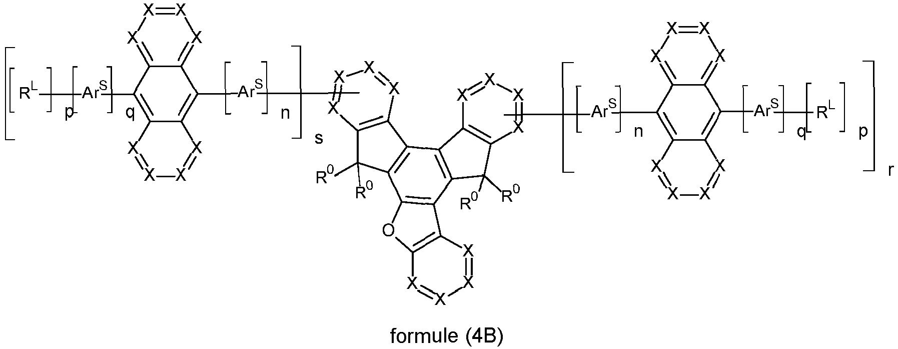 dans laquelle ce qui suit s'applique aux symboles et indices utilisés :
Ar^{S} représente à chaque occurrence, de manière identique ou différente, un noyau aromatique ou hétéroaromatique ayant de 5 à 25 atomes de cycle aromatique, qui peut dans chaque cas être substitué par un ou plusieurs radicaux R¹ ;
X représente CR², N ; ou X représente C, s'il est lié à un groupement adjacent ;
R⁰, R¹, R² représentent à chaque occurrence, de manière identique ou différente, H, D, F, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C, chacun d'entre eux pouvant être substitué par un ou plusieurs radicaux R, où un ou plusieurs atomes de H peuvent être remplacés par D ou F, un noyau aromatique ou hétéroaromatique ayant de 5 à 30 atomes de cycle aromatique, qui peut dans chaque cas être substitué par un ou plusieurs radicaux R, où deux radicaux R⁰ peuvent former un noyau l'un avec l'autre, qui peut être substitué par un ou plusieurs radicaux R ;
R^{L} représente à chaque occurrence, de manière identique ou différente, H, D, F, un groupement alkyle à chaîne linéaire ayant de 1 à 20 atomes de C ou un groupement alkyle ramifié ou cyclique ayant de 3 à 20 atomes de C, chacun d'entre eux pouvant être substitué par un ou plusieurs radicaux R, ou un noyau aromatique ayant de 5 à 25 atomes de cycle aromatique, qui peut dans chaque cas être substitué par un ou plusieurs radicaux R ;
R représente à chaque occurrence, de manière identique ou différente, H, D, F, un groupement alkyle à chaîne linéaire ayant de 1 à 10 atomes de C ou un groupement alkyle ramifié ou cyclique ayant de 3 à 20 atomes de C, chacun d'entre eux pouvant être substitué par un ou plusieurs radicaux R', où un ou plusieurs atomes de H peuvent être remplacés par D ou F, ou un noyau aromatique ayant de 6 à 18 atomes de cycle aromatique ;
Ar est un noyau aromatique ou hétéroaromatique ayant de 5 à 24 atomes de cycle aromatique, qui peut dans chaque cas être également substitué par un ou plusieurs radicaux R' ;
R' représente à chaque occurrence, de manière identique ou différente, H, D, F, un groupement alkyle à chaîne linéaire ayant de 1 à 10 atomes de C ou un groupement alkyle ramifié ou cyclique ayant de 3 à 10 atomes de C, où un ou plusieurs atomes de H peuvent être remplacés par D ou F, ou un noyau aromatique ayant de 5 à 18 atomes de C ;
n, q, p sont de manière identique ou différente un nombre entier choisi parmi 0 ou 1 ; et
r, s sont de manière identique ou différente, égaux à 0 ou 1 ; à condition que r + s ≥ 1.

2. Composé selon la revendication 1, **caractérisé en ce que** n et q sont, de manière identique ou différente, égaux à 0 ou 1 et p est égal à 1.

3. Composé selon la revendication 1 ou 2, **caractérisé en ce que** le groupement Ar^{S} représente à chaque occurrence, de manière identique ou différente, phényle, biphényle, fluorène, spirobifluorène, naphtalène, phénanthrène, anthracène, dibenzofurane, dibenzothiophène, carbazole, pyridine, pyrimidine, pyrazine, pyridazine, triazine, benzopyridine, benzopyridazine, benzopyrimidine et quinazoline, chacun d'entre eux pouvant être substitué par un ou plusieurs radicaux R¹.

4. Composé selon l'une ou plusieurs parmi les revendications précédentes, **caractérisé en ce que** le groupement R^{L} est un noyau aromatique choisi parmi phényle, biphényle, terphényle, quaterphényle, fluorène, naphtalène, phénanthrène, triphénylène, fluoranthène, tétracène, chrysène, benz-anthracène, benzophénanthracène, pyrène ou pérylène, chacun d'entre eux pouvant être substitué par un ou plusieurs radicaux R au niveau d'une position libre quelconque.

5. Formulation comprenant au moins un composé selon l'une ou plusieurs parmi les revendications 1 à 4 et au moins un solvant.

6. Dispositif électronique comprenant au moins un composé selon l'une ou plusieurs parmi les revendications 1 à 4, choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les cellules solaires organiques à colorant, les détecteurs optiques organiques, les photorécepteurs organiques, les dispositifs organiques à extinction de champ, les cellules électrochimiques émettrices de lumière, les diodes laser organiques et les dispositifs organiques émetteurs de plasmons.

7. Dispositif électronique selon la revendication 6, qui est un dispositif électroluminescent organique, **caractérisé en ce que** le composé selon l'une ou plusieurs parmi les revendications 1 à 4 est employé comme matériau de matrice pour un émetteur fluorescent dans une couche émettrice.
